# EUROPÄISCHE PATENTSCHRIFT

(11) **EP 0 778 844 B1**
(45) Veröffentlichungstag und Bekanntmachung des Hinweises auf die Patenterteilung: **18.12.2002**
(21) Anmeldenummer: 95931224.0
(22) Anmeldetag: 30.08.1995
(51) Int. Cl.: C07H 21/00, C12Q 1/68, C07D 213/00

(54) **FUNKTIONELLE TERPYRIDIN-METALLKOMPLEXE, VERFAHREN ZU DEREN HERSTELLUNG UND OLIGONUKLEOTID-KONJUGATE MIT TERPYRIDIN-METALLKOMPLEXEN**
FUNCTIONAL TERPYRIDINE METAL COMPLEXES, METHODS FOR THEIR PREPARATION AND OLIGONUCLEOTIDE CONJUGATES WITH TERPYRIDINE METAL COMPLEXES
COMPLEXES TERPYRIDINE-METAL FONCTIONNELS, LEUR PROCEDE DE FABRICATION ET CONJUGUES D'OLIGONUCLEOTIDES COMPRENANT DES COMPLEXES TERPYRIDINE-METAL

(30) Priorität: 02.09.1994 CH 269394
(43) Veröffentlichungstag der Anmeldung: 18.06.1997
(73) Patentinhaber: Novartis AG, 4056 Basel (CH); Novartis-Erfindungen Verwaltungsgesellschaft m.b.H., 1235 Wien (AT)
(72) Erfinder: HÄNER, Robert, CH-4232 Fehren (CH); HALL, Jonathan, CH-3012 Bern (CH); HÜSKEN, Dieter, D-79102 Freiburg (DE); PIELES, Uwe, D-79418 Schliengen (DE); MOSER, Heinz, West Sussex RH13 7GN (GB)
(86) Internationale Anmeldenummer: EP9503409
(87) Internationale Veröffentlichungsnummer: WO96007668

(56) Entgegenhaltungen:
- WO-A-94/29316
- JOURNAL OF THE AMERICAN CHEMICAL SOCIETY, Bd. 116, Nr. 16, 10.August 1994 DC US, Seiten 7439-7440, D.MAGDA ET AL. 'Site-Specific Hydrolysis of RNA by Europium (III) Texaphyrin Conjugated to a Synthetic Oligodeoxyribonucleotide.' in der Anmeldung erwähnt
- CHEMISTRY AND BIOLOGY , Bd. 1, Nr. 3, November 1994 Seiten 185-190, J.HALL ET AL. 'Efficient Sequence-Specific Cleavage of RNA Using Novel Europium Complexes Conjugated to Oligonucleotides,' in der Anmeldung erwähnt
- POLYHEDRON, Bd. 7, Nr. 24, 1988 Seiten 2531-2536, E.C.CONSTABLE ET AL. 'The Preparation and Coordination Chemistry of 2,2':6'2''-Terpyridine Macrocycles - VI.* Planar Hexadentate Macrocycles Incorporating 2,2':6',2''-Terpyridine.'
- POLYHEDRON, Bd. 1, Nr. 3, 1982 Seiten 303-306, E.C.CONSTABLE ET AL. 'The Preparation and Coordination Chemistry of 2,2':6',2''-Terpyridine Macrocycles - 1'

## Beschreibung

Die vorliegende Erfindung betrifft cyclische Terpyridin-Lanthanid Komplexe mit 8 N-Atomen und 10 C-Atomen im Macrocyclus, die im Terpyridinteil eine funktionelle Gruppe enthalten, ein Verfahren zu deren Herstellung durch Kondensation von Terpyridinhydrazinen mit Pyridin-2,6-dialdehyden oder -ketonen, Assoziate dieser Komplexe mit Oligonukleotiden und Verfahren zur sequenzspezifischen Spaltung von RNA unter Verwendung dieser Assoziate.

Die hydrolytische Spaltung von RNA unter der katalytischen Einwirkung von Metallionen ist schon seit längerem bekannt. Die Spaltung erfolgt grundsätzlich in ungepaarten Bereichen der RNA, die in englisch als "loops" bezeichnet werden. W. J. Krzyzosiak et al., Biochemistry 27:5771-5777 (1988) schlagen hierfür die Verwendung von Bleidiacetat vor. G. J. Murakawa et al., Nucleic Acid Research 17:5361-5375 (1989) beschreiben den Einsatz von Kupferkomplexen des 1,10-Phenantrolins. J. Ciesiolka et al., Eur. J. Biochem. 182:445-450 (1989) offenbart Europiumtrichlorid für den gleichen Verwendungszweck zur Spaltung von tRNA^{Phe}. C. S. Chow et al. verwenden in J. Am. Chem. Soc., Band 112, Seiten 2839 bis 2841 (1990) für die gleiche RNS Ruthenium- und Rhodium-Komplexe mit Phenantrolinliganden. In Biochemistry, Band 29, Seiten 2515 bis 2523 erwähnen L. S. Behlen et al. tRNA^{Phe} Mutanten mit Bleidiacetat. Ferner beschreiben N. Hayashi et al. in Inorg. Chem., Band 32, Seiten 5899 bis 5900 (1993), dass für die Spaltung von tRNA auch Lanthanidmetallkomplexe geeignet sind.

Es ist ferner von D. Magda et al. im J. Am. Chem. Soc., Band 116, 7439 bis 7440 (1994) beschrieben worden, dass Konjugate aus Europium(III)-Texaphyrin und Oligonukleotiden mit DNA-Bausteinen eine Ziel-RNA zu spalten vermögen, wobei im Bereich des Texaphyrin-Komplexes in dem RNA/Oligonukleotid-Komplex eine Spaltung von lediglich etwa 30 % beobachtet wird. Nachteilig bei diesen Texaphyrin-Komplexen ist auch, dass zusätzlich Hydroxypropyl im Liganden gebunden sein müssen, damit eine ausreichende Löslichkeit gewährleistet ist. Ferner sind die Imingruppen des Liganden hydrolyseanfällig, so dass die Wirksamkeit in wässriger Umgebung relativ schnell nachlässt, beziehungsweise eine zu geringe Verweilzeit bei therapeutischer Anwendung besteht. Durch die Hydrolyse des Liganden wird zudem das Metall frei gesetzt, was ernsthafte toxische Probleme und unspezifische Spaltungen von RNA hervorrufen kann. Ferner sind es schwache Lewissäuren, weil eine Ladung des Eu-Kations durch einen Liganden neutralisiert wird und daher ein zweifach geladener Komplex vor liegt. Die beschriebenen Komplexe sind zudem nur durch synthetisch aufwendige Verfahren zugänglich.

Es ist bekannt, dass in Zellen das Entstehen von physiologisch schädlichen Polypeptiden durch die von Genen gesteuerte Bildung von mRNA erfolgt. Zur Bekämpfung bzw. Verhinderung von Krankheiten sind daher Mittel wünschenswert, die die Wirkung der mRNA verhindern. Im besonderen soll erreicht werden, dass durch irreversible Spaltung an definierter Stelle die mRNA zerstört wird und dadurch der Informationsgehalt verloren geht. Es ist ferner wünschenswert, durch eine sequenzspezifische Spaltung von RNA-Ketten Bruchstücke bereitzustellen, die man zum schnelleren Aufbau von Oligonukleotiden im "Antisense-Gebiet" für diagnostische Zwecke (Biosensoren) oder für die Therapie von Krankheiten unter Beeinflussung von Stoffwechselvorgängen in der Zelle verwenden kann.

Es wurde nun gefunden, dass Oligonukleotide, deren Sequenz komplementär zu einer Ziel-RNA ist und an die ein Terpyridin-Lanthanid-Komplex gebunden ist, hoch wirksam sind und man sequenzspezifische Spaltungen in einer Ziel-RNA erzielen kann.

Ein Gegenstand der vorliegenden Erfindung sind Verbindungen der Formel I 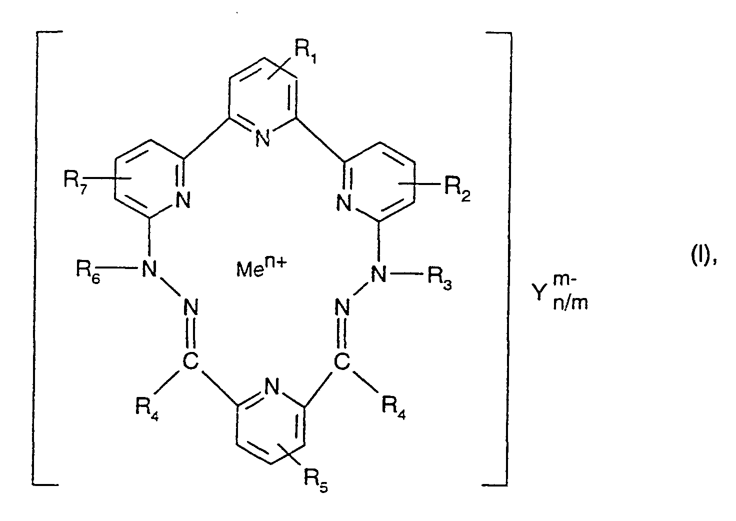 worin
R₁ H, C₁-C₄-Alkyl, C₁-C₄-Alkoxy, C₇-C₁₂-Aralkyl, C₆-C₁₆-Aryl, C₄-C₁₂-Heteroaryl mit O, S oder N als Heteroatome, C₁-C₄-Alkylthio, Di(C₁-C₄-Alkyl)amino, Halogenid, Sulfonamid oder Carboxamid und R₅ eine monovalente funktionelle Gruppe
oder
R₁ eine monovalente funktionelle Gruppe und R₅ H, C₁-C₄-Alkyl, C₁-C₄-Alkoxy, C₇-C₁₂-Aralkyl, C₆-C₁₆-Aryl, C₄-C₁₂-Heteroaryl mit O, S oder N als Heteroatome, C₁-C₄-Alkylthio, Di(C₁-C₄-Alkyl)amino, Halogenid, Sulfonamid oder Carboxamid darstellen,
wobei die funktionelle Gruppe direkt oder über eine Gruppe Z an den Pyridinring gebunden ist und die Gruppe Z einen gegebenenfalls durch -O-, -S-, -NR₁₂-, -C(O)-O- oder -C(O)-NR₁₂- unterbrochenen Rest ausgewählt aus der Gruppe bestehend aus C₁-C₂₀-Alkylen, C₂-C₁₂-Alkenylen, C₂-C₁₂-Alkinylen, C₅-C₈-Cycloalkylen, C₆-C₁₂-Arylen und C₇-C₁₂-Aralkylen bedeutet,
R₂ und R₇ unabhängig voneinander H, C₁-C₄-Alkyl, C₁-C₄-Alkoxy, C₇-C₁₂-Aralkyl, C₆-C₁₆-Aryl, Halogen, C₄-C₁₂-Heteroaryl mit O, S oder N als Heteroatome, C₁-C₄-Alkylthio, Di(C₁-C₄-Alkyl)amino, Sulfonamid oder Carboxamid bedeuten,
R₃ und R₆ unabhängig voneinander H, C₁-C₄-Alkyl, C₇-C₁₂-Aralkyl, C₆-C₁₆-Aryl, Halogen, C₄-C₁₂-Heteroaryl mit O, S oder N als Heteroatome, C₁-C₄-Alkylthio, Di(C₁-C₄-Alkyl)amino, Sulfonamid oder Carboxamid sind,
R₄ für H, C₁-C₂₀-Alkyl, C₅-C₈-Cycloalkyl, C₆-C₁₂-Aryl oder C₇-C₁₂-Aralkyl steht,
R₁₂ H oder C₁-C₆-Alkyl bedeutet,
Me für ein Lanthanidmetall oder Yttrium steht,
Y für ein Anion einer Säure steht,
n die Zahlen 2 oder 3 bedeutet, und
m die Zahlen 1, 2 oder 3 bedeutet,
wobei die Reste Alkyl, Cycloalkyl, Aralkyl, Aryl und die Gruppe Z unsubstituiert oder mit C₁-C₄-Alkoxy, F, Cl, Br, -CN, C₁-C₄-Alkyl oder -NO₂ substituiert sind.

R₁ und R₅ als Substituent sind bevorzugt C₁-C₄-Alkyl, C₁-C₄-Alkoxy, C₇-C₁₂-Aralkyl oder C₆-C₁₆-Aryl, C₄-C₁₂-Heteroaryl mit O, S, N als Heteroatomen, C₁-C₄-Alkylthio, Di(C₁-C₄-Alkyl)amino, Halogenid, Sulfonamid und Carboxamid.

R₁ und R₅ sind bevorzugt in p-Stellung zum N-Atom des Pyridinrings gebunden.

R₂, R₃, R₆ und R₇ bedeuten als Alkyl bevorzugt Methyl oder Ethyl, als Alkoxy bevorzugt Methoxy oder Ethoxy, als Aralkyl bevorzugt Benzylen oder Phenylethylen und als Aryl bevorzugt Naphthyl und besonders Benzyl. In einer bevorzugten Ausführungsform bedeuten R₂ und R₇ H und R₃ und R₆ Alkyl. Besonders bedeuten R₂ und R₇ H sowie R₃ und R₆ C₁-C₄-Alkyl, und ganz besonders Methyl. Bei R₂, R₃, R₆ und R₇ kann es sich auch um C₄-C₁₂-Heteroaryl mit O, S, N als Heteroatomen handeln. Beispiele sind Pyrridyl, Thiazolyl, Imidazolyl, Oxazolyl, Furanosyl, Pyrrolyl, Thiophenyl. Weiter kann es sich um C₁-C₄-Alkylthio, Halogenid, Di(C₁-C₄-Alkyl)amino, Sulfonamid und Carboxamid handeln.

R₄ enthält als Alkyl bevorzugt 1 bis 12, besonders bevorzugt 1 bis 8 und insbesondere 1 bis 4 C-Atome. Einige Beispiele für Alkyl sind Methyl, Ethyl und die Isomeren von Propyl, Butyl, Pentyl, Hexyl, Heptyl, Octyl, Nonyl, Decyl, Undecyl, Dodecyl, Tridecyl, Tetradecyl, Pentadecyl, Hexadecyl, Octadecyl, Nonadecyl und Eicosyl.

R₄ enthält als Cycloalkyl bevorzugt 5 oder 6 Ringkohlenstoffatome. Einige Beispiele für Cycloalkyl sind Cyclopropyl, Cyclobutyl, Cyclopentyl, Cyclohexyl, Cyclopentyl und Cyclooctyl.

R₄ stellt als Aryl bevorzugt Naphthyl und besonders Phenyl dar. Wenn R₄ Aralkyl ist, handelt es sich bevorzugt um Benzyl oder Phenylethyl.

Eine bevorzugte Untergruppe für R₄ ist H, C₁-C₄-Alkyl, besonders Methyl, sowie Phenyl oder Benzyl.

R₁ beziehungsweise R₅ bedeuten als Alkyl bevorzugt Methyl oder Ethyl, als Alkoxy bevorzugt Methoxy oder Ethoxy, als Aryl bevorzugt Naphthyl oder Phenyl, und als Aralkyl bevorzugt Phenyl oder Phenylethyl. Bevorzugt bedeuten R₁ beziehungsweise R₅ H, Methyl, Ethyl, Methoxy oder Ethoxy.

Im Rahmen der vorliegenden Erfindung ist die monovalente funktionelle Gruppe vorzugsweise ausgewählt aus der Gruppe bestehend aus -OR₁₀, -SR₁₀, -NCO, -NCS, -NHR₁₁, -C(O)OR₁₁, -C(O)SH, -C(O)NHR₁₁, -C(O)Cl, -C(S)SR₁₁, -C(S)NHR₁₁, -C(S)OR₁₁, -SO₃R₁₁, -SO₂NHR₁₁, -SO₂Cl, -P(O)(OH)₂, -P(O)(OH)-NHR₁₁, -P(S)(SH)₂, -P(S)(SH)-NHR₁₁, -P(S)(OH)₂, -P(S)(OH)-NHR₁₁, -P(O)(SH)₂, -P(O)(SH)-NHR₁₁, -P(O)(OH)H, -P(O)(NHR₁₁)H, -P(S)(SH)H, -P(S)(NHR₁₁)H, -P(S)(OH)H, -P(O)(SH)H, wobei R₁₀ H, -C(O)NH₂, -C(S)NH₂, -C₁-C₆-Alkyl, -CₓH₂ₓ-NH₂, -CₓH₂ₓ-SH oder -(CₓH₂ₓO)_{y}-H und R₁₁ H, -C₁-C₆-Alkyl, -CₓH₂ₓ-NH₂, CₓH₂ₓ-SH oder -(CₓH₂ₓO)_{y}-H bedeutet und x gleich einer Zahl von 2 bis 6 und y gleich einer Zahl von 1 bis 20 ist. Besonders bevorzugt ist die funktionelle Gruppe ausgewählt aus der Gruppe bestehend aus -OR₁₀, -SR₁₀, -NCO, -NCS, -NHR₁₁, -C(O)OR₁₁ und -P(O)(OH)₂, insbesondere ausgewählt aus der Gruppe bestehend aus -NCS, -C(O)OR₁₁ und -P(O)(OH)₂.

Eine bevorzugte Untergruppe von Verbindungen der Formel 1 sind solche, worin R₂ und R₇ für H stehen, R₃ und R₆C₁-C₄-Alkyl bedeuten, R₄ H, C₁-C₄-Alkyl, Phenyl oder Benzyl darstellen, R₁ eine über C₁-C₃-Alkylen, C₃-Alkinylen, Phenylen oder C₇-Aralkylen, vorzugsweise C₂-C₃-Alkylen oder Phenylen gebundene monovalente funktionelle Gruppe und R₅ H, Methyl oder Methoxy oder R₅ eine über C₁-C₃-Alkylen, C₃-Alkinylen, Phenylen oder C₇-Aralkylen, vorzugsweise C₂-C₃-Alkylen oder Phenylen gebundene monovalente funktionelle Gruppe und R₁ H, Methyl oder Methoxy darstellen.

Unter einem Lanthanidmetall sind im Rahmen der vorliegenden Erfindung alle Lanthanide, das heisst Lanthan (La), Cer (Ce), Praseodym (Pr), Neodym (Nd), Promethium (Pm), Samarium (Sm), Europium (Eu), Gadolinium (Gd), Terbium (Tb), Dysprosium (Dy), Holmium (Ho), Erbium (Er), Thulium (Tm), Ytterbium (Yb) und Lutetium (Lu) zu verstehen. Bevorzugt sind La, Ce, Nd, Eu und Gd, insbesondere La und Eu, ganz besonders bevorzugt Eu.

Geeignete Anionen für die Komplexsalze können zum Beispiel aus der folgenden Gruppe ausgewählt sein: Halogenid (zum Beispiel Cl⁻, Br⁻ und I⁻), das Anion einer Sauerstoffsäure, BF₄⁻, PF₆⁻, SiF₆⁻ und AsF₆⁻.

Bei den Anionen von Sauerstoffsäuren kann es sich zum Beispiel um Sulfat, Phosphat, Perchlorat, Perbromat, Periodat, Antimonat, Arsenat, Nitrat, Carbonat, das Anion einer C₁-C₈-Carbonsäure wie zum Beispiel Formiat, Acetat, Propionat, Butyrat, Benzoat, Phenylacetat, Mono-, Di- oder Trichlor- oder -fluoracetat, Sulfonate wie zum Beispiel Methylsulfonat, Ethylsulfonat, Propylsulfonat, Butylsulfonat, Trifluormethylsulfonat (Triflat), gegebenenfalls mit C₁-C₄-Alkyl, C₁-C₄-Alkoxy oder Halogen, besonders Fluor, Chlor oder Brom substituiertes Phenylsulfonat oder Benzylsulfonat, wie zum Beispiel Tosylat, Mesylat, Brosylat, p-Methoxy- oder p-Ethoxypenylsulfonat, Pentafluorphenylsulfonat oder 2,4,6-Triisopropylsulfonat, und Phosphonate wie zum Beispiel Methylphosphonat, Ethylphosphonat, Propylphoshonat, Butylphosphonat, Phenylphosphonat, p-Methylphenylphosphonat und Benzylphosphonat handeln. Geeignete Anionen sind auch Tartrat, Citrat und Lactat. Bevorzugte Anionen im Rahmen der vorliegenden Erfindung sind F⁻, Cl⁻, Br⁻, J⁻, PF₆⁻, SbF₆⁻, BF₄⁻, B(Ph)₄⁻, Acetat, NO₃⁻, Sulfat und Phosphat, besonders bevorzugte Anionen sind Cl⁻, Acetat und NO₃⁻.

Die oben dargestellten Bevorzugungen und Erläuterungen, insbesondere der Substituenten, der Verbindung der Formel (I) gelten entsprechend auch für die nachfolgend beschriebene Verbindung der Formel (V).

Ein weiterer Gegenstand der vorliegenden Erfindung sind Verbindungen der Formel V 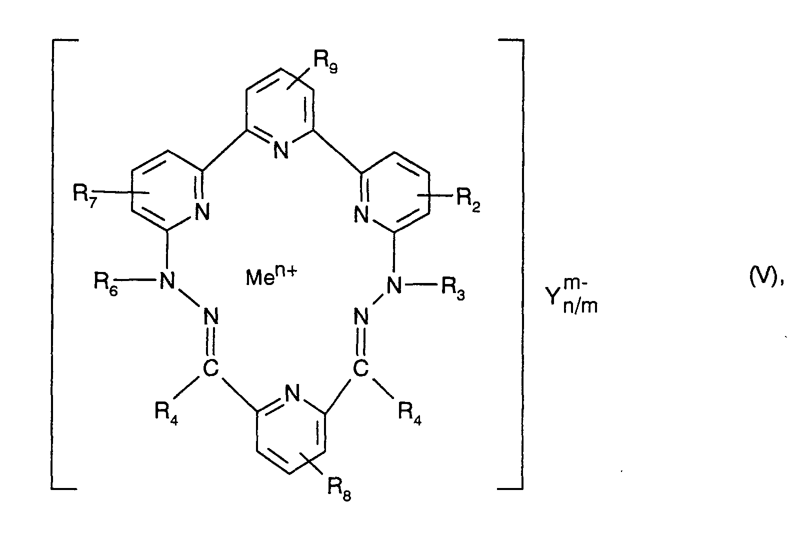 worin
R₂ und R₇ unabhängig voneinander H, C₁-C₄-Alkyl, C₁-C₄-Alkoxy, C₇-C₁₂-Aralkyl, C₆-C₁₆-Aryl, Halogen, C₄-C₁₂-Heteroaryl mit O, S oder N als Heteroatome, C₁-C₄-Alkylthio, Di(C₁-C₄-Alkyl)amino, Sulfonamid oder Carboxamid bedeuten,
R₃ und R₆ unabhängig voneinander H, C₁-C₄-Alkyl, C₇-C₁₂-Aralkyl, C₆-C₁₆-Aryl, Halogen, C₄-C₁₂-Heteroaryl mit O, S oder N als Heteroatome, C₁-C₄-Alkylthio, Di(C₁-C₄-Alkyl)amino, Sulfonamid oder Carboxamid sind,
R₄ für H, C₁-C₂₀-Alkyl, C₅-C₈-Cycloalkyl, C₆-C₁₂-Aryl oder C₇-C₁₂-Aralkyl steht,
wobei die Reste Alkyl, Cycloalkyl, Aralkyl und Aryl unsubstituiert oder mit C₁-C₄-Alkoxy, F, Cl, Br, -CN, C₁-C₄-Alkyl oder -NO₂ substituiert sind,
Me für ein Lanthänidmetall oder Yttrium steht,
Y für ein Anion einer Säure steht,
n die Zahlen 2 oder 3 bedeutet, und
m die Zahlen 1, 2 oder 3 bedeutet,
R₉ einen Rest der Formel VI

-Xₚ-A-X'_{q}-A'ᵣ-Oligo (VI)

darstellt, und R₈ H, C₁-C₄-Alkyl, C₁-C₄-Alkoxy, C₇-C₁₂-Aralkyl, C₆-C₁₆-Aryl, C₄-C₁₂-Heteroaryl mit O, S oder N als Heteroatome, C₁-C₄-Alkylthio, Di(C₁-C₄-Alkyl)amino, Halogenid, Sulfonamid oder Carboxamid bedeutet, oder
R₉ H, C₁-C₄-Alkyl, C₁-C₄-Alkoxy, C₇-C₁₂-Aralkyl, C₆-C₁₆-Aryl C₄-C₁₂-Heteroaryl mit O, S oder N als Heteroatome, C₁-C₄-Alkylthio, Di(C₁-C₄-Alkyl)amino, Halogenid, Sulfonamid oder Carboxamid darstellt und R₈ einen Rest der Formel VI bedeutet,
p, q und r unabhängig voneinander für 0 oder 1 stehen,
X und X' unabhängig voneinander einen unsubstituierten oder mit C₁-C₄-Alkoxy, F, Cl, Br, -CN, C₁-C₄-Alkyl oder -NO₂ substituierten Rest ausgewählt aus der Gruppe bestehend aus C₁-C₂₀-Alkylen, C₂-C₁₂-Alkenylen, C₂-C₁₂-Alkinylen, -(CₓH₂ₓO)_{y}-, worin x gleich einer Zahl von 2 bis 6 und y gleich einer Zahl von 1 bis 20 ist, C₅-C₈-Cycloalkylen, C₆-C₁₂-Arylen und C₇-C₁₂-Aralkylen bedeuten,
A und A' unabhängig voneinander -O-, -S-, -S-S-, -NR₁₂-CO-NR₁₂-, -NR₁₂-CS-NR₁₂-, -NR₁₂-, -NR₁₂-C(O)-O-, -C(O)O-, -C(O)S-, -C(O)NR₁₂-, -C(S)S-, -C(S)O-, -C(S)NR₁₂-, -SO₂NR₁₂-, -SO₂-, -P(O)(OH)O-, -P(S)(SH)S-, -P(S)(SH)O-, -P(S)(OH)O-, -P(O)(SH)S-, -P(O)(OH)S-, -P(O)(SH)O-, -P(O)(OH)-NR₁₂-, -P(S)(SH)-NR₁₂-, -P(S)(OH)-NR₁₂-, -P(O)(SH)-NR₁₂-, -HP(O)O-, -HP(S)S-, -HP(O)NR₁₂- oder -HP(S)NR₁₂- bedeuten, wobei R₁₂ für H oder C₁-C₆-Alkyl steht; und
Oligo eine natürliche, modifizierte oder synthetische Sequenz aus natürlichen, modifizierten oder synthetischen Desoxynukleosiden oder Peptidnukleinsäurebausteinen darstellt, die über eine Nukleinbase, eine internukleotidische Brücke oder einen Zucker gebunden ist und deren innerer Bereich komplementär zu einer Ziel-RNA ist.

Dabei steht q bevorzugt für 1.

Für R₈ und R₉ als Substituent gelten die oben für R₅ bzw. R₁ gegebenen Definitionen und Bevorzugungen entsprechend.

Ziel-RNA bedeutet im Rahmen der vorliegenden Erfindung, dass im Target eine RNA-Sequenz vorliegen muss. Demgemäss können Polyribonukleinsäuren (RNA) vorliegen. Bevorzugt handelt es sich um m-RNA (Boten-RNA), pre-m-RNA (Vorläufer-m-RNA) t-RNA (Transfer-RNA), sn-RNA (small nuclear RNA), r-RNA (ribosomale RNA) und virale RNA. Es können aber auch Mischsequenzen aus RNA und Polydesoxyribonukleinsäuren (DNA) vorliegen, zum Beispiel die Chimären RNA-DNA (Okazaki-Fragment). Die RNA weist soviele Bausteine auf, dass ein Komplex (Doppelstrang) mit dem Oligonukleotid gebildet werden kann.

Im Rahmen der Erfindung ist unter dem inneren Bereich einer Sequenz zu verstehen, dass zum Beispiel bis zu 5, vorzugsweise bis zu 3 und besonders bevorzugt 1 oder 2 der äusseren Nukleotidbausteine der Sequenz nicht mit der Ziel-RNA komplementär sein müssen. Dies kann insofern von Vorteil sein, als der am Ende der Sequenz gebundene Terpyridin-Lanthanid-Komplex beweglicher und damit effizienter sein kann.

Das Oligonukleotid kann teilweise oder vollständig aus zur Ziel-RNA komplementären natürlichen DNA-Bausteinen oder vollständig aus zur Ziel-RNA ebenfalls komplementären unnatürlichen synthetischen Nukleotiden aufgebaut sein, wobei teilweise bedeutet, dass in der Oligonukleotidsequenz zur Ziel-RNA natürliche DNA-Bausteine durch ebenfalls komplementäre unnatürliche synthetische Nukleotide ersetzt sind. Synthetische Bausteine umfassen die Modifikationen natürlicher Bausteine in der Nukleinbase, dem Furanosering und/oder den Brückengruppen der Oligonukleotide. Synthetische Bausteine werden im allgemeinen eingesetzt, um die Komplexbindung in Duplexstrukturen zu verstärken und/oder die Stabilität der Oligonukleotide gegenüber dem durch zum Beispiel Nukleasen verursachten Abbau zu erhöhen. Modifizierte Nukleoside sind im Bereich der "Antisense-Technologie" für die Synthese oder Modifikation von komplementären Oligonukleotiden in grosser Vielzahl bekannt geworden und werden daher hier nicht näher erläutert (siehe zum Beispiel E. Uhlmann et al., Chemical Reviews, Band 90, Nummer 4, Seiten 543 bis 584 (1990).

Als Modifikationen kommen Abwandlungen im Nukleinbaseteil (zum Beispiel Substitutionen, Weglassen von Substituenten), in der Nukleotidbrückengruppe (zum Beispiel Abwandlung der Phosphorsäureestergruppe oder deren Ersatz durch andere Brückengruppen) und im Furanosering (zum Beispiel Substitutionen an der 2'-Hydroxylgruppe, Ersatz des Furanose-O-Atoms, Ersatz des Furanoserings durch mono- oder bicarbacycliche Ringe, Ersatz des Furanoserings durch offenkettige Strukturen) in Frage.

Die Wahl und die Reihenfolge der Bausteine in der Sequenz des Oligonukleotids wird durch die notwendige Duplexbildung mit einer Ziel-RNA bestimmt. Auch die Art und der Ort der Verknüpfung mit dem Terpyridin-Lanthanid-Komplex kann die Wahl und die Reihenfolge der Bausteine beeinflussen.

Die Anzahl der Bausteine in dem Oligonukleotid wird so bemessen, dass eine Hybridisierung mit der Ziel-RNA erfolgt. Die Oligonukleotide können zum Beispiel 5 bis 100, bevorzugt 5 bis 50, besonders bevorzugt 8 bis 30 und ganz besonders 10 bis 25 Bausteine enthalten. Die Paarbildung mit der Ziel-RNA erhöhende Bereiche (paarende Nukleotidbausteine) sind bevorzugt in den mittleren Sequenzfolgen des Oligonukleotids angeordnet, zum Beispiel zwischen den jeweils viertletzten, oder den jeweils drittletzten, oder den jeweils zweitletzten oder den jeweils letzten Bausteinen der Sequenz. Bei einem Oligonukleotid mit zum Beispiel 20 Bausteinen befinden sich paarende Bausteine bevorzugt im Bereich vom vierten bis siebzehnten Baustein.

Die Oligonukleotide sind bevorzugt aus Nukleosiden der Purinreihe und der Pyrimidinreihe aufgebaut. Besonders bevorzugt aus 2'-Desoxy-2-aminoadenosin, 2'-Desoxy-5-methylcytidin, 2'-Desoxyadenosin, 2'-Desoxycytidin, 2'-Desoxyuridin, 2'-Desoxyguanosin und 2'-Thymidin. Ganz besonders bevorzugt sind 2'-Desoxyadenosin (A), 2'-Desoxycytidin (C), 2'-Desoxyguanosin (G) und 2'-Thymidin (T). Modifizierte Bausteine leiten sich bevorzugt von natürlichen Nukleosiden der Purinreihe und der Pyrimidinreihe, besonders bevorzugt von Adenosin, Cytidin, Guanosin, 2-Aminoadenosin, 5-Methylcytosin, Thymidin und den zuvor genannten Desoxyderivaten ab. Bei den Nukleosiden kann es sich auch um 2'-modifizierte Ribonukleoside handeln.

In einer ganz besonders bevorzugten Ausführungsform der Erfindung ist das zu einer Ziel-RNA komplementäre Oligonukleotid aus natürlichen Desoxynukleosiden, besonders bevorzugt aus der Gruppe 2'-Desoxyadenosin (A), 2'-Desoxycytidin (C), 2'-Desoxyguanosin (G), und 2'-Thymidin (T) oder aus komplementären unnatürlichen synthetischen Bausteinen aufgebaut. Im Rahmen der Erfindung sind solche modifizierten Nukleoside besonders bevorzugt, die die Stabilität des Oligonukleotids gegenüber Nukleasen erhöhen.

Das Oligonukleotid kann auch aus Sequenzen von Peptidnukleinsäuren (PNA) bestehen, wobei der Terpyridin-Lanthanid-Komplex bevorzugt an die Nukleinbase, das Amino- oder das Carboxylende gebunden ist. Für den Aufbau der PNA-Sequenz gelten die gleichen Bevorzugungen wie für die Oligonukleotide. Beispiele für PNA's finden sich in Science, Band 254, Seiten 1497 bis 1500.

Der Terpyridin-Lanthanid-Komplex ist vorzugsweise über eine Brückengruppe an N-, -Soder O-Atome in den 3'- oder 5'-Endgruppen in der Oligonukleotidsequenz gebunden. Er kann aber auch an C, N- oder O-Atome von Nukleinbasen in oder am Ende der Sequenz, an 2'-Stellungen des Furanoserings, an O-, S- oder N-Atome in oder am Ende der Sequenz oder an O-, S- oder N-Atome der Nukleotidbrückengruppe in der Sequenz gebunden sein. Die Art der Bindung hängt vom Terpyridin-Lanthanid-Komplex und der Art seiner funktionellen Gruppen ab. Eine Brückengruppe kann zum Beispiel eine umgewandelte funktionelle Gruppe sein, die ihrerseits direkt oder über eine Verbindungsgruppe an den Terpyridin-Lanthanid-Komplex und/oder das Oligonukleotid gebunden sein kann. Die Bindung an das Oligonukleotid kann ionogen und bevorzugt kovalent sein. Der Terpyridin-Lanthanid-Komplex kann auch an das 6'-Kohlenstoffatom eines carbacyclischen Nukleotidanalogen gebunden sein.

Bei X und X' als Bestandteile der Brücke zwischen Terpyridin-Lanthanid-Komplex und Oligonukleotid kann es sich im Falle X₀ oder X₀' um eine direkte Bindung oder im Fall X₁ oder X₁' um eine bivalente, offenkettige oder cyclische Kohlenwasserstoffgruppe mit 1 bis 22 C-Atomen, die ununterbrochen oder mit Resten aus der Gruppe -S-, -NR₁₂-, -C(O)-O-, -C(O)-NR₁₂- unterbrochen ist, oder einen Polyoxaalkylenrest mit 1 bis 12 Oxaalkyleneinheiten und 2 oder 3 C-Atomen im Alkylen handeln. Bei der Kohlenwasserstoffgruppe kann es sich zum Beispiel um lineares oder verzweigtes C₁-C₂₂-Alkylen, bevorzugt C₁-C₁₈-Alkylen, besonders bevorzugt C₁-C₁₂-Alkylen und ganz besonders bevorzugt C₁-C₈-Alkylen; um C₃-C₈-Cycloalkylen, bevorzugt C₅- oder C₆-Cycloalkylen; C₆-C₁₂-Arylen oder um C₇-C₁₂-Aralkylen handeln. Einige Beispiele für bivalente Kohlenwasserstoffgruppen sind Methylen, Ethylen, 1,2- oder 1,3-Butylen, 1,2-, 1,3- oder 1,4-Butylen, 1,2-, 1,3-, 1,4- oder 1,5-Pentylen, 1,2-, 1,3-, 1,4-, 1,5- oder 1,6-Hexylen, 1,2-, 1,3-, 1,4-, 1,5-, 1,6- oder 1,7-Heptylen, 1,2-, 1,3-, 1,4-, 1,5-, 1,6-, 1,7- oder 1,8-Octylen, und die Isomeren von Nonylen, Decylen, Undecylen, Dodecylen, Tridecylen, Tetradecylen, Pentadecylen, Hexadecylen, Heptadecylen, Octadecylen, Nonadecylen und Eicosylen; Cyclopentylen, Cyclohexylen; Naphthylen und besonders Phenylen; Benzylen und Phenylethylen. Einige Beispiele für Polyoxaalkylene sind Ethylenoxy, Bisethylenoxy, Trisethylenoxy, Tetraethylenoxy und 1,2-Propoxy. Besonders bevorzugt sind Brückengruppen, in denen X die Bedeutung C₁-C₃-Alkylen, C₃-Alkinylen, Phenylen oder C₇-Aralkylen, besonders bevorzugt die Bedeutung C₂-C₃-Alkylen oder Phenylen hat. Besonders bevorzugt sind auch Brückengruppen, in denen X' C₁-C₂₀-Alkylen, insbesondere C₁-C₁₀-Alkylen ist.

Die bivalente Gruppe A ist vorzugsweise -NR₁₂-CS-NR₁₂- oder -C(O)NR₁₂-, insbesondere -NH-CS-NH- oder -C(O)NH-.

Bevorzugte Verbindungen der Formel V sind solche, in denen A' nicht vorhanden ist oder -P(O)(OH)O- bedeutet.

Bevorzugte Verbindungen der Formel V sind solche, in denen R₂ und R₇ unabhängig voneinander H oder C₁-C₄-Alkyl bedeuten.

Vorteilhafterweise bedeuten R₃ und R₆ unabhängig voneinander H oder C₁-C₄-Alkyl.

In einer anderen bevorzugten Ausführungsform steht R₄ für H oder C₁-C₂₀-Alkyl.

Die Bevorzugungen im Zusammenhang mit geeigneten Lanthaniden und Anionen sind bereits genannt worden. Sie gelten auch für die Verbindungen der Formel V.

Ein weiterer Gegenstand der vorliegenden Erfindung sind Zwischenprodukte bei der Herstellung von Verbindungen der Formel I. Es sind dies die Verbindungen der Formel II 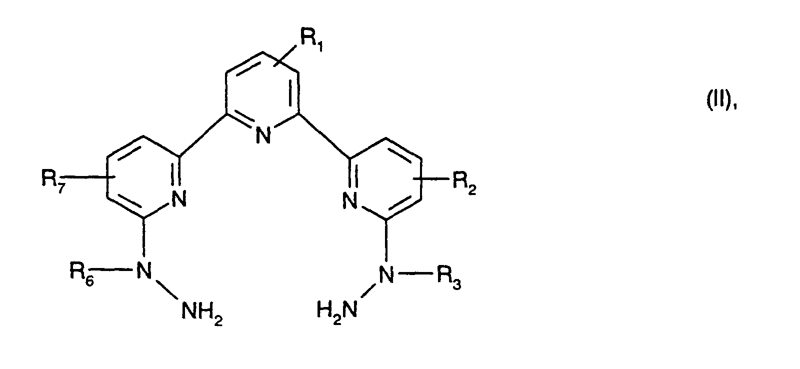 worin
R₁ H, C₁-C₄-Alkyl, C₁-C₄-Alkoxyl C₇-C₁₂-Aralkyl oder C₆-C₁₆-Aryl oder eine monovalente funktionelle Gruppe bedeutet, wobei die funktionelle Gruppe direkt oder über eine Gruppe Z an den Pyridinring gebunden ist und die Gruppe Z für einen gegebenenfalls durch -O-, -S-, -NR₁₂-, -C(O)-O- oder -C(O)-NR₁₂- unterbrochenen Rest ausgewählt aus der Gruppe bestehend aus C₁-C₂₀-Alkylen, C₂-C₁₂-Alkenylen, C₂-C₁₂-Alkinylen, C₅-C₈-Cycloalkylen, C₆-C₁₂-Arylen und C₇-C₁₂-Aralkylen steht,
R₂ und R₇ unabhängig voneinander H, C₁-C₄-Alkyl, C₁-C₄-Alkoxy, C₇-C₁₂-Aralkyl, C₆-C₁₆-Aryl oder Halogen bedeuten, und
R₃ und R₆ unabhängig voneinander H, C₁-C₄-Alkyl, C₇-C₁₂-Aralkyl oder C₆-C₁₆-Aryl sind,
R₁₂ H oder C₁-C₆-Alkyl bedeutet,
wobei die Reste Alkyl, Cycloalkyl, Aralkyl, Aryl und die Gruppe Z unsubstituiert oder mit C₁-C₄-Alkoxy, F, Cl, Br, -CN, C₁-C₄-Alkyl oder -NO₂ substituiert sind.

Weitere Zwischenprodukte bei der Herstellung von Verbindungen der Formel I und ebenfalls ein Gegenstand der vorliegenden Erfindung sind Verbindungen der Formel III, 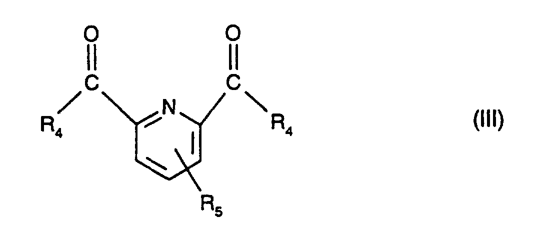 worin
R₅ eine über C₂-C₂₀-Alkylen an den Pyridinring gebundene monovalente funktionelle Gruppe darstellt, wobei die funktionelle Gruppe ausgewählt ist aus der Gruppe bestehend aus -C(O)-OR₁₂, -C(O)-NHR₁₂, -SO₂-R₁₂ und -SO₂-NHR₁₂, wobei R₁₂ H oder C₁-C₆-Alkyl ist, und
R₄ für H oder C₁-C₂₀-Alkyl steht.

Für diese Zwischenprodukte gelten die für das Endprodukt angegebenen Bevorzugungen in entsprechender Weise.

Ein weiterer Gegenstand der vorliegenden Erfindung ist ein Verfahren zur Herstellung der Verbindungen der Formel I, in dem man ein Terpyridin der Formel II 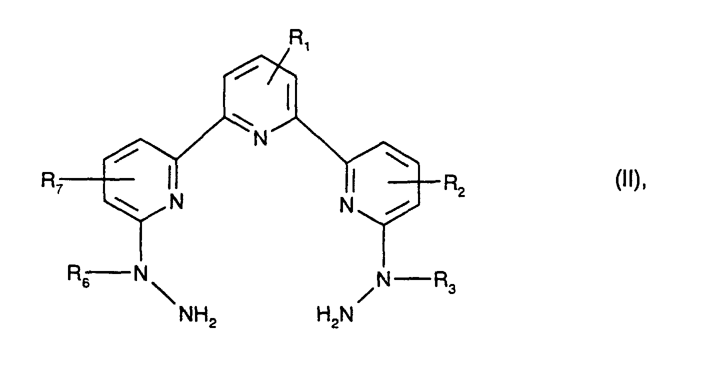 mit einem Pyridindialdehyd oder Pyridindiketon der Formel III 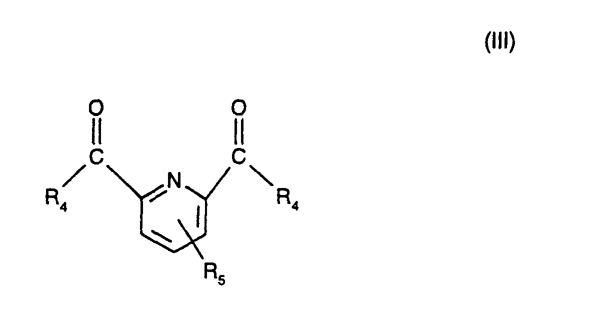 in Gegenwart eines Salzes der Formel IV

Meⁿ⁺(Y^{m-})_{n/m} (IV)

kondensiert, worin R₁, R₂, R₃, R₄, R₅, R₆, R₇, Me, Y, n und m die zuvor, insbesondere bei den Definitionen, Bevorzugungen und Erläuterungen zu der Verbindung der Formel (I), angegebenen Bedeutungen haben.

Das Verfahren kann zum Beispiel so durchgeführt werden, dass man die Verbindungen der Formeln II, III und IV in bevorzugt äquivalenten Mengen in einem Lösungsmittel löst und dann bei erhöhten Temperaturen miteinander umsetzt. Zweckmässig werden Kondensationskatalysatoren mitverwendet, zum Beispiel konzentrierte Mineralsäuren, besonders Salzsäure, oder saure lonenaustauscher. Es kann zweckmässig sein, wasserbindende Mittel zuzugeben, oder das Reaktionswasser aus dem Reaktionsgemisch zu entfernen.

Die Reaktionstemperatur kann zum Beispiel 40 bis 220 °C, bevorzugt 50 bis 150 °C betragen.

Als Lösungsmittel werden vorteilhaft organische polare aprotische Lösungsmittel eingesetzt. Geeignete Lösungsmittel sind zum Beispiel Wasser und polare aprotische Lösungsmittel, die vorteilhaft mit Wasser mischbar sind. Beispiele für solche Lösungsmittel sind Alkohole (Methanol, Ethanol, n- oder i-Propanol, Butanol, Ethylenglykol, Propylenglykol, Ethylenglykolmonomethylether, Diethylenglykol, Diethylenglykolmonomethylether), Ether (Diethylether, Dibutylether, Tetrahydrofuran, Dioxan, Ethylenglykoldimethylether, Ethylenglykoldiethylether, Diethylenglykoldiethylether, Triethylenglykoldimethylether), halogenierte Kohlenwasserstoffe (Methylenchlorid, Chloroform, 1,2-Dichlorethan, 1,1,1-Trichlorethan, 1,1,2,2-Tetrachlorethan, Chlorbenzol), Carbonsäureester und Lactone (Essigsäureethylester, Propionsäuremethylester, Benzoesäureethylester, 2-Methoxyethylacetat, γ-Butyrolacton, δ-Valerolacton, Pivalolacton), N-alkylierte Carbonsäureamide und Lactame (N,N-Dimethylformamid, N,N-Diethylformamid, N,N-Dimethylacetamid, Tetramethylharnstoff, Hexamethylphosphorsäuretriamid, N-Methyl-γ-Butyrolactam, N-Methyl-ε-Caprolactam, N-Methylpyrrolidon,), Sulfoxide (Dimethylsulfoxid, Tetramethylensulfoxid), Sulfone (Dimethylsulfon, Diethylsulfon, Trimethylensulfon, Tetramethylensulfon), tertiäre Amine (Trimethylamin, Triethylamin, N-Methylpiperidin, N-Methylmorpholin, Pyridin), substituierte Benzole (Chlorbenzol, o-Dichlorbenzol, 1,2,4-Trichlorbenzol, Nitrobenzol, Toluol, Xylol) und Nitrile (Acetonitril, Propionitril, Benzonitril, Phenylacetonitril).

Die Metallsalze der Formel IV sind allgemein bekannt und zum grossen Teil käuflich.

Die Herstellung der neuen eine funktionelle Gruppe enthaltenden Verbindungen der Formel II kann analog zu dem von E. C. Constable in Polyhedron, Band 7, Nr. 24, Seiten 2531 bis 2536 (1988) beschriebenen Verfahren erfolgen, wobei funktionelle Gruppen gegebenenfalls mit Schutzgruppen versehen werden.

Die Verbindungen der Formeln II und III mit oder ohne funktionelle Gruppen sind grossenteils bekannt oder sie können nach bekannten beziehungsweise analogen Verfahren hergestellt werden. Verbindungen der Formel III, worin R₄ für H steht, R₅ für C₂-C₁₈-Alkylen-X₅ steht und X₅ -C(O)-OR, -C(O)-NHR, -SO₂-R oder -SO₂-NHR bedeutet, sowie R H oder C₁-C₆-Alkyl ist, sind neu und auf folgende Weise erhältlich: Man alkenyliert unter Palladiumkatalyse einen entsprechenden 3-Halogen-pyridin-1,5-dicarbonsäureester mit einem Alken der Formel CH₂=CH-C₁-C₁₆-Alkylen-carbonsäurester, hydriert die Alkengruppe zum Beispiel katalytisch, reduziert anschliessend zum entsprechenden 1,5-Dihydroxymethylpyridinalkylcarbonsäureester, oxidiert die Hydroxymethylgruppen zu Aldehydgruppen und hydrolysiert gegebenenfalls die Ester- zur Carbonsäuregruppe oder amidiert die Estergruppe zum Carbonsäureamid.

Verbindungen der Formel III, worin R₄ für C₁-C₁₂-Alkyl steht, R₅ für C₂-C₁₈-Alkylen-X₅ steht und X₅ -C(O)-OR, -C(O)-NHR, -SO₂-R oder -SO₂-NHR bedeutet, sowie R H oder C₁-C₆-Alkyl ist, sind neu und auf folgende Weise erhältlich: Man alkenyliert unter Palladiumkatalyse ein entsprechendes mit zum Beispiel Acetyl geschütztes 3-Halogen-1,5-dihydroxymethylpyridin (erhältlich durch Reduktion des entsprechenden 3,5-Dicarbonsäuremethylesters) mit einem Alken der Formel CH₂=CH-C₁-C₁₆-Alkylen-carbonsäurester, hydriert die Alkengruppe zum Beispiel katalytisch, entschützt die Hydroxylgruppen und oxidiert gegebenenfalls zum entsprechenden 3,5-Pyridinaldehyd, dessen Aldehydgruppen mit zum Beispiel Grignardreagenzien C₁-C₁₂-alkyliert werden können, hydrolysiert gegebenenfalls die Esterzur Carbonsäuregruppe oder amidiert die Estergruppe zum Carbonsäureamid, und oxidiert die sekundären Alkoholgruppen zu Ketogruppen.

Ein weiterer Gegenstand der Erfindung ist ein Verfahren zur Herstellung von Verbindungen der Formel V, in dem man eine Verbindung der Formel I
(a) mit einer Verbindung der Formel Vla

   A"-X'-A'_{0 oder 1}-Oligo (VIa)

   worin
   A" eine geeignete monovalente funktionelle Gruppe ist ausgewählt aus der Gruppe bestehend aus -OR₁₀, -SR₁₀, -NCO, -NCS, -NHR₁₁, -C(O)OR₁₁, -C(O)SH, -C(O)NHR₁₁, -C(O)Cl, -C(S)SR₁₁, -C(S)NHR₁₁, -C(S)OR₁₁, -SO₃R₁₁, -SO₂NHR₁₁, -SO₂Cl, -P(O)(OH)₂, -P(O)(OH)-NHR₁₁, -P(S)(SH)₂, -P(S)(SH)-NHR₁₁, -P(S)(OH)₂, -P(S)(OH)-NHR₁₁, -P(O)(SH)₂, -P(O)(SH)-NHR₁₁, -P(O)(OH)H, -P(O)(NHR₁₁)H, -P(S)(SH)H, -P(S)(NHR₁₁)H, -P(S)(OH)H, -P(O)(SH)H, wobei R₁₀ H, -C(O)NH₂, -C(S)NH₂, -C₁-C₆-Alkyl, -CₓH₂ₓ-NH₂, -CₓH₂ₓ-SH oder -(CₓH₂ₓO)_{y}-H und R₁₁ H, -C₁-C₆-Alkyl, -CₓH₂ₓ-NH₂, -CₓH₂ₓ-SH oder -(CₓH₂ₓO)_{y}-H bedeutet und x gleich einer Zahl von 2 bis 6 und y gleich einer Zahl von 1 bis 20 ist,
   X' einen unsubstituierten oder mit C₁-C₄-Alkoxy, F, Cl, Br, -CN, C₁-C₄-Alkyl oder -NO₂ substituierten Rest ausgewählt aus der Gruppe bestehend aus C₁-C₂₀-Alkylen, C₂-C₁₂-Alkenylen, C₂-C₁₂-Alkinylen, -(CₓH₂ₓO)_{y}-, worin x gleich einer Zahl von 2 bis 6 und y gleich einer Zahl von 1 bis 20 ist, C₅-C₈-Cycloalkylen, C₆-C₁₂-Arylen und C₇-C₁₂-Aralkylen bedeutet, A'-O-, -S-, -S-S-, -NR₁₂-CO-NR₁₂-, -NR₁₂-CS-NR₁₂-, -NR₁₂-, -NR₁₂-C(O)-O-, -C(O)O-, -C(O)S- , -C(O)NR₁₂-, -C(S)S-, -C(S)O-, -C(S)NR₁₂-, -SO₂NR₁₂-, -SO₂-, -P(O)(OH)O-, -P(S)(SH)S-, -P(S)(SH)O-, -P(S)(OH)O-, -P(O)(SH)S-, -P(O)(OH)S-, -P(O)(SH)O-, -P(O)(OH)-NR₁₂-, -P(S)(SH)-NR₁₂-, -P(S)(OH)-NR₁₂-, -P(O)(SH)-NR₁₂-, -HP(O)O-, -HP(S)S-, -HP(O)NR₁₂- oder -HP(S)NR₁₂- bedeutet, wobei R₁₂ für H oder C₁-C₆-Alkyl steht; und
   Oligo eine natürliche, modifizierte oder synthetische Sequenz aus natürlichen, modifizierten oder synthetischen Desoxynukleosiden oder Peptidnukleinsäurebausteinen darstellt, die über eine Nukleinbase, eine internukleotidische Brücke oder einen Zucker gebunden ist und deren innerer Bereich komplementär zu einer Ziel-RNA ist, umsetzt, oder
(b) mit einer Verbindung der Formel Vlb

   A"-Oligo (VIb)
worin
A" und Oligo die in (a) beschriebenen Bedeutungen haben, umsetzt.

Das erfindungsgemässe Verfahren zur Herstellung der Oligonukleotidkonjugate kann zum Beispiel so durchgeführt werden, dass man ein gegebenenfalls funktionalisiertes Oligonukleotid in einem Lösungsmittel oder Lösungsmittelgemisch löst und dann den eine geeignete funktionelle Gruppe tragenden Terpyridin-Lanthanid-Komplex zugibt und darauf das Reaktionsgemisch gegebenenfalls unter Rühren ausreagieren lässt. Das gebildete Konjugat kann anschliessend in an sich bekannter Weise gereinigt und wenn gewünscht isoliert werden.

Die Reaktionstemperatur kann zum Beispiel 0 bis 120 °C, bevorzugt 20 bis 80 °C betragen. Besonders bevorzugt wird die Reaktion bei Raumtemperatur durchgeführt.

Ist die Verknüpfung eine Veresterungs-, Umesterungs- oder Amidierungsreaktion, können entsprechende Cabonsäuregruppen zuvor in bekannter Weise aktiviert werden, zum Beispiel durch Reaktion mit Carbodiimiden und N-Hydroxysuccinimid.

Die Reaktanden werden zweckmässig in molaren Verhältnissen eingesetzt. Es kann jedoch ein Überschuss des Katalysators oder des Oligonukleotids verwendet werden.

Zur Reinigung können die üblichen Methoden angewendet werden, vorteilhaft zum Beispiel Dialyse, Elektrophorese, und chromatographische Verfahren wie Hochdruckflüssigkeitschromatographie (HPLC), Umkehr-HPLC, Affinitätschromatographie, lonentauscherchromatographie, und Gelchromatographie.

Die zu verwendenden gegebenenfalls funktionalisierten Oligonukleotide können in an sich bekannter Weise mittels automatisierten Synthesizern hergestellt werden, die käuflich sind. Nukleoside für deren Synthese sind bekannt, teilweise käuflich oder nach anologen Verfahren herstellbar.

Die erfindungsgemässen Terpyridin-Oligonukleotid-Konjugate eignen sich hervorragend zur insbesondere sequenzspezifischen Spaltung von RNA-Sequenzen, wobei durch deren Fähigkeit zur katalytischen Wirkung nur überraschend niedrige Mengen eingesetzt werden müssen.

Ein weiterer Gegenstand der Erfindung ist ein Verfahren zur Spaltung der Phosphatnukleotidbrücke von Ribonukleinsäuren unter physiologischen Bedingungen und unter Einwirkung eines synthetischen Terpyridin-Lanthanid-Komplex, das dadurch gekennzeichnet ist, dass man (a) die Ziel-RNA mit einem Oligonukleotid komplexiert, dessen innere Sequenz komplementär mit der Ziel-RNA ist, und an das ein Terpyridin-Lanthanid-Komplex gebunden ist, und (b) dann reagieren lässt und spaltet.

Das erfindungsgemässe Verfahren kann in vivo durch Verabreichung der Oligonukleotide oder in vitro unter Zusammenführung einer Ziel-RNA und eines erfindungsgemäss zu verwendenden Oligonukleotids durchgeführt werden.

Physiologische Bedingungen sind dem Fachmann geläufig und umfassen zum Beispiel die Durchführung des Verfahrens im wässrigen Medium und in einem pH-Bereich von 5 bis 9, bevorzugt 5 bis 8 und besonders bevorzugt 5 bis 7,5, wobei das wässrige Medium weitere inerte Bestandteile enthalten kann, zum Beispiel Salze von Alkalimetallen oder Erdalkalimetallen, und besonders Puffersysteme.

Das Verfahren kann bei einer Temperatur von zum Beispiel 0 bis 100 °C, bevorzugt 20 bis 50 °C und insbesondere 30 bis 40 °C durchgeführt werden.

Bei dem erfindungsgemässen Verfahren erfolgt die Spaltung bei einer Umesterung der Phosphatbrückenbindung unter Bildung eines Bruchstückes mit einer 2',3'-cyclischen Phosphatendgruppe und eines weiteren Bruchstückes mit einer 5'-Hydroxylendgruppe. Das cyclische Phosphat kann anschliessend weiter hydrolysieren.

Die erfindungsgemässen Terpyridin-Oligonukleotid-Konjugate finden als Arzneimittel Verwendung. Sie weisen zudem eine hohe Stabilität gegenüber einem Abbau durch Nukleasen auf. Besonders überraschend ist ihre ausgezeichnete Paarung mit komplementären Nukleinsäuresträngen vom RNA-Typ. Zusätzlich zeigen sie eine unerwartet hohe zelluläre Aufnahme. Die erfindungsgemässen Oligonukleotide eignen sich daher besonders für die Antisense-Technologie, das heisst zur Inhibition der Expression unerwünschter Proteinprodukte durch die Bindung an geeignete komplementäre Nukleotidsequenzen von mRNA (EP266,099, WO 87/07300 und WO89/08146). Sie können zur Behandlung von Infektionen und Krankheiten zum Beispiel durch die Blockierung der Expression von bioaktiven Proteinen auf der Stufe der Nukleinsäuren (zum Beispiel Onkogene) eingesetzt werden.

Ein anderer Gegenstand der Erfindung betrifft auch die erfindungsgemässen Oligonukleotide zur Anwendung in einem therapeutischen Verfahren zur Behandlung von Krankheiten bei Warmblütern einschliesslich des Menschen durch Inaktivierung von Nukleotidsequenzen im Körper. Die Dosierung bei Verabreichung an Warmblüter von etwa 70 kg Körpergewicht kann zum Beispiel 0,01 bis 1000 mg pro Tag betragen. Die Verabreichung erfolgt vorzugsweise in Form pharmazeutischer Präparate parenteral, zum Beispiel intravenös oder intraperitoneal. Zur parenteralen Verabreichung eignen sich in erster Linie wässrige Lösungen eines wasserlöslichen Wirkstoffes, zum Beispiel eines wasserlöslichen physiologisch unbedenklichen Salzes, oder wässrige Suspensionen solcher Wirkstoffe, welche viskositätssteigernde Mittel wie zum Beispiel Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der Verabreichung durch Zugabe von geeigneten Lösungsmitteln in Lösung gebracht werden. Die erfindungsgemässen Konjugate können auch mittels Inhalation oder in einer liposomalen Verabreichungsform angewendet werden.

Die erfindungsgemässen Konjugate können auch für diagnostische Zwecke oder als molekularbiologische Hilfsmittel als sequenzspezifische Endoribonukleasen Verwendung finden.

Ein weiterer Gegenstand der Erfindung betrifft eine wässrige Zusammensetzung und insbesondere ein pharmazeutisches Präparat auf Basis einer wässrigen Lösung oder Suspension, enthaltend eine wirksame Menge der Verbindungen der Formel V alleine oder zusammen mit anderen Wirkstoffen, Wasser als pharmazeutisches Trägermaterial vorzugsweise in einer signifikanten Menge und gegebenenfalls Hilfsstoffe.

Man kann die pharmakologisch wirksamen erfindungsgemässen Verbindungen in Form von parenteral verabreichbaren Präparaten oder von Infusionslösungen verwenden. Solche Lösungen sind vorzugsweise isotonische wässrige Lösungen oder Suspensionen, wobei diese zum Beispiel bei lyophilisierten Präparaten, welche die Wirksubstanz allein oder zusammen mit einem Trägermaterial, zum Beispiel Mannit, enthalten, vor Gebrauch hergestellt werden können. Die pharmazeutischen Präparate können sterilisiert sein und/oder Hilfsstoffe, zum Beispiel Konservier- Stabilisier-, Netz- und/oder Emulgiermittel, Löslichkeitsvermittler, Salze zur Regulierung des osmotischen Drucks und/oder Puffer enthalten. Die pharmazeutischen Präparate, die gewünschtenfalls weitere pharmakologisch wirksame Stoffe wie zum Beispiel Antibiotka enthalten können, werden in an sich bekannter Weise, zum Beispiel mittels konventioneller Lösungs- oder Lyophilisierungsverfahren, hergestellt, und enthalten etwa 0,1 % bis 90 %, insbesondere von etwa 0,5 % bis etwa 30 %, zum Beispiel 1 % bis 5 % Aktivstoff(e).

In den Zeichnungen sind beispielhaft die Strukturen von Hybriden aus einem Antisense-Oligonukleotid und einem Substrat-RNA-Molekül dargestell.

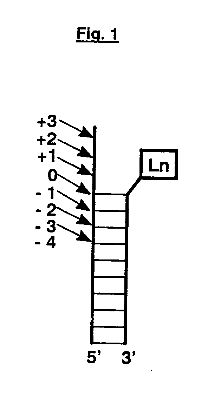

Fig. 1 zeigt schematisch ein Hybrid aus einer Substrat-RNA (mit "5' " bezeichnete Linie) und einem Antisense-Oligonukleotid (mit "3' " bezeichnete Linie), an das erfindungsgemäss ein Komplex (mit "Ln" bezeichnet) gebunden ist (sog. Konjugat). Die angegebene Numerierung bezieht sich auf die Nukleotidbausteine der Substrat-RNA, wobei die Numerierung so erfolgt, dass das Nukleotid der Substrat-RNA, das komplementär zu dem Nukleotid des Antisense-Oligonukleotides ist, an das der Komplex gebunden ist, als "0" bezeichnet wird. Die weitere Numerierung erfolgt dann jeweils aufsteigend (+1, +2 usw.) in 3'-Richtung bzw. absteigend (-1, -2 usw.) in 5'-Richtung der Substrat-RNA.

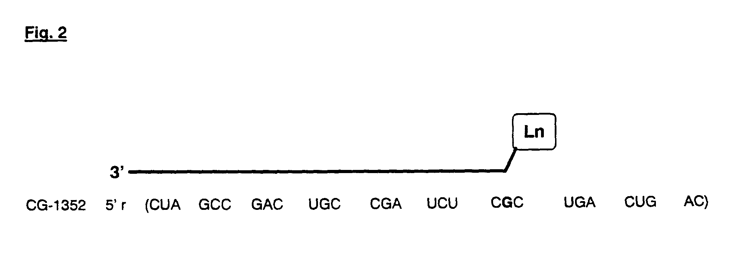

Fig. 2 zeigt schematisch ein Hybrid aus einer konkreten Substrat-RNA (CG-1352, siehe Beispiele) und einem erfindungsgemässen Antisense-Oligonukleotid-Konjugat (mit "3' ") bezeichnete Linie. Das fettgedruckte Nukleotid der Substrat-RNA (hier: G) ist komplementär zu demjenigen Nukleotid des Antisense-Oligonukleotid-Konjugates, an das der Komplex gebunden ist.

Die nachfolgenden Beispiele erläutern die Erfindung.

### A. Herstellung von Ausgangsverbindungen für die Terpyridin-Lanthanid-Komplexe

### Beispiel A1: Herstellung von Terpyridin-bis-hydrazino-Verbindungen

(a) Zu einer Lösung von 6-Acetyl-2-brompyridin (100 mMol) in 200 ml Methanol werden unter Kühlung mit einem Eisbad 40 ml einer 2 N wässrigen Kaliumhydroxid-Lösung gegeben. Nach Zugabe des entsprechend substituierten Benzaldehyds (400 mMol) wird das Kühlbad entfernt und das Gemisch 4 Stunden bei Raumtemperatur weitergerührt. Das Produkt wird abfiltriert, dreimal mit Wasser und zweimal mit kaltem Methanol gewaschen und am Hochvakuum getrocknet.
   Nach dieser Vorschrift werden die Verbindungen a.1 (R₁: Phenyl-4-OCH₃; MS 317.7), a.2 (R₁: Phenyl-4-NO₂; MS 333.6), a.3 (R₁: Phenyl-3-NO₂, MS 334), a.4 (R₁: Phenyl-2-NO₂, MS 334) und a.5 (R₁: Phenyl) hergestellt. 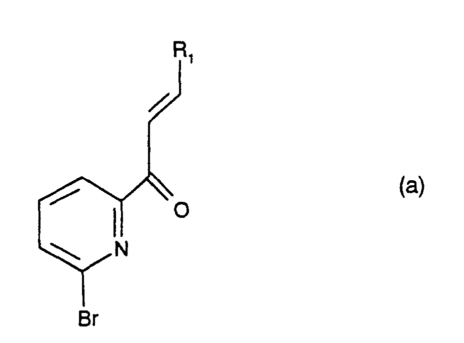
(b) Die α,β-ungesättigte Carbonylverbindung aus (a) (30 mMol), 1-(2-Brompyridylcarbonylmethyl)pyridinjodid (12,1 g, 30 mMol) und Ammoniumacetat (13,9 g, 180 mMol) werden in einem Kolben vorgelegt und mit 100 ml Essigsäure versetzt. Das Gemisch wird am Rückfluss gekocht. Nach 2 Stunden wird auf Raumtemperatur abgekühlt, abfiltriert und das so erhaltene Produkt am Hochvakuum getrocknet.
   Nach dieser Vorschrift werden die Verbindungen b.1 (R₁: Phenyl-4-OCH₃; MS 497.1), b.2 (R₁: Phenyl-4-NO₂; MS 512) ,b.3 (R₁: Phenyl-3-NO₂, MS 513), b.4 (R₁; Phenyl-2-NO₂, MS 512) und b.5 (R₁: Phenyl) hergestellt.
   Zu einer Lösung von Titantetrachlorid (30 mMol) in 75 ml Tetrahydrofuran (abs.) wird bei Raumtemperatur unter Argon-Atmosphäre portionenweise Lithiumaluminiumhydrid (22 mMol) gegeben. Die erhaltene Suspension wird 20 Minuten bei Raumtemperatur gerührt und anschliessend auf 0°C gekühlt. Die Verbindung b.2 (10 mMol) wird zugegeben und die Suspension 30 Minuten bei Raumtemperatur gerührt. Nach vorsichtiger tropfenweise Zugabe von 50 ml Wasser bei 0°C werden 25 ml einer 25 % igen wässsrigen Ammoniaklösung zugegeben. Das Gemsich wird mit 150 ml Chloroform versetzt und über Celite filtriert. Die wässrige Phase wird abgetrennt und dreimal mit Chloroform extrahiert. Alle organischen Phasen werden vereinigt, einmal mit Wasser gewaschen, über Natriumsulfat getrocknet und konzentriert. Nach dieser Vorschrift wird die Verbindung b.6 (R₁: Phenyl-4-NH₂; MS 482.5) hergestellt. Analog werden die Verbindungen b.7 (R₁: Phenyl-3-NH₂, MS 482) und b.8 (R₁: Phenyl-2-NO₂, MS 482) hergestellt. 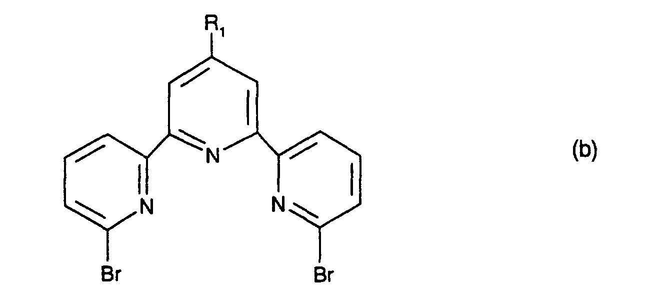
(c) Die entsprechende Dibromoterpyridin-Verbindung aus (b) (10 mMol) wird in 30 ml Methylhydrazin gelöst und 17 Stunden unter Rückfluss erhitzt. Nach Abkühlen auf Raumtemperatur wird eingeengt und der Rückstand in 20 ml Methanol aufgenommen. Das Produkt wird abfiltriert und im Hochvakuum getrocknet.

Nach dieser Vorschrift werden die Verbindungen c.1 (R₁: Phenyl-4-OCH₃; MS 427), c.2 (R₁: Phenyl-4-NH₂; MS 412.5), c.3 (R₁: Phenyl-3-NH₂, MS 412), c.4 (R₁: Phenyl-2-NH₂, MS 412) und c.5 (R₁: Phenyl) hergestellt.

Die Methoxyverbindung c.1 (10 mMol) wird in 100 ml Chloroform suspendiert und unter Kühlen mit dem Eisbad während 20 Minuten mit einer 1 molaren Lösung von Bortribromid (50 mMol) in Methylenchlorid versetzt. Die Suspension wird 5 Tage unter Rückfluss erhitzt. Nach Abkühlen auf Raumtemperatur wird auf 300 ml Eiswasser gegossen, mit 200 ml 2 N wässriger Salzsäure angesäuert. Nach Extraktion mit Ether (2 mal) wird die wässrige Phase mit 10 % iger wässriger Natriumcarbonat-Lösung auf pH 9.0 gestellt und 30 Minuten gerührt. Das ausgefallene Produkt c.6 (R₁: Phenyl-4-OH; MS 413.5) wird abfiltriert und im Hochvakuum getrocknet.

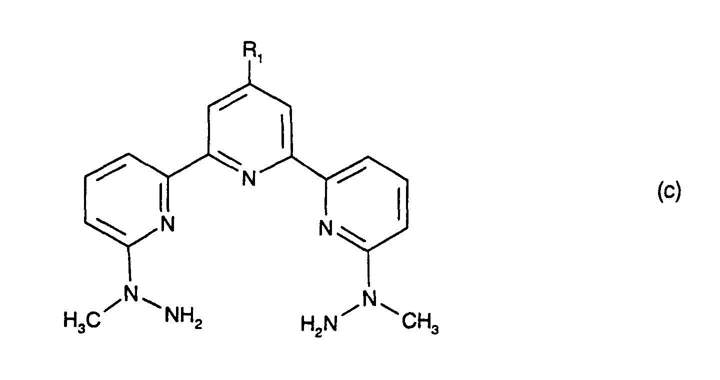

### Beispiel A2: Herstellung von 3-[4'-(2',6'-Diformylpyridin)]propionsäure

(a) 3,5 g 4-Brompyridin-2,6-carbonsäuredimethylester, 390 mg Tritolylphosphin, 9,3 ml Acrylsäuretert.butylester, 7,1 ml Triethylamin, 30 ml Dimethylformamid und 287 mg Palladiumacetat werden gemischt und auf 110°C erwärmt. Nach 90 Minuten wird das Reaktionsgemisch auf Raumtemperatur abgekühlt, mit Ether/Methylenchlorid (1:1) verdünnt und mit NH₄Cl/H₂O ausgeschüttelt. Die organische Phase wird mit Na₂SO₄ getrocknet, am Rotationsverdampfer eingeengt und im Hochvakuum getrocknet.

| | C | H | N |
|---|---|---|---|
| berechnet | 59,81 | 5,96 | 4,36 |
| gefunden | 59,8 | 6,0 | 4,1 |

250 mg Palladium auf Aktivkohle (5 %) und 2,5 g der oben erhaltenen Verbindung werden in 250 ml Methanol gelöst und über Nacht bei Raumtemperatur unter H₂-Atmosphäre hydriert. Das Produkt wird durch Hyflo filtriert, das Filtrat wird am Rotatationsverdampfer eingeengt und bei Raumtemperatur im Hochvakuum getrocknet.

| | C | H | N |
|---|---|---|---|
| berechnet | 59,43 | 6,55 | 4,33 |
| gefunden | 59,3 | 6,6 | 4,3 |

5,0 g der oben erhaltenen Verbindung werden in 50 ml Methanol und 50 ml Tetrahydrofuran gelöst. Nach Abkühlung auf 0°C werden 1,1 g NaBH₄ dazugegeben. Nach 50 Minuten werden weitere 1,1 g NaBH₄ dazugegeben und nach 130 Minuten weitere 0,5 g NaBH₄ dazugegeben. Nach insgesamt 165 Minuten wird auf Raumtemperatur erwärmt. Es wird auf 0°C abgekühlt. Nach 3,5 Stunden werden nochmals 1,1 g NaBH₄ dazugegeben. Nach 6 Stunden wird auf ein Volumen von 60 ml eingeengt. Danach wird eine gesättigte Ammoniumchlorid-Lösung zugetropft, viermal mit CH₂Cl₂ extrahiert, die organischen Phasen werden einmal mit Ammoniumchlorid-Lösung gewaschen, mit Na₂SO₄ getrocknet, abfiltriert und eingeengt.

| | C | H | N |
|---|---|---|---|
| berechnet | 62.90 | 7.92 | 5.24 |
| gefunden | 63.0 | 7.9 | 5.2 |

19,8 g der oben erhaltenen Verbindung werden in 300 ml Dioxan gelöst. Anschliessend werden 16,2 g Selendioxid dazugegeben. Das Reaktionsgemisch wird auf 100°C erwärmt und gerührt, nach 45 Minuten wird auf Raumtemperatur abgekühlt. Nach weiteren zwei Stunden Rühren wird das Reaktionsgemisch filtriert und am Rotationsverdampfer eingeengt.

| | C | H | N |
|---|---|---|---|
| berechnet | 63,87 | 6,51 | 5,32 |
| gefunden | 64,14 | 6,53 | 5,43 |

4,7 g der oben erhaltenen Verbindung werden in 17,2 ml eiskalte Trifluoressigsäure gegeben. Nach der Umsetzung zur Säure wird das Gemisch bei 0°C eingeengt.

| | C | H | N |
|---|---|---|---|
| berechnet | 57,97 | 4,38 | 6,76 |
| gefunden | 57,55 | 4,21 | 6,61 |

(b) 5 g 4-Brompyridin-2,6-dicarbonsäuredimethylester werden in 175 ml Tetrahydrofuran bei Raumtemperatur gelöst. Anschliessend werden 75 ml Methanol zugegeben. Man kühlt auf 0°C ab, gibt portionenweise während 45 Minuten 3,44 g Natriumborhydrid hinzu und lässt auf Raumtemperatur erwärmen. Nach 1 Stunde werden 30 ml Aceton innerhalb von 10 Minuten zugetropft. Man erwärmt das Reaktionsgemisch während 1 Stunde unter Rückfluss, Das Reaktionsgemisch wird anschliessend am Rotationsverdampfer zur Trockne eingeengt. Der Rückstand wird bei Raumtemperatur in 50 ml Pyridin eingerührt. Dazu werden 0,1 g 4-Dimethylaminopyridin gegeben, anschliessend wird auf 0°C abgekühlt. Innerhalb von 30 Minuten werden 34,4 ml Essigsäureanhydrid zugetropft. Man lässt die Suspension auf Raumtemperatur erwärmen. 50 ml Tetrahydrofuran werden zugegeben. Nach Rühren über Nacht bei Raumtemperatur wird das Reaktionsgemisch filtriert und zweimal mit je 50 ml Tetrahydrofuran gewaschen. Das Filtrat wird am Rotationsverdampfer konzentriert. Durch Kristallisation erhält man 4-Brom-2,6-di(acetoxymethyl)pyridin. (Schmelzpunkt: 66-69°C).

0,982 g 4-Brom-2,6-di(acetoxymethyl)pyridin, 1,5 g 3-(Tributylstannyl)-acrylsäureethylester und 176 mg Palladiumtetrakis(triphenylphosphin) werden in 25 ml Dioxan gelöst und auf 90°C erwärmt. Nach 90 Minuten wird das Reaktionsgemisch abgekühlt. Das feste Produkt wird abgetrennt und aus Hexan/Essigester umkristallisiert.
MS 321 (M⁺)

2,74 g der oben erhaltenen Verbindung und 70 mg Wilkinsonskatalysator werden in 150 ml Benzol gelöst. 12,2 ml Triethylsilan werden zugegeben und die Lösung am Rückfluss erhitzt. 270 mg Katalysator Triethylsilan im Überschuss werden portionenweise innerhalb von einer Stunde zugegeben. Das Produkt wird chromatographisch gereinigt. MS 323.

267 mg Natrium werden in 50 ml Ethanol gelöst. 7,2 ml dieser Lösung werden zu einer Lösung von 1,845 g der oben erhaltenen Verbindung in 35 ml Ethanol gegeben. Nach 2,5 Stunden Rühren bei Raumtemperatur wird das Reaktionsgemisch durch Kieselgel filtriert und das Filtrat zur Trockne eingeengt. Das Produkt wird über Nacht im Hochvakuum getrocknet. NMR (CDCl₃) δ 7.0 (2H,s), 4,7 (4H,s), 4,1 (2H,q), 2,9 (2H,t), 1,2 (3H,t)

1,27 g der oben erhaltenen Verbindung werden in 30 ml Dioxan gelöst. Dazu werden 714 mg Selendioxid gegeben. Das Reaktionsgemisch wird erwärmt und nach 2 Stunden durch Watte filtriert. Das Filtrat wird zur Trockne eingeengt. Der Rückstand wird in Essigester/Methylenchlorid (5 %) aufgenommen und über Kieselgel filtriert. ¹H NMR (CDCl₃)δ 10,1 (2H,s), 8,0 (2H,s), 4,1 (2H,q), 3,1 (2H,t), 2,7 (2H,t), 1,2 (3H,t)

Zu einer Lösung von 45 ml Ether und 350 mg der oben erhaltenen Verbindung werden bei 0°C 13 ml einer zuvor bereiteten Lösung (0,949 g Kupferbromid.Dimethylsulfid in 10ml Ether, auf 0°C abgekühlt, 5,9 ml Methyllithium zugegeben) gegeben. Nach 5,5 Stunden Rühren bei Raumtemperatur wird auf 0°C abgekühlt. Es werden 2 ml Eisessig in 8 ml Ether zugegeben und 8 ml Ethanthiol. Nach Rühren über Nacht bei Raumtemperatur werden 60 ml Wasser zugefügt und viermal mit Methylenchlorid ausgeschüttelt. Die organische Phase wird mit Na₂SO₄ vorgetrocknet, filtriert, am Rotationsverdampfer eingeengt und durch Chromatographie gereinigt. MS 266 (M+H)⁺

Zu einer Lösung von 4,5 ml Methylenchlorid + Oxalylchlorid werden bei -78°C 0,5 ml DMSO zugegeben. Nach 15 Minuten wird diese Lösung zu einer Lösung von 193 mg der oben erhaltenen Verbindung in 4 ml Methylenchlorid gegeben. Nach zwei Stunden bei -78°C werden 1,5 ml Trieethylamin zugegeben. Nach 30 Minuten Rühren bei 0°C werden 15 ml Wasser zugegeben, und mit Diethylether viermal ausgeschüttelt. Die organische Phase wird mit Na₂SO₄ vorgetrocknet, am Rotationsverdampfer eingeengt und durch Chromatographie gereinigt.

| | C | H | N |
|---|---|---|---|
| berechnet | 63,87 | 6,51 | 5,32 |
| gefunden | 63,96 | 6,55 | 5,45 |

0,464 g der oben erhaltenen Verbindung und 5 ml 4 N HCI werden zusammen auf 50°C erwärmt. Nach 90 Minuten wird das Reaktionsgemisch auf Raumtemperatur abgekühlt und mit Eiswasser verdünnt. Man erhält das kristalline Produkt.

| | C | H | N |
|---|---|---|---|
| berechnet | 61,27 | 5,57 | 5,95 |
| gefunden | 61,2 | 5,5 | 6,2 |

### Beispiel A3: Herstellung von weiteren Terpyridin-bis-hydrazino-Verbindungen und 2,6-Dicarbonylpyridin-Verbindungen

1. Herstellung der 2,6-Dicarbonyl-Verbindung (f)
(a) Herstellung der Verbindung (d): 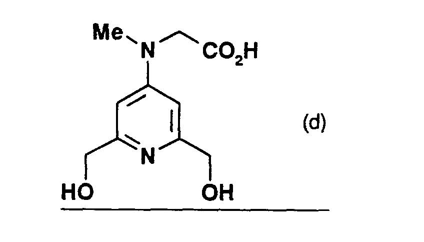
   0.3 g (1.4 mmol) 2,6-Di(hydroxymethyl)-4-brompyridin, 0.25 g (2.8 mmol) Sarccosin und 0.11 g (2.8 mmol) NaOH werden zu einer Lösung aus 15 ml Methanol und 15 ml Wasser gegeben. Es wird unter Stickstoff bei einem Druck von 250 bar gearbeitet. Man erhitzt auf 100° C und lässt solange kochen, bis kein 2,6-Di(hydroxymethyl)-4-brompyridin mehr festellbar ist. Nach zwei Tagen ist die Reaktion zu Ende. Das braune Reaktionsgemisch wird eingeengt und das Produkt chromatographisch gereinigt (Laufmittel: 20 - 80% MeOH / CH₂Cl₂). Man erhält ein gelbes Oel.
   ¹H-NMR (MeOH): δ 6.6 (2H)s; 4.5 (4H)s; 3.8 (2H)s; 3.1 (2H)s; 3.0 (3H)s.
(b) Herstellung der Verbindung (e): 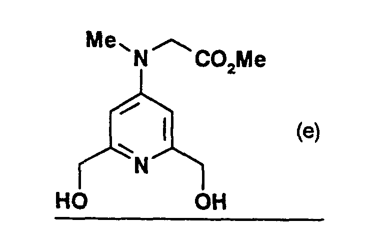
   Es wird eine Suspension von 5,4 g des wie oben erhaltenen Rohproduktes (d) in 450 ml Methanol hergestellt und zum Sieden erhitzt. Dazu werden 6 ml konzentrierte Schwefelsäure hinzugegeben, so dass man eine klare Lösung erhält. Diese wird 3 Stunden Stunden unter Rückfluss erhitzt. Man lässt die Lösung abkühlen, gibt 5 g Kaliumcarbonat dazu und lässt 10 Minuten rühren. Der vorhandene Feststoff wird abfiltriert und das Lösungsmittel am Rotationsverdampfer entfernt. Anschliessend wird das Rohprodukt über eine Kieselgelsäule (Kieselgel 44g; Laufmittel 1 : 9 : 50
   Essigsäure/Methanol/Methylenchlorid) gereinigt. Man erhält einen gelben kristallinen Feststoff.
   ¹H-NMR (MeOD): δ 6,8 (2 H)s; 4,6 (4H)s; 3,7 (2 H)s; 3,7 (3 H)s; 3.2 (3H)s.
(c) Herstellung der Verbindung (f): 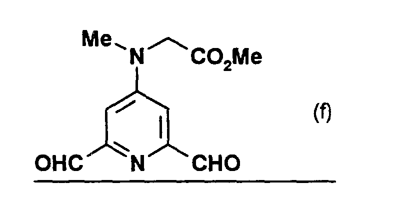
   Eine Lösung der wie oben erhaltenen Verbindung (e) (0,557 g; 2,31 mmol) in 8 ml Pyridin/Dioxan (1:1) wird zum Sieden erhitzt. Zu der entstandenen gelben Lösung werden 1,54 g Selendioxid (13,7 mmol) hinzugegeben und 4 Stunden lang unter Rückfluss erhitzt (Laut DC ist kein Edukt mehr vorhanden). Man lässt auf Raumtemperatur abkühlen, nimmt es in ≈ 100 ml Aceton/Methylenchlorid (1 : 9) auf, filtriert es über eine kleine Silicagelsäule und wäscht mit dem gleichen Lösungsmittel nach. Die erhaltene klare gelbe Lösung wird am Rotationsverdampfer eingengt, und das Rohprodukt wird mittels Säulenchromatographie gereinigt (Laufmittel Aceton/Methylenchlorid 1:40). Man erhält einen weissen Feststoff.
   ¹H-NMR (CDCl₃): δ 10,1 (2H)s; 7,3 (2H)s; 4,2 (2H)s; 3,8 (3H)s; 3,2 (3H)s.

2. Herstellung der 2,6-Dicarbonylpyridin-Verbindung (k)
(a) Herstellung der Verbindung (g): 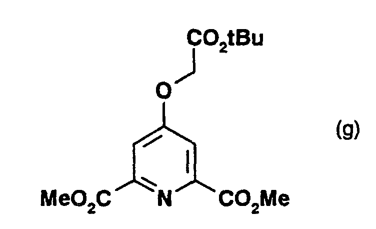
   Es werden 580 mg Kaliumtertiärbutylat (5,17 mmol) in 20 ml Dimethylsulfoxid in einem 100 ml Sulvierkolben unter Argon vorgelegt und portionsweise 1 g Cheliclamsäuredimethylester (4,7 mmol) hinzugegeben (leicht exotherme Reaktion). Nach 10 Minuten werden 1,04 ml Bromessigsäuretertiärbutylester (7,05 mmol) in 2 ml Dimethylsulfoxid zur Lösung langsam hinzugetropft und 3 Stunden rühren gelassen. Nachdem mit Eiswaser gequenscht worden ist, wird das Reaktionsgemisch dreimal mit 20 ml Diethylether ausgeschüttelt. Die vereinigten Etherphasen werden einmal mit 30 ml Wasser gegengewaschen und anschliessend mit Natriumsulfat getrocknet. Die Lösung wird am Rotationsverdampfer eingeengt und am Hochvakuum getrocknet. Man erhält weiss/gelbliche Kristalle, die über eine Kieselgelsäule (70 g Kieselgel 60 F, Merck 9385; Laufmittel Methanol/Dichlormethan 1 : 100) gereinigt werden. Man erhält weisse Kristalle.
   ¹H-NMR (CDCl₃): δ 7,8 (2H)s; 4,7 (2H)s; 4,0 (6H)s; 1,5 (9H)s.
(b) Herstellung der Verbindung (h): 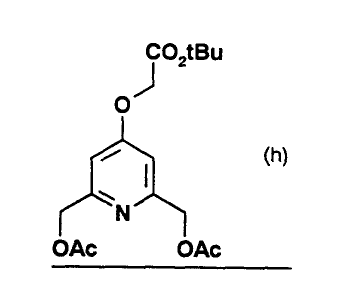
   1.17 g (3.6 mmol) der wie oben hergestellten Verbindung (g) werden in 65 ml Dimethoxyethan gelöst und auf 0° C abgekühlt. Daraufhin werden 680 mg (18 mmol) NaBH₄ in kleinen Portionen zugegeben. Es wird während 15 Minuten bei 0° C gerührt. Anschliessend lässt man das Reaktionsgemisch auf Raumtemperatur erwärmen. Nach 3 Stunden wird das Reaktionsgemisch wieder auf 0° C abgekühlt. Es werden 12 ml Aceton zugegeben und danach 15 Minuten gerührt. Das Gemisch wird auf Raumtemperatur erwärmt und filtriert. Man engt das Lösungsmittel am Rotionsverdampfer ein und trocknet anschliessend am Hochvakuum. Das rohe Produkt aus der ersten Reaktion wird in 55 ml Pyridin gelöst und auf 0° C abgekühlt. Es werden 6.8 ml (72 mmol) Essigsäureanhydrid und 44 mg (0.36 mmol) Dimethylamionpyridin zugegeben. Man lässt das Gemisch auf Raumtemperatur erwärmen. Nun wird mit Wasser verdünnt und dreimal mit Diethylether ausgeschüttelt. Die organischen Phasen werden mit Na₂SO₄ getrocknet und am Rotionsverdampfer eingeengt. Anschliessend wird am Hochvakuum getrocknet. Es bleibt ein braunes Oel zurück. Das Produkt wird mittels Säulenchromatographie gereinigt (100 g Kieselgel; Laufmittel: 1% Methanol in CH₂Cl₂). Man erhält das reine Produkt.
   ¹H-NMR (CDCl₃): δ 6.8 (2H)s; 5.2 (4H)s; 4.6 (2H)s;
(c) Herstellung der Verbindung (i): 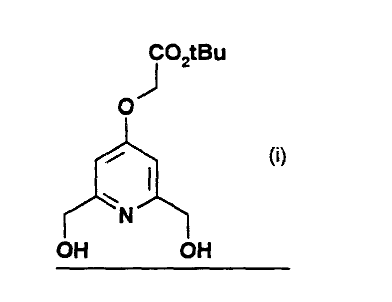
   170 mg (0,48 mmol) des wie oben erhaltenen Eduktes (h) werden in 6,5 ml Methanol gelöst und auf 0 °C abgekühlt. Es werden 1,7 ml 32 %ige Ammoniaklösung hinzugegeben und 1,5 Stunden rühren gelassen. Das Reaktionsgemisch wird am Rotationsverdampfer eingeengt und am Hochvakuum getrocknet. Man erhält ein gelbes Gel, welches mittels Säulenchromatographie (20 g Kieselgel 60 F, Merck No 9385; Laufmittel
   Methanol/Dichlormethan 1 : 15) gereinigt wird. Als Endprodukt liegt ein weisser Feststoff vor.
   ¹H-NMR (CD₃OD): δ 7,0 (2H)s; 4,8 (2H)s; 4,5 (4H)s; 1,5 (9H)s.
(d) Herstellung der Verbindung (j): 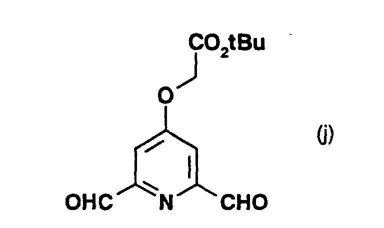
   In einem 25 ml Spitzkolben werden 260 µl Oxalylchlorid(3,0 mmol) in 6 ml Dichlormethari unter Argon gelöst und auf -78 °C abgekühlt. Anschliessend werden 370 µl Dimethylsulfoxid (5,25 mmol) hinzugegeben. Dle Reaktionsmischung lässt man 15 Minuten bei -78 °C rühren. Nun gibt man eine Lösung der wie oben erhaltenen Verbindung (i) (200 mg; 0,75 mmol) in 2 ml Dichlormethan/200 µl Dimethylsulfoxid hinzu(Temperaturkontrolle!). Nachdem man 2 Stunden bei -78 °C rühren gelassen hat, wird mit 1,04 ml Triethylamin(2,5 mmol) in 2 ml Dichlormethan gequenscht. Man lässt noch 15 Minuten lang bei 0 °C rühren. Das Reaktionsgemisch wird am Rotationsverdampfer eingeengt und unter Hochvakuum getrocknet. Man erhält braune Kristalle, die mittels Kieselgel 60 F filtriert werden (Lösungsmittel:Hexan:Essigsäure ethylester 2:1).
   ¹H-NMR (CDCl₃): δ 10,1 (2H)s; 7,6 (2H)s; 4,7 (2H); 1,5 (9H)s.
(e) Herstellung der Verbindung (k): 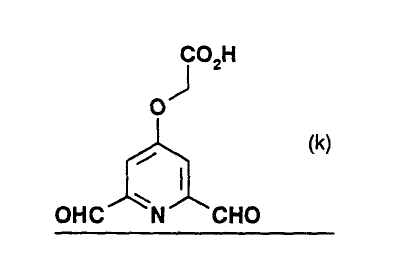
   65 mg der wie oben erhaltenen Verbindung (j) werden in 14 ml 4N Salzsäurelösung bei Raumtemperatur 1,5 Stunden verrührt. Das Reaktionsgemisch wird zur Trockene am Rotationsverdampfer eingeengt und unter Hochvakuum getrocknet. Man erhält als Rohprodukt leicht gelbliche Kristalle.
   ¹H-NMR (CD₃OD): δ 7,4 (2H)s; 5,1 (2H)s.

3. Herstellung der Terpyridin-bis-hydrazino-Verbindung (o)
(a) Herstellung der Verbindung (m): 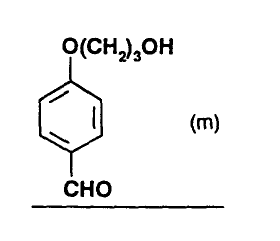
11.49 g (205 mmol) KOH werden pulverisiert. Dieses wird zusammen mit 20 g (164 mmol) 4-Hydroxybenzaldehyd und 1.66 g (4.09 mmol) Aliquat 336 vorgelegt. Man rührt mit einem Halbankerrührer, kühlt im Eisbad ab und tropft vorsichtig 14.24 ml (164 mmol) 3-Brompropanol zu. Das Reaktionsgemisch wird auf 100° C erwärmt. Die dunkelbraune Suspension lässt man über Nacht bei 100° C unter Argon weiterrühren. Dem Reaktionsgemisch werden 250 ml CH₂Cl₂ zugegeben. Man lässt weiterrühren. Die erhaltene Suspension wird über Hyflo filtriert, eingeengt und am Hochvakuum getrocknet. Das Rohprodukt wird in zwei Teilen mit Flashsäulen chromatographisch gereinigt (Laufmittel: 2% THF / CH₂Cl₂), wodurch man das Endprodukt erhält.
   ¹H-NMR (CDCl₃): δ 9.9 (1H)s; 7.8 (2H)d; 7.0 (2H)d; 4.2 (2H)t; 3.9 (2H)m; 2.1 (2H)m; 1.9 (1H)s.

(b) Herstellung der Verbindung (n): 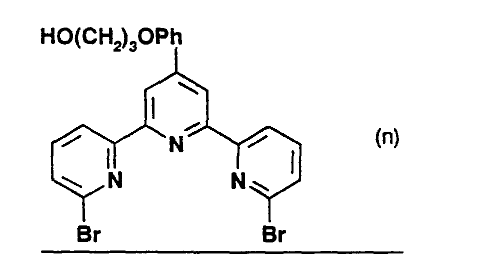
13 g der wie oben erhaltenen Verbindung (m) (0,072 mol), 28,86 g 2-Acetyl-6-brompyridin (0,144 mol), 63,72 g Acetamid(1,08 mol) und 41,58 g Ammoniumacetat (0,54 mol) werden in einen Kolben vorgelegt und 2 Stunden bei 180 °C rühren gelassen. Die braune Suspension lässt man auf 120 °C abkühlen und tropft eine Lösung von 140 g Natriumhydroxidplätzchen in 300 ml Wasser dazu. Das Reaktionsgemisch lässt man 2 Stunden lang kochen. Es bildet sich ein dunkelbrauner Gummi, der von dem überstehenden Lösungsmittel mittels Dekantieren befreit und noch einmal mit Wasser gewaschen wird. Die schwarze gummiartige Substanz wird in sowenig Eisessig wie nötig gelöst. In die heisse Lösung wird die äquivalente Menge Bromwasserstoff (48 % in Wasser) gegeben und über Nacht stehen gelassen. Die hellgelben Kristalle werden abgenutscht, mit Wasser versetzt und mit 4 normaler Kaliumhydroxidlösung auf den pH-Wert 7-8 eingestellt. Die gelbe Suspension wird dreimal mit Dichlormethan extrahiert. Die organische Phase wird über Watte filtriert,am Rotationsverdampfer eingeengt und unter Hochvakuum getrocknet. Das Rohprodukt wird mit Ethanol umkristallisiert. Man erhält gelbe Kristalle.

| | | |
|---|---|---|
| MS | Peak berechnet | 543 m/z (M + H⁺) |
| | Peak gefunden | 542 m/z (M + H⁺) |

(c) Herstellung der Verbindung (o): 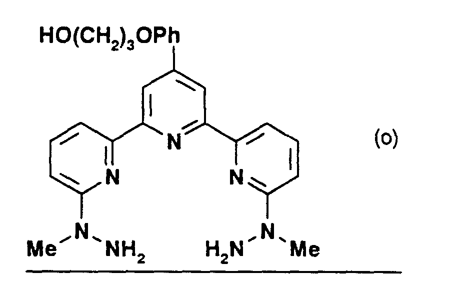
6,38 g der wie oben erhaltenen Verbindung (n) werden in 88 ml Methylhydrazin vorgelegt. Es wird auf 85°C erwärmt und 2 Stunden rühren gelassen. Nach Abkühlung werden 150 ml MeOH zugegeben, so dass das Produkt ausfällt. Die Suspension wird abfiltriert, und die erhaltenen Kristalle werden getrocknet.
¹H-NMR (DMSO): δ 8.5 (2H)s; 7,8 (4H)m; 7,7 (2H)t, 7,2 (2H)d; 7,1 (2H)d; 4,6 (2H)t; 4,1 (2H)t; 3,5 (2H)m; 3,3 (6H)s; 1,9 (2H)m.
4. Herstellung der Terpyridin-bis-hydrazino-Verbindung (q)
(a) Herstellung der Verbindung (p): 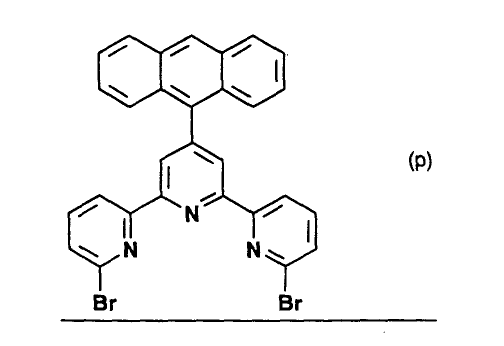
Es werden 2,6 g Anthracen-9-carbaldehyd (12,5 mmol), 5 g 2-acetyl-6-brompyridin (25 mmol), 11,2 g Acetamid (187,5 mmol) und 7,1 g Ammoniumacetat (93,7 mmol) in einem Kolben vorgelegt. Die entstandene braune Lösung wird 2 Stunden lang unter Rückfluss erhitzt (Badtemperatur 180 °C). Nachdem man auf 110 °C hat abkühlen lassen, werden 33 g in 71 ml Wasser gelöste Natriumhydroxidplätchen (0,83 mol) hinzugetropft. Das Reaktionsgemisch wird weitere 2 Stunden refluxiert und anschliessend auf 90 °C abgekühlt. Die überstehende Lösung wird abdekantiert, und der zurückbleibende Feststoff wird zweimal mit Wasser gewaschen. Die schwarze Masse wird in 25 ml Essigsäure gelöst. Es werden 1,75 ml 48 %ige Bromwasserstofflösung hinzugegeben und 5 Tage stehen gelassen. Nun werden die Suspension abfiltriert und der erhaltene grüne Niederschlag mit Diethylether gewaschen. Die Kristalle werden in Wasser suspendiert, welches mit 2 normaler Kaliumhydroxidlösung auf den pH-Wert von 8 eingestellt wird, und mit Dichlormethan ausgeschüttelt. Nach dem Trocknen der organischen Phase mit Natriumsulfat, Einengen am Rotationsverdampfer und Trocknem am Vakuum erhält man eine grüne Masse. Diese wird mit ungefähr 400 ml Ethanol umkristallisiert. Die erhaltenen Kristalle werden in einem Ethanol/Dichlormethan- Gemisch aufgenommen und zweimal mit einer gesättigten Ammoniumcarbonatlösung ausgeschüttelt. Die organischen Phasen werden über Natriumsulfat getrocknet, einrotiert und am Hochvakuum getrocknet. Das Rohprodukt wird über eine Säule gereinigt (Laufmittel Hexan : Essigsäureethylester 9:1). Man erhält grün-braune Kristalle.

| | | |
|---|---|---|
| MS | Peak berechnet | 568 m/z (M + H⁺) |
| | Peak gefunden | 569 m/z (M + H⁺) |

(b) Herstellung der Verbindung (q): 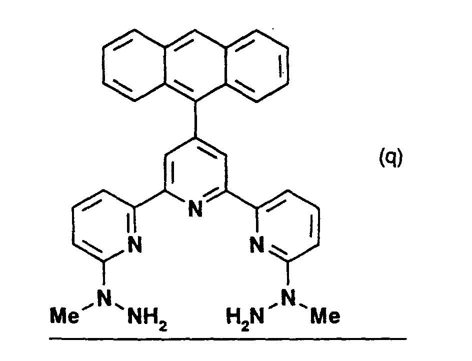
1.43 g (2.5 mol) der wie oben erhaltenen Verbindung (p) werden in 50 ml Methylhydrazin gegeben und über Nacht am Rückfluss gekocht. Es entsteht ein dunkelbraune Lösung. Das Lösungsmittel wird am Rotationsverdampfer eingeengt. Das Produkt wird in heissem Methanol suspendiert und anschliessend filtriert. Man erhält 400 mg grüne Kristalle. Diese werden aus CH₃CN umkristallisiert. Zuerst wird klarfiltriert, abgekühlt und abfiltriert. Danach trocknet man am Hochvakuum und erhält grüne Kristalle. Diese werden nochmals durch Umkristallisation aus CH₃CN gereinigt. Man erhält wiederum grüne Kristallle. Die Mutterlauge wird ebenfalls eingeengt. Es fallen 1 g braune Kristalle aus. Diese werden ebenfalls aus CH₃CN umkristallisiert und anschliessend am Hochvakuum getrocknet. Man erhält hellbraune Kristalle.

| Elementaranalyse: | | | |
|---|---|---|---|
| berechnet | 74,83% C | 5,47% H | 19.70% N |
| gefunden | 74,76% C | 5,55% H | 19.42% N |

5. Herstellung der 2.6-Dicarbonyl-Verbindung (r): 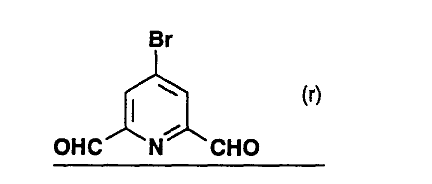
Es werden 118 µl (1.38 mmol) Oxalylchlorid unter Argon in 2 ml CH₂Cl₂ vorgelegt und auf -78°C abgekühlt. Danach werden vorsichtig 195 µl (2.75mmol) DMSO zugetropft. Man lässt das Reaktionsgemisch während 15 Minuten bei -78° C rühren. Anschliessend werden 100 mg (0.458 mmol) 2,6-Di(hydroxymethyl)-4-brompyridin in 100 µl DMSO gelöst und mit 1 ml CH₂Cl₂ versetzt. Diese Lösung wird dem Reaktionsgemisch zugegeben. Es wird 1 Stunde bei -78° C gerührt. Nun werden 381 µl (2.75 mmol) Et₃N zugegeben und 15 Minuten bei 0° C gerührt. Man engt ein und löst in H₂O und CH₂Cl₂. Die beiden Phasen werden extrahiert. Die wässrige Phase wird zweimal mit CH₂Cl₂ gewaschen. Man filtriert die organischen Phasen über Watte. Es wird eingeengt und am Hochvakuum getrocknet. Man erhält hellbraune Kristalle.
¹H-NMR (CDCl₃): δ 10.1 (2H)s; 8.7 (2H)s.
6. Herstellung der Terpyridin-bis-hydrazino-Verbindung (s): 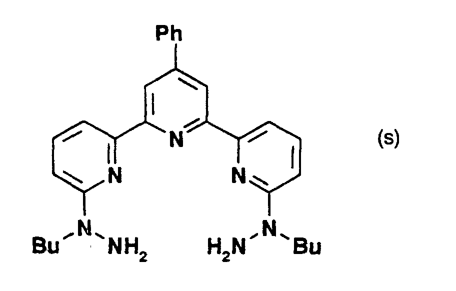
72 mg der wie unter Beispiel A1(b) erhaltenen Verbindung b.5 (0,16 mmol) werden in 480 mg Butylhzdrazin (5,44 mmol) aufgenommen und auf 110 °C unter Argon erwärmt. Die Suspension lässt man über Nacht kochen. Nachdem man das Reaktionsgemisch hat abkühlen lassen wird mit Diethylether und Methanol verdünnt und vom Feststoff mit Hilfe einer Filtration befreit. Das Filtrat wird am Rotationsverdampfer aufkonzentriert. Durch Zugabe von Methanol wird eine Ausfällung vorgenommen. Die Kristalle werden von der kaltgestellten Lösung abfiltriert und mit wenig Methanol nachgewaschen. Das Produkt wird am leichten Vakuum getrocknet. Man erhält beige Kristalle.

| | | |
|---|---|---|
| MS | Peak berechnet | 482 m/z (M + H⁺) |
| | Peak gefunden | 481 m/z (M + H⁺) |

### B Herstellung der Terpyridin-Lanthanid-Komplexe

### Beispiel B1:

1.) 1 mMol der entsprechenden Terpyridin-bis-hydrazino-Verbindung aus Beispiel A1 (c) wird unter Argon in 60 ml absolutem Methanol aufgenommen, mit dem Lanthanid(III)acetat (1 mMol) versetzt und 10 Minuten unter Rückfluss erhitzt. Zu dieser Lösung werden nacheinander 1,2 mMol der entsprechenden 2,6-Dicarbonylverbindung und 5 mMol konzentrierte wässrige Salzsäure gegeben. Es wird 2 Tage gekocht. Nach Abkühlen auf Raumtemperatur wird das Produkt abfiltriert und am Hochvakuum getrocknet.
Nach dieser Vorschrift werden die Verbindungen 1.1 bis 1.28 der Tabelle 1 hergestellt.
2.) 1 mMol der entsprechenden Terpyridin-bis-hydrazino-Verbindung aus Beispiel A1 (c) wird unter Argon in 60 ml absolutem Methanol aufgenommen, mit dem Lanthanid(III)chlorid (1 mMol) versetzt und 10 Minuten unter Rückfluss erhitzt. Zu dieser Lösung werden nacheinander 1,2 mMol der in Beispiel A2(a) erhaltenen Verbindung gegeben. Es wird über Nacht gekocht. Nach Abkühlen auf Raumtemperatur wird das Lösungsmittel abgezogen und das Produkt durch Umkristallisation aus Dimethylsulfoxid und Toluol erhalten.
Nach dieser Vorschrift werden die Verbindungen 1.29 bis 1.32 der Tabelle 1 hergestellt. Analog erhält man die Verbindungen 1.33 bis 1.44 der Tabelle 1.
3.) Herstellung von Terpyridin-Lanthanid-Komplexen mit weiteren Substituenten R₅
(a) Herstellung der Verbindung 1.45: 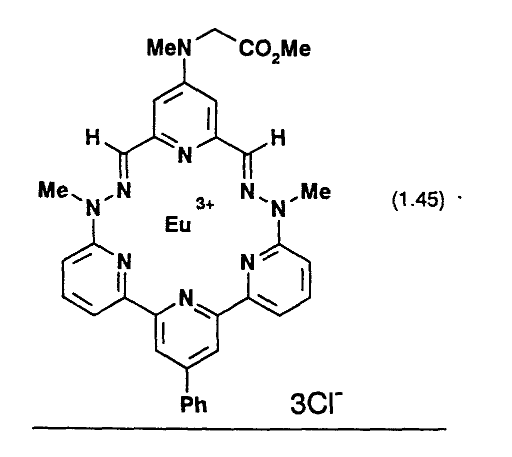
168 mg der wie im Beispiel A1 (c) erhaltenen Substanz c.5 (0,423 mmol) werden in 20 ml trockenem Methanol vorgelegt und zum Sieden erhitzt. Nun gibt man 155 mg Europiumtrichloridhexahydrat (0,423 mmol) hinzu und lässt die gelbe Suspension 15 Minuten lang rühren.Nach Zugabe von 100 mg der wie im Beispiel A1 (c) erhaltenen Substanz (f) (0,423 mmol) in 15 ml trockenem Methanol lässt man die Suspension über Nacht unter Argon am Rückfluss kochen. Nachdem das Reaktionsgemisch auf Raumtemperatur abgekühlt ist, filtriert man dieses und zieht 2/3 des Lösungsmittels der erhaltenen gelben Lösung ab. Mit Hilfe von Diethylether werden orange-frabige Kristalle aus der Lösung gefällt und über einen Milliporefilter abfiltriert. Der Feststoff wird unter Hochvakuum getrocknet. Man erhält orange-farbige Kristalle.

| | | |
|---|---|---|
| MS | Peak berechnet | 821 m/z (M - Cl⁻) |
| | Peak gefunden | 821 m/z (M - Cl⁻) |

Analog erhält man die in Tabelle 1 angegebenen Verbindungen 1.46 bis 1.58.
(b) Herstellung der Verbindung 1.59: 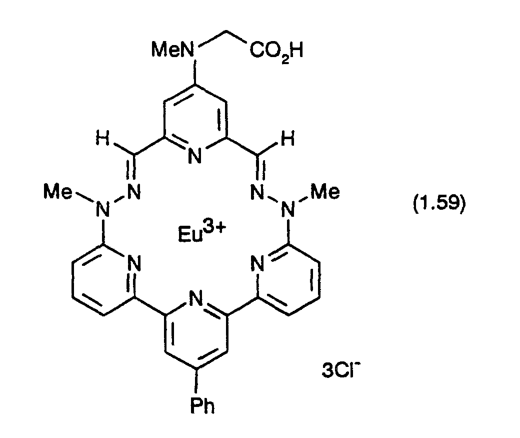
10 mg (0.0116 mmol) wie oben erhaltenen Verbindung 1.45 werden in 5 ml Wasser gegeben und im Rückfluss erwärmt. Es entsteht eine gelbe Lösung. Nach drei Tagen wird das Reaktionsgemisch über Acradisk filtriert. Man engt ein und trocknet am Hochvakuum. Man erhält eine gelben Feststoff.
4.) Herstellung von Terpyridin-Lanthanid-Komplexen mit weiteren Substituenten R₅
(a) Herstellung der Verbindung 1.60: 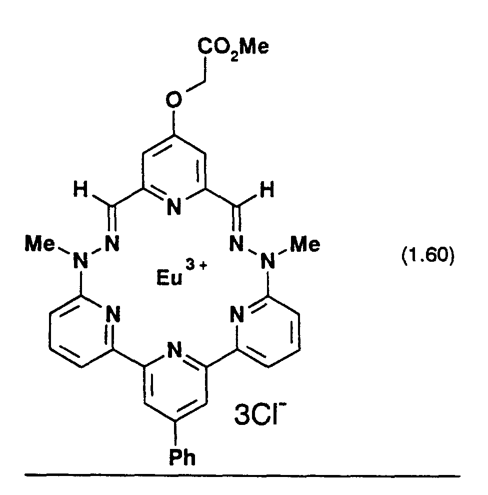
48 mg der wie im Beispiel A1 (c) erhaltenen Substanz c.5 (0,12 mmol) werden in 7,5 ml Methanol unter Argon und Rückfluss erhitzt. Nun gibt man 44 mg Europiumtrichloridhexahydrat (0.12 mmol) hinzu und lässt 15 Minuten unter Rückfluss kochen. Nachdem man die wie im Beispiel A3.2(e) erhaltene Verbindung (k) in 2,5 ml Methanol hinzugefügt hat, hält man die Lösung 45 Min. am Sieden. Die abgekühlte Suspension wird über einen Filter abfiltriert und die klare gelbe Lösung am Rotationsverdampfer auf ca. 2 ml aufkonzentriert. Hieraus wird mit Hilfe von Diethylether ein gelber Niederschlag ausgefällt, der dann einmal mit einer Lösung von Diethylether/Methanol 1:1 und zweimal mit Diethylether gewaschen wird. Der Rückstand wird unter Hochvakuum getrocknet. Man erhält gelbe Kristalle.

| | | |
|---|---|---|
| MS | Peak berechnet | 807 m/z (M - Cl⁻) |
| | Peak gefunden | 809 m/z (M - Cl⁻) |

(b) Herstellung der Verbindung 1.61: 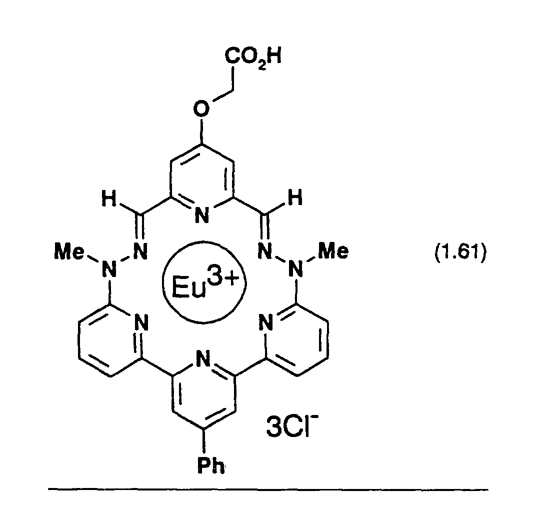
Die Verbindung 1.61 wird aus der Verbindung 1.60 hergestellt, in Analogie zur Herstellung von Verbindung (1.59) aus Verbindung (1.45).

| | | | |
|---|---|---|---|
| MS | Peak berechnet | 1056 m/z | [M - 3Cl⁻+2(C₈H₇O₄)⁻] |
| | Peak gefunden | 1055 m/z | [M - 3Cl⁻+2(C₈H₇O₄)⁻] |

5.) Herstellung von Terpyridin-Lanthanid-Komplexen mit weiteren Substituenten R₁
(a) Herstellung der Verbindung 1.62: 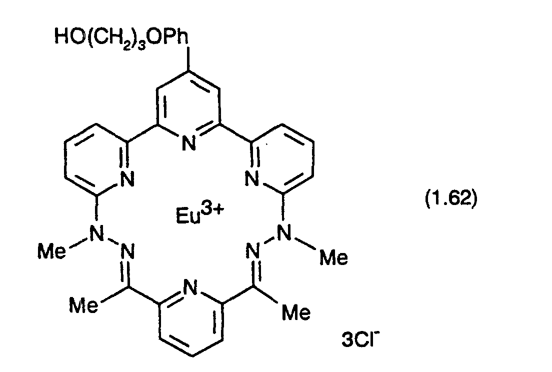
136 mg der im Beispiel A3.3(c) erhaltenen Verbindung (o) (5,94 mmol) werden in 400 ml trockenem Methanol gelöst und unter Argon zum Rückfluss erhitzt. Dann gibt man 1,53 g Europiumtrichloridhexahydrat (5,94 mmol) und lässt das Reaktionsgemisch 0,5 Stunden am Rückfluss kochen. Danach werden 1 2,6-Diacetylpyridin (5,94 mmol), welches in 100 ml trockenem Methanol gelöst ist, und 2 Tropfen konzentrierte Salzsäure zu der entstandenen klaren gelben Lösung hinzugefügt. Man lässt dieses Reaktionsgemisch 8 Tage unter Rückfluss kochen. Nach Abkühlen auf Raumtemperatur wird die Suspension über einen Hydrofilter abfiltriert. Die erhaltene klare gelbe Lösung wird am Rotationsverdampfer aufkonzentriert. Mit Hilfe von Diethylether wird eine Fällung herbeigeführt. Der Niederschlag wird einmal mit einem Gemisch von Diethylether /Methanol 1 : 1 und zweimal mit Diethylether gewaschen. Der Rückstand wird unter Hochvakuum getrocknet. Man erhält einen orange-farbigen Feststoff.

| | | |
|---|---|---|
| MS | Peak berechnet | 822 m/z (M - Cl⁻) |
| | Peak gefunden | 822 m/z (M - Cl⁻) |

6.) Herstellung von Terpyridin-Lanthanid-Komplexen mit weiteren Substituenten R₅
(a) Herstellung der Verbindung 1.63: 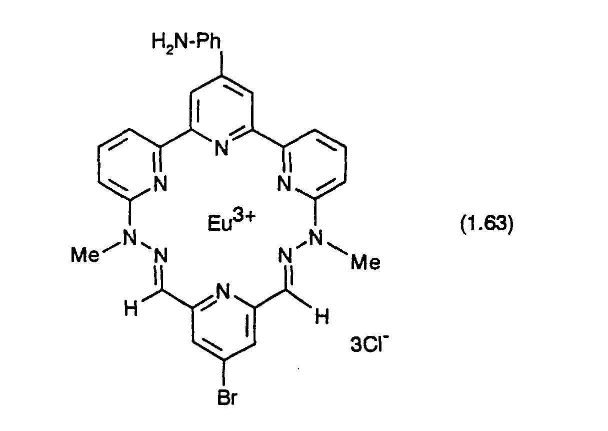
96 mg der wie im Beispiel A1 (c) erhaltenen Verbindung c.2 (0,234 mmol) werden in 20 ml trockenem Methanol gelöst und unter Argon zum Rückfluss erhitzt. Dann gibt man 60 mg Europiumtrichloridhexahydrat (0,234 mmol) und lässt das Reaktionsgemisch 0,5 Stunden am Rückfluss kochen. Danach werden 50mg der wie im beispiel A3.5 erhaltenen Verbindung (r) (0,234 mmol), welches in 15 ml trockenem Methanol gelöst ist, und 2 Tropfen konzentrierte Salzsäure zu der entstandenen klaren gelben Lösung hinzugefügt. Man lässt dieses Reaktionsgemisch über Nacht unter Rückfluss kochen. Nach Abkühlen auf Raumtemperatur wird die dunkelrote Suspension über einen Hydrofilter abfiltriert. Die erhaltene klare rote Lösung wird am Rotationsverdampfer aufkonzentriert und mit Hilfe von Diethylether eine Fällung herbeigeführt. Der Niederschlag wird einmal mit einem Gemisch von Diethylether /Methanol 1 : 1 und zweimal mit Diethylether gewaschen. Der Rückstand wird unter Hochvakuum getrocknet. Man erhält einen dunkelroten Feststoff.

| | | |
|---|---|---|
| MS | Peak berechnet | 813 m/z (M - Cl⁻) |
| | Peak gefunden | 812 m/z (M - Cl⁻) |

7. Herstellung von Terpyridin-Lanthanid-Komplexen mit weiteren Substituenten R₁ bzw. R₅
(a) Herstellung der Verbindungen 1.64 bis 1.74:
   In Analogie zu den oben beschriebenen Reaktionen, beispielsweise zur Herstellung der Verbindungen 1.62 und 1.63, werden die in der Tabelle 1 aufgeführten Verbindungen 1.64 bis 1.74 aus den entsprechen substituierten 2,6-Dicarbonylpyridin-Verbindungen und den entsprechend substituierten Terpyridin-bis-hydrazino-Verbindungen hergestellt.

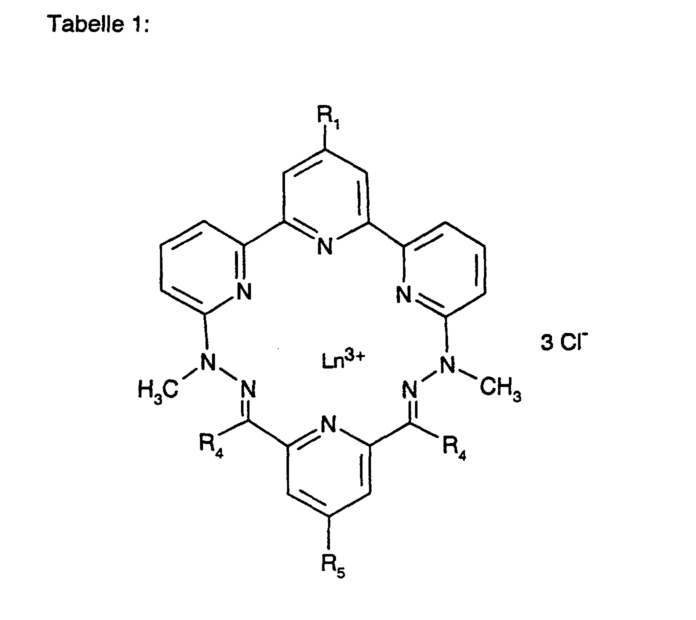

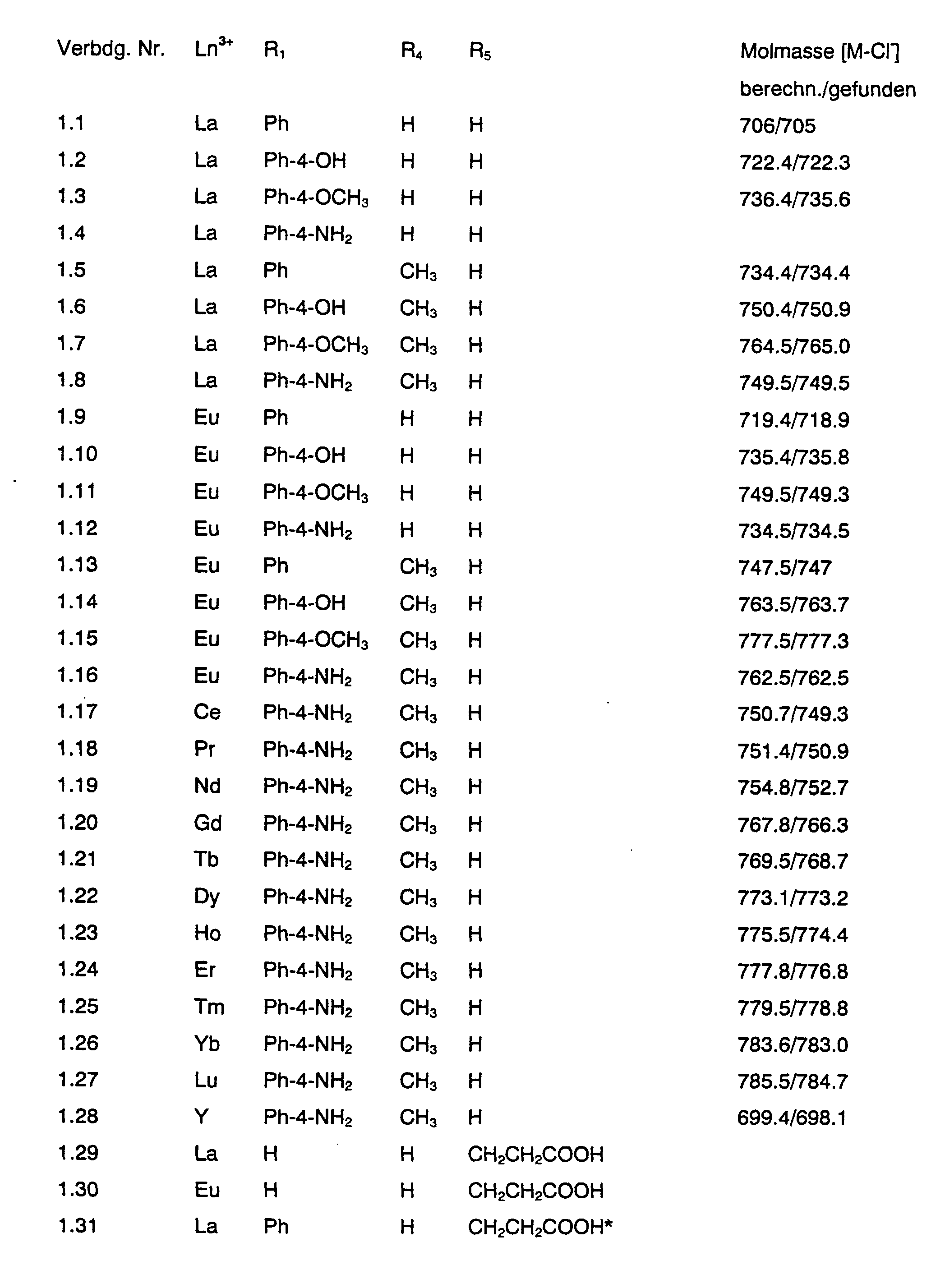

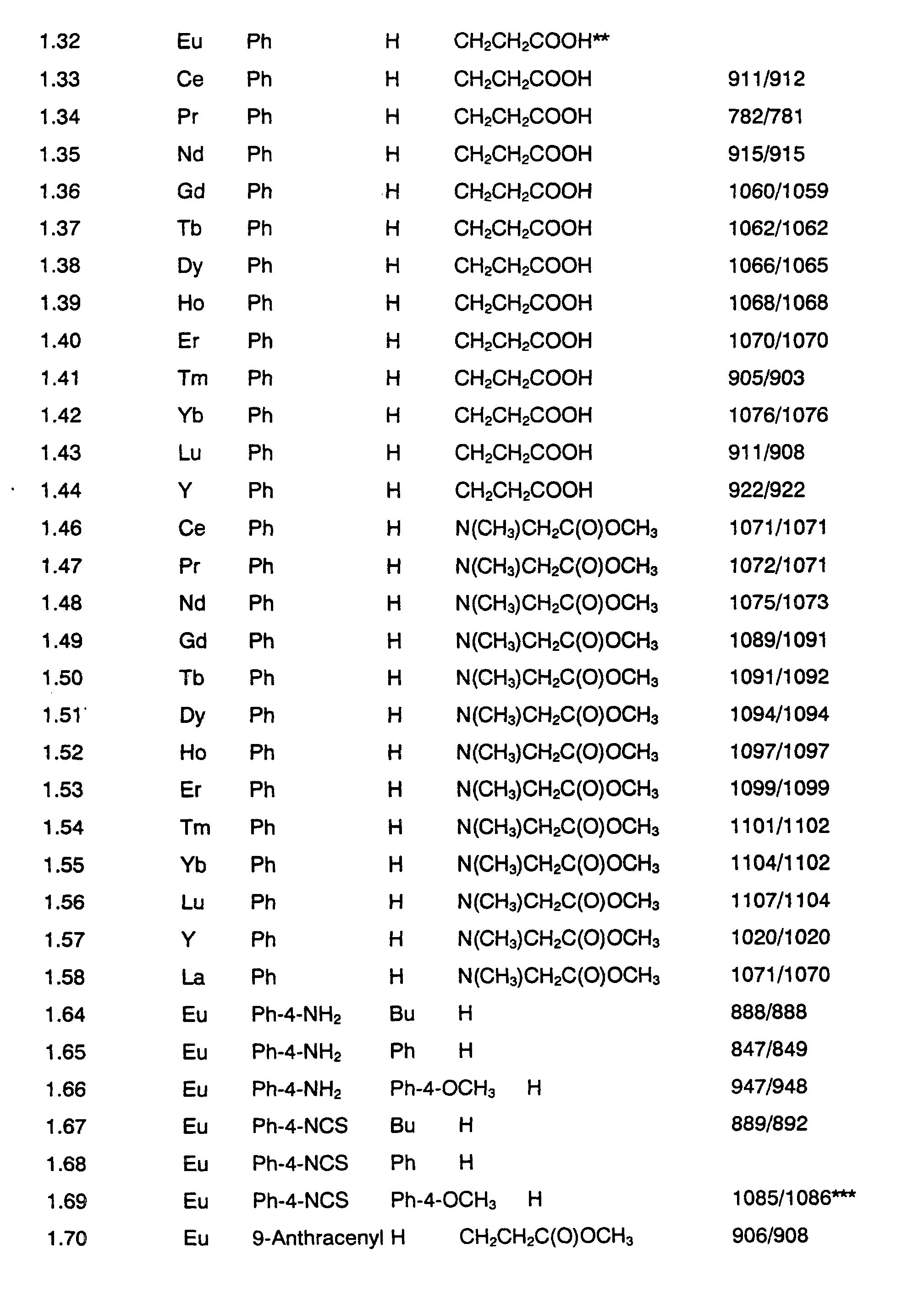

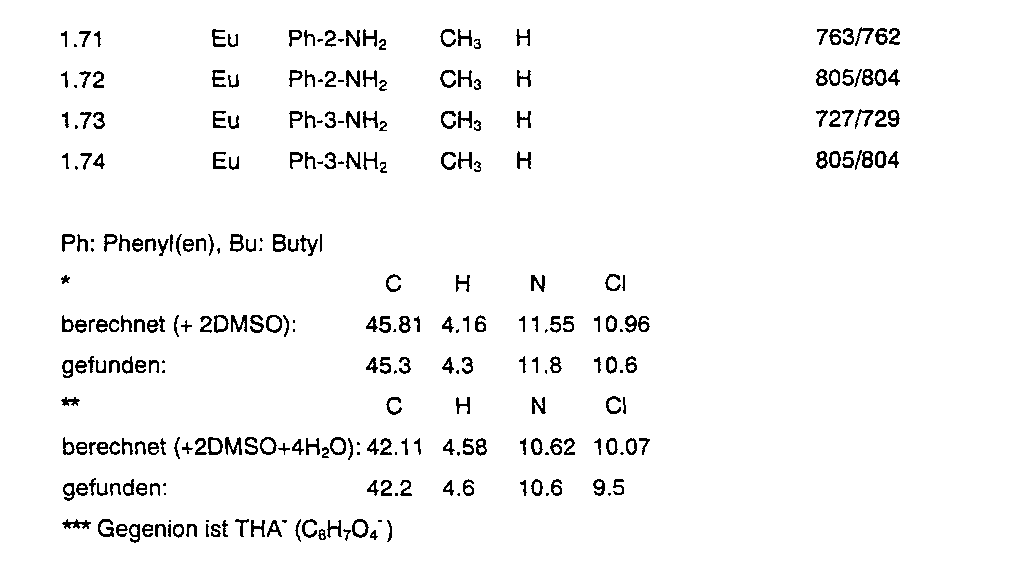

### Beispiel B2: Herstellung von Isothiocyanat-Derivaten

Zu einer Suspension von 4,4 mMol Natriumbicarbonat und 3,5 mMol Thiophosgen in 4 ml Chloroform wird eine Lösung des entsprechenden Komplexes der Tabelle 1 gegeben. Das Gemisch wird bei Raumtemperatur 2,5 Stunden heftig gerührt. Die Chloroform-Phase wird abgetrennt und einmal mit Wasser gewaschen. Alle wässrigen Phasen werden vereinigt und getrocknet. Die so erhaltenen Produkte 2.1 bis 2.15 der Tabelle 2 werden ohne weitere Reinigung weiterverwendet. Analog werden aus weiteren Verbindungen der Tab. 1, die primäre Aminogruppen als Substituenten enthalten, die entsprechenden Isothiocyanatverbindungen hergestellt.

**Tabelle 2:**

| Verbdg. Nr. | Ln³⁺ | R₁ | R₄ | R₅ | Molmasse [M-Cl] berechnet/gefunden |
|---|---|---|---|---|---|
| 2.1 | Ce | Ph-NCS | CH₃ | H | 792.7/792.7 |
| 2.2 | Pr | Ph-NCS | CH₃ | H | 793.5/791.2 |
| 2.3 | Gd | Ph-NCS | CH₃ | H | 809.9/807.4 |
| 2.4 | Tb | Ph-NCS | CH₃ | H | 811.5/811.7 |
| 2.5 | Dy | Ph-NCS | CH₃ | H | 815.1/815.9 |
| 2.6 | Ho | Ph-NCS | CH₃ | H | 817.5/816.2 |
| 2.7 | Er | Ph-NCS | CH₃ | H | 819.9/819.0 |
| 2.8 | Tm | Ph-NCS | CH₃ | H | 821.5/820.1 |
| 2.9 | Yb | Ph-NCS | CH₃ | H | 825.6/826.4 |
| 2.10 | Lu | Ph-NCS | CH₃ | H | 827.6/825.5 |
| 2.11 | Y | Ph-NCS | CH₃ | H | 741.5/740.2 |
| 2.12 | La | Ph-NCS | CH₃ | H | 791.5/792.1 |
| 2.13 | Eu | Ph-NCS | CH₃ | H | 804.6/804.7 |
| 2.14 | La | Ph-NCS | H | H | |
| 2.15 | Eu | Ph-NCS | H | H | |

### C Herstellung der Aminooligonukleotide

Etwa 30 mg der 'controled pore glass' (CPG) Festphase werden in einem Standard Applied Biosystem Reaktionsgefäß für eine 1.5 µMol Synthese eingewogen. Die CPG-Festphase (1) trägt den geschützten 3'-Baustein (im Beispiel, dC) des zu synthetisierenden Amino-Oligonukleotids.

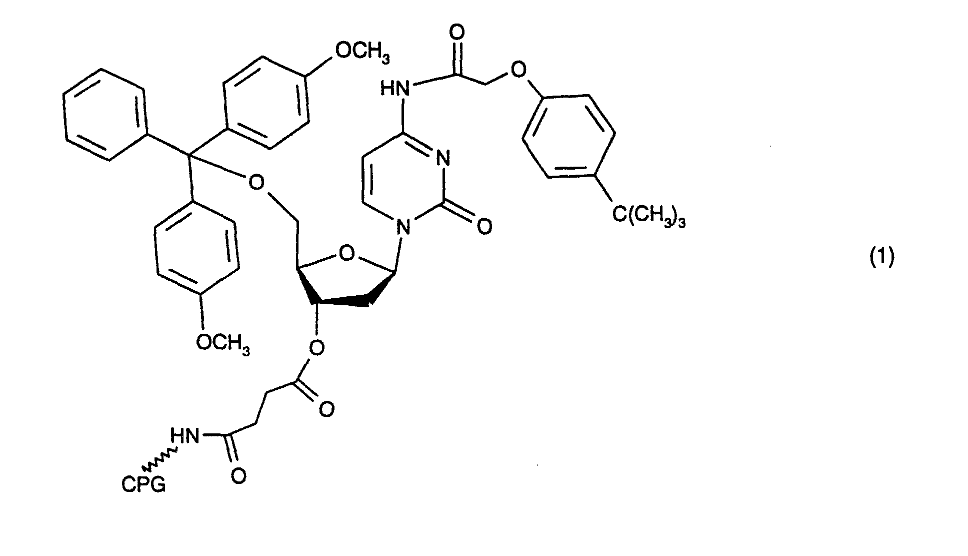

Für die Oligomerisierung werden die Phosphoramidite (6), (7), (8) und (9) eingesetzt.

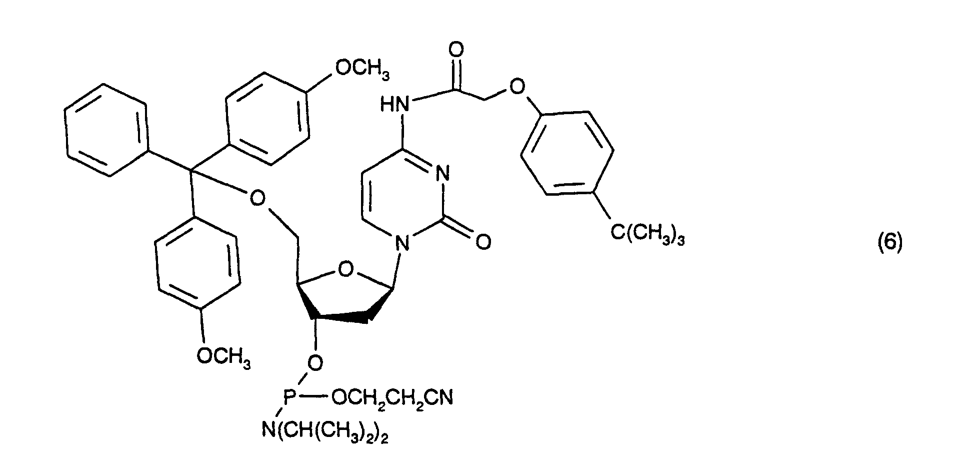

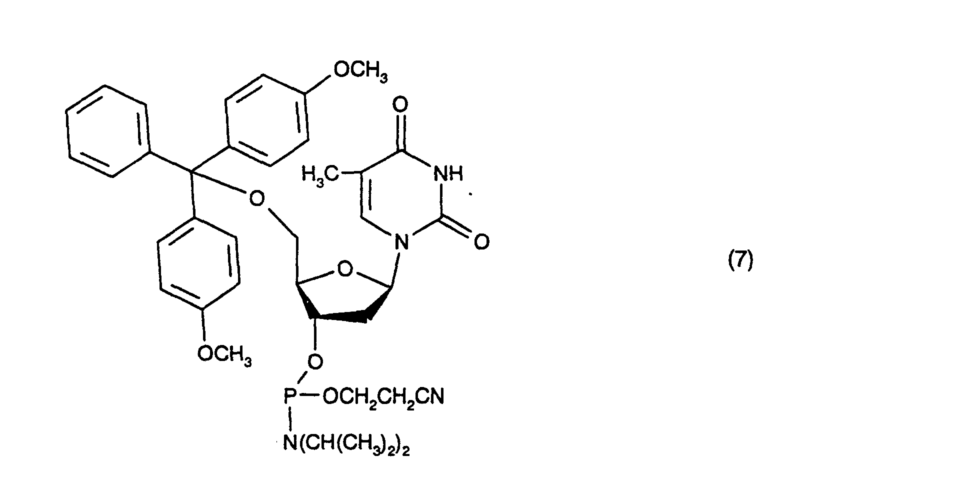

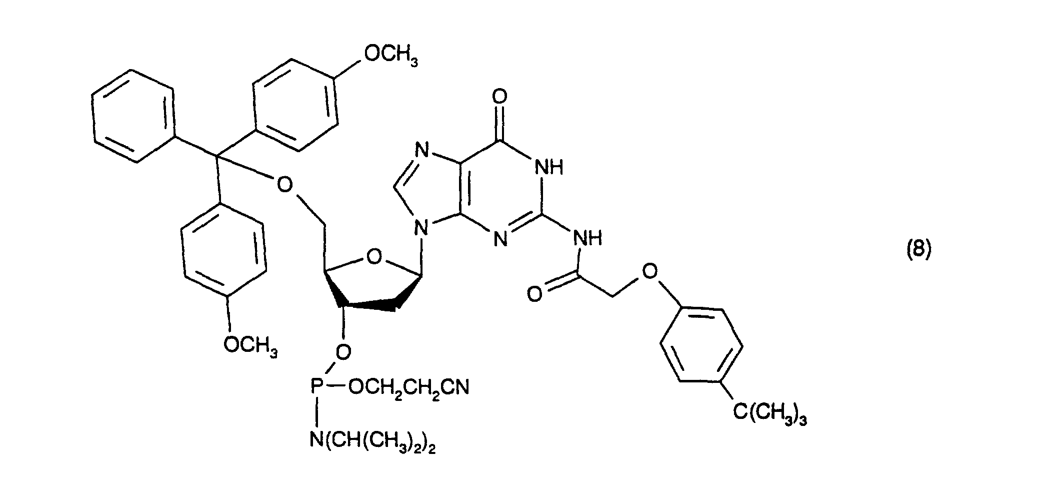

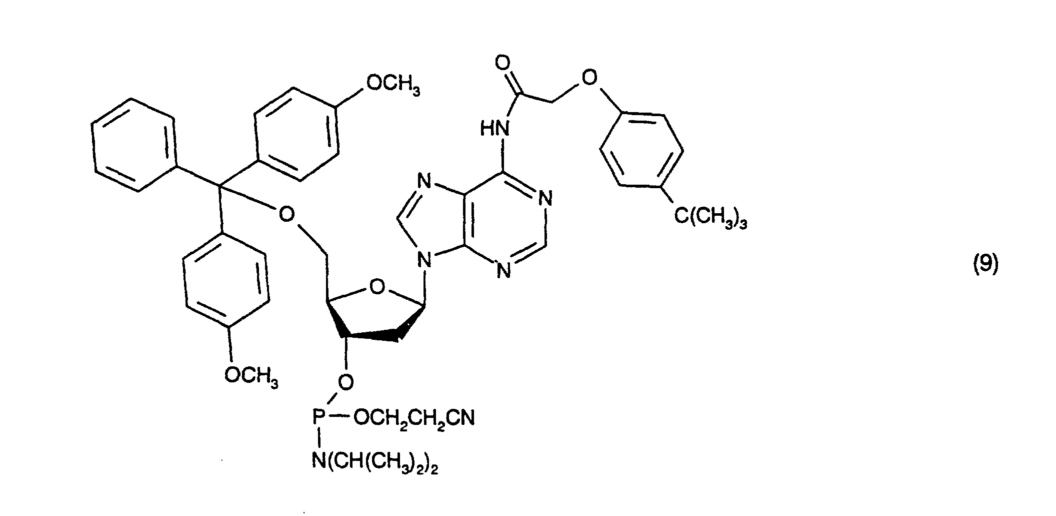

Für die spätere Aufknüpfung der Metallkomplexe via der Aminofunktion werden gesonderte Phosphoramidite (10), (11), (12), (13), (14), (15) und (16) eingesetzt. 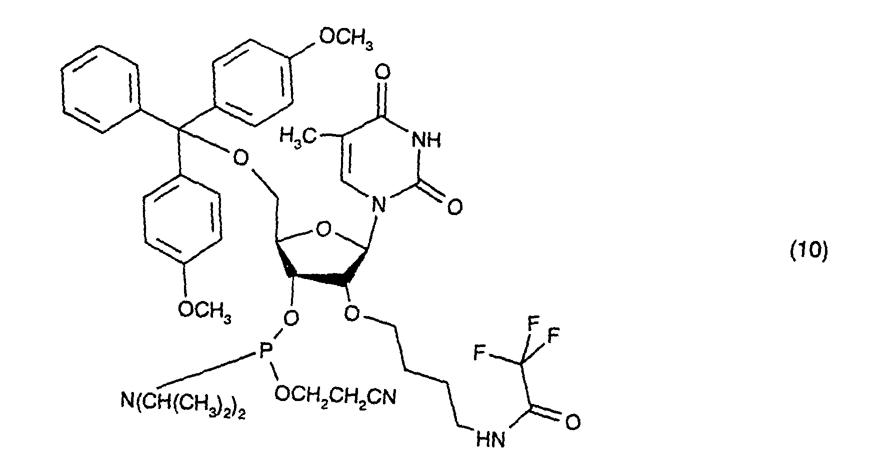 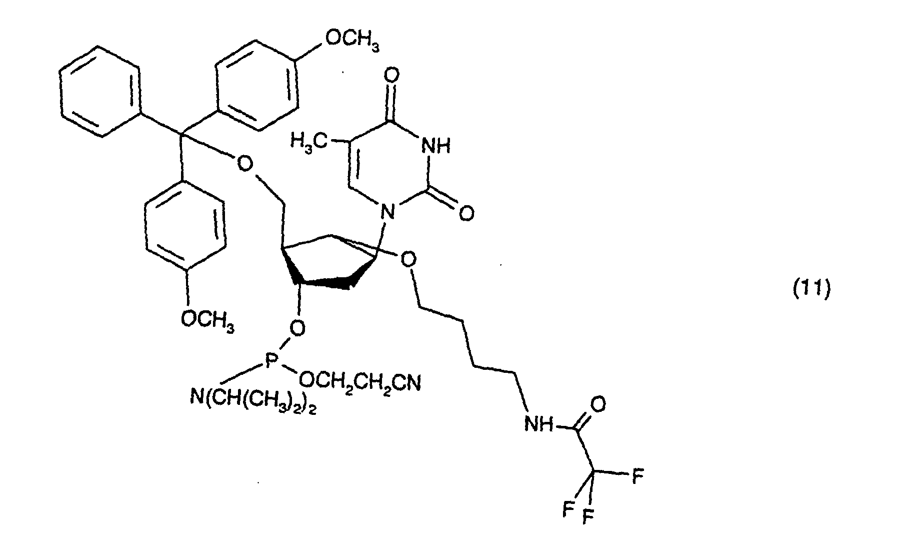 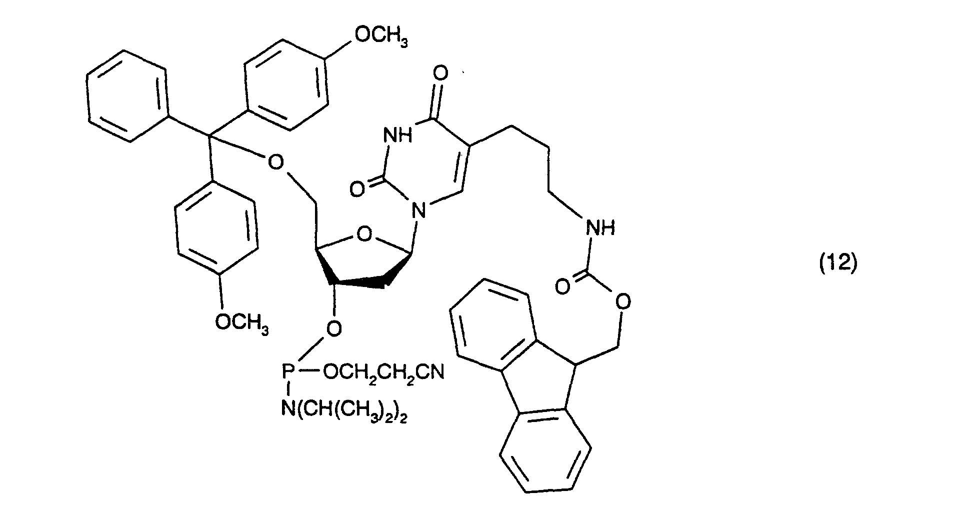 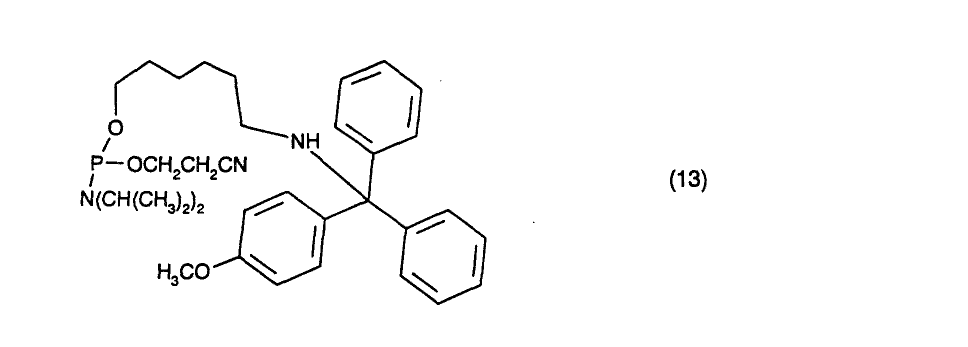 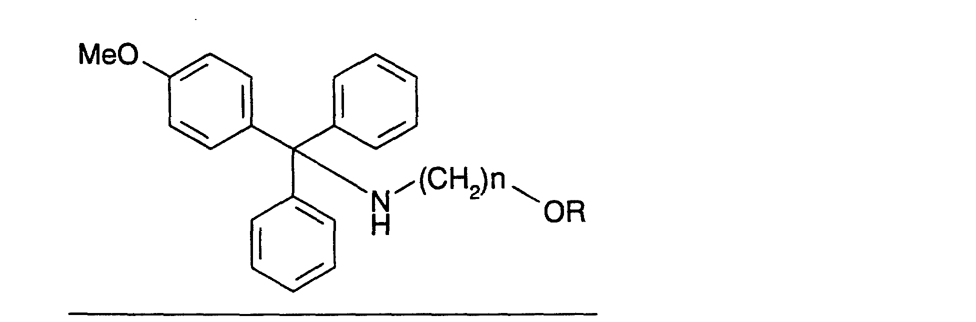
(14): n = 3, R = P(N(i-Propyl)₂)OCH₂CH₂CN
(15): n = 4, R = P(N(i-Propyl)₂)OCH2CH2CN
(16): n = 5, R = P(N(i -Propyl)₂)OCH₂CH₂CN

### Herstellungsbeispiele für Phosphoramidite (14), (15) und (16):

- Herstellung von Ausgangsverbindung (14a) (n = 3, R = H):
   4,0g 3-Amino-1-propanol, 3,28g 4-Methoxytriphenylchlormethan und 35ml Pyridin werden in einem wasserfreien Kolben vorgelegt. Bei Raumtemperatur und unter Argon wird während 4,5 Stunden gerührt. Das Lösungsmittel wird eingedampft. Der Rückstand wird einmal mit Toluol und zweimal mit Acetonitril versetzt und jeweils einrotiert. Der Rückstand wird in Methylenchlorid geloest und zweimal mit ges. Natriumhydrogencarbonatlösung gewaschen. Die wässrigen Phasen werden dreimal mit Methylenchlorid extrahiert. Die organischen Phasen werden vereinigt, über Natriumsulfat getrocknet und konzentriert. Anschliessend wird das Rohprodukt mittels Flaschchromatographie (Kieselgel) gereinigt.
   (Laufmittel: Essigester/Hexan = 1:2 ). Man erhält ein gelbes Oel.
   ¹H-NMR: δ in CDCl₃, OCH₃ = 3,65.
   Die Ausgangsverbindungen (15a) (n= 4, R = H) und (16a) (n = 5, R = H) werden analog aus 4-amino-1-butanol bzw. 5-amino-1-pentanol hergestellt.
   ¹H-NMR: δ in CDCl₃, OCH₃ = 3,65 (15a) bzw. 3,65 (16a).
- Herstellung des Phosphoramidites (14):
   Unter Argon werden 1,99g N,N-Diisopropylammoniumtetrazolid und 3,5g 2-Cyanoethyl-N,N,N',N'-tetraisopropyl-phosphordiamidit in 150ml Methylenchlorid vorgelegt. Innert 20 min. wird eine Lösung der wie oben erhaltenen Verbindung (14a) in 120ml Methylenchlorid dazugetropft. Die feine gelbe Suspension wird während 4,5 Stunden gerührt. Danach wird mit 250ml Methylenchlorid verdünnt und zweimal mit ges. Natriumhydrogencarbonatlösung gewaschen. Die wässrigen Phasen wurden dreimal mit Methylenchlorid extrahiert. Die organischen Phasen werden vereinigt, über Natriumsulfat getrocknet und konzentriert. Anschliessend wird das Rohprodukt mittels Flaschchromatographie (Kieselgel) gereinigt (Laufmittel: Essigester/Hexan=1:4 + 0,5% N-Methylmorpholin). Man erhält ein gelbes Oel.
   ³¹P-NMR: δ in CDCl₃, 146,9).

Die Phosphoramidite (15) und (16) werden anaolg aus den wie oben erhaltenen Verbindungen (15a) bzw. (16a) hergestellt.
³¹P-NMR: δ in CDCl₃, 146,9 (15) bzw. 147,0 (16).

Die Synthesezyklen werden mit dem Syntheseautomat 394 von Applied Biosystem mit einer Veränderung (Kopplungszeit der Phosphoramidite der Desoxy-Reihe (6), (7), (8) und (9) beträgt 2 Minuten, die der Amidite (10) und (11) beträgt 10 Minuten, (12) beträgt 5 Minuten und (13) beträgt 40 Minuten; (13) wird 100fach im Überschuss eingesetzt) nach dem Standardprotokoll der Firma Applied Biosystem durchgeführt (User Manual Version 2.0 (1992) 1.0 µMol Cyclus, Appen. I-41). Zur Konjugation der erhaltenen Oligonukleotide mit den Metallkomplexen werden die jeweiligen Schutzgruppen unter Standard-Bedingungen abgespalten.

Als weitere käufliche Reagenzien werden eingesetzt:
0,1 M Phosphoramidit
Tetrazol/Acetonitril: 4 %, 96 %
Tert.-Butylphenoxyessigsäureanhydrid/Pyridin/Tetrahydrofuran: 10 %, 10 %, 80 %
N-Methylimidazol/Tetrahydrofuran: 16%, 84%
Trichloressigsäure/Dlmethylchlormethan: 2%, 98%
Jod/Wasser/Pyridin/Tetrahydrofuran: 3%, 2%, 20%, 75%

Synthetisiert werden die folgenden Amino-Oligonukleotide: (821) 5'- GAC TGG CGA GAT* CGG CAG TCG GCT AG-3', worin T* 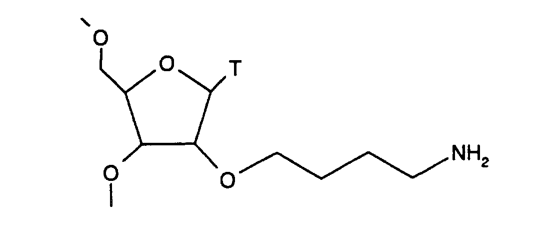 bedeutet, wobei T für Thymin steht, (823) 5'- GAC TGG CGA GAT* CGG CAG TCG GCT AG-3', worin T* 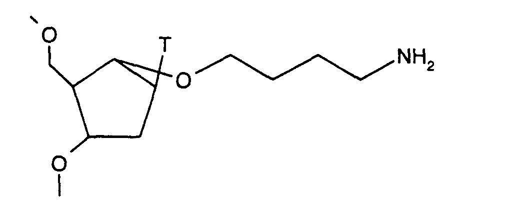 bedeutet, wobei T für Thymin steht, (940) 5'-GAC TGG CGA GAT CGG CAG T*CG GCT AG-3', worin T* 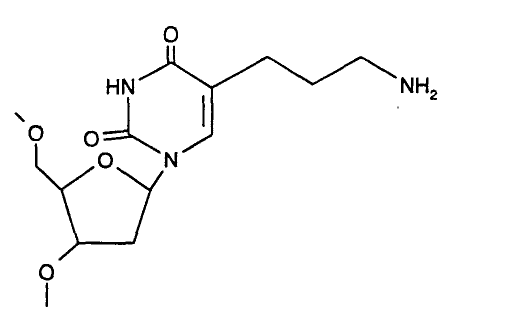 bedeutet, 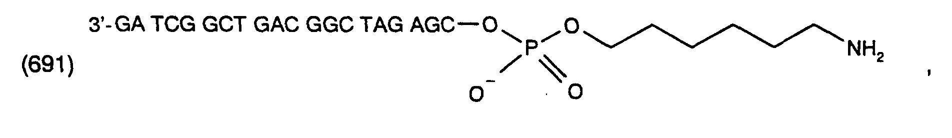
3'-GA TCG GCT GAC GGC TAG AGC—O
(691) NH2 ,
O⁻ O
 (1759) 5'-H₂N(CH₂)₃OP(O)₂-CGA GAT CGG CAG TCG GCT AG-3', (1760) 5'-H2N(CH2)4OP(O)2-CGA GAT CGG CAG TCG GCT AG-3', (1761) 5'-H2N(CH2)5OP(O)2-CGA GAT CGG CAG TCG GCT AG-3', und

(1757) 5'-H2N(CH2)6OP(O)2-GGA GAT CGG CAG TCG GCT AG-3'.

### D Herstellung der Terpyridin-Lanthanid-Oligonukleotid-Konjugate

### Beispiel D1: Herstellung von Konjugaten, bei denen das Oligonukleotid an den Terpyridin-Teil des Lanthanid-Komplexes gebunden ist

(a) 0,2 mg des entsprechenden Aminooligonukleotids werden in 150 µl
   Pyridin/Wasser/Triethylamin (90:15:1) gelöst. Nach Zugabe von 1 mg des entsprechenden Isothiocyanato-Komplexes der Tabelle 2 wird das Gemisch 1 Stunde bei Raumtemperatur stehengelassen. Das Reaktionsgemisch wird einmal gegen eine 0,1 molare Kaliumchloridlösung und dreimal gegen Wasser dialysiert. Das Produkt wird durch Umkehrphasen-HPLC (Gradient: von 0 % bis 30 % Acetonitril in 0,05 M Triethylammoniumacetat in 90 Minuten) auf einer Nucleosil ®-C₁₈-Säule oder durch lonentauscher-HPLC (Gradient: 10 Minuten 20 % einer 1 M Kaliumchloridlösung und 80 % einer 20 mM Kaliumphosphatlösung pH 6.0, welche 20 % Acetonitril enthält; dann innerhalb von 60 Minuten auf 80 % Kaliumchloridlösung) bei 60°C auf einer PVDI.4000A-Säule, 5 µm ergibt die reinen Konjugate 3.1 bis 3.13, 3.18 und 3.21 der Tabelle 3.
(b) 3 mg des entsprechenden Aminooligonukleotids werden in 200 µl DMSO/100 µl N-Methyl-morpholin suspendiert. Nach Zugabe von 1 mg des entsprechenden Isothiocyanat-Komplexes (siehe Tabelle 2 sowie analog aus entsprechenden Verbindungen der Tab. 1 hergestellte Isothiocyanat-Komplexe) wird das Gemisch 2-3 Stunden bei Raumtemperatur stehengelassen. Das Produkt wird durch Behandlung mit 32% wässrigem Ammoniak von der Festphase gespalten und völlig entschützt (3 Std bei Raumtemperatur). Die Reinigung durch Umkehrphasen-HPLC ergibt die Verbindungen 3.26 bis 3.44 der Tabelle 3.

### Beispiel D2: Herstellung von Konjugaten, bei denen das Oligonukleotid an den Pyridin-Teil des Lanthanid-Komplexes gebunden ist

(a) Eine Lösung von 3 µMol des entsprechenden Carbonsäure-Derivates 1.29 bis 1.32 (Tab. 1) in 200 µl Dimethylsulfoxid wird mit 3.3 µMol Dicyclohexylcarbodiimid und 3.3 µMol N-Hydroxysuccinimid versetzt und 16 Stunden bei Raumtemperatur stehengelassen. Nach Zugabe von 100 µMol N,N-Diisopropylethylamin werden 0,2 mg des entsprechenden Aminooligonukleotids zugegeben. Nach vier Tagen bei Raumtemperatur wird zweimal gegen 50 mM Triethylammoniumhydrogencarbonat und zweimal gegen Wasser dialysiert. Die Reinigung durch Umkehrphasen-HPLC (siehe D1 (a)) ergibt die Verbindungen 3.14 bis 3.17, 3.19, 3.20 und 3.22 bis 3.25 der Tabelle 3.
(b) Eine Lösung von 3 µmol des entsprechenden Carbonsäure-Derivates (siehe auch Tabelle 1) in 200 µl Dimethylsulfoxid wird mit 3.3 µmol Dicyclohexylcarbodiimid und 3.3 µmol N-Hydroxysuccinimid versetzt und 16 Stunden bei Raumtemperatur stehengelassen. Nach Zugabe von 3 mg Aminooligonukleotid werden 100 µl N-Methylmorpholin zugefügt. Nach drei Tagen bei Raumtemperatur wird zweimal mit DMSO und einmal mit Wasser gewaschen. Das Produkt wird durch Behandlung mit 32% wässrigem Ammoniak von der Festphase gespalten und völlig entschützt (3 Std bei Raumtemperatur). Die Reinigung durch Umkehrphasen-HPLC ergibt die Verbindungen 3.45 bis 3.49 der Tabelle 3.

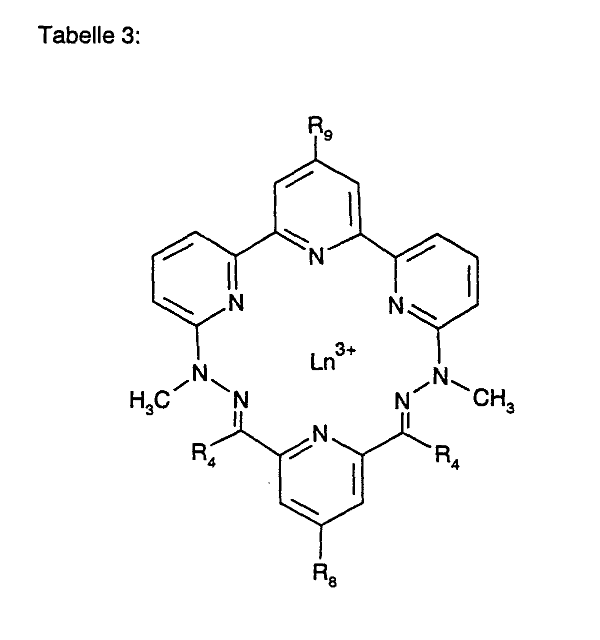

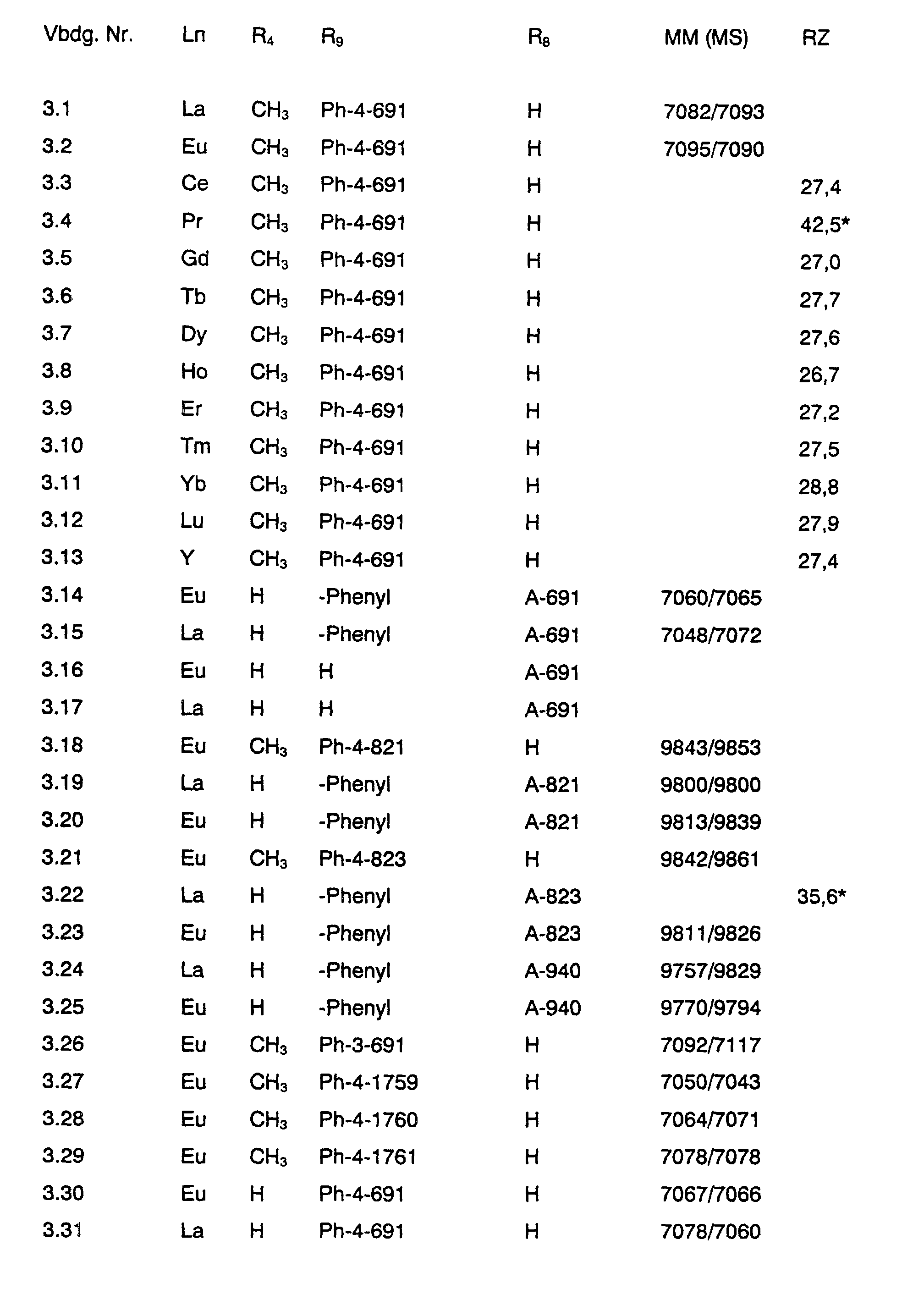

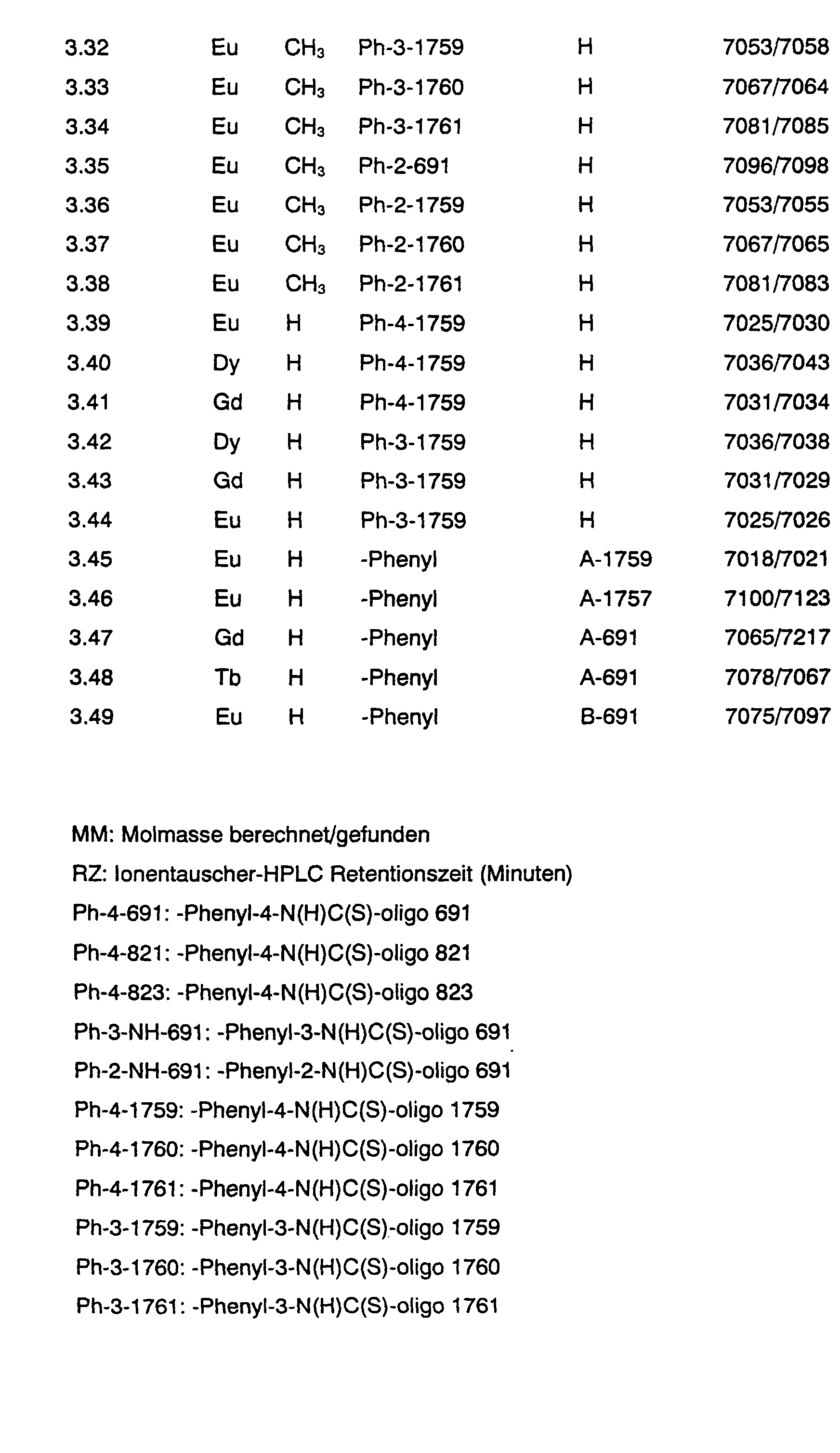

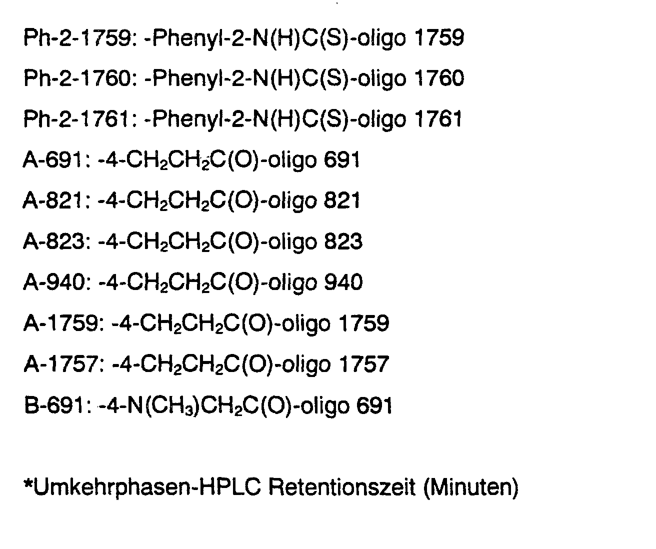

### E Herstellung von Substrat RNA (Ziel-RNA)

### Beispiel E1: Substrat RNA Synthese

Etwa 30 mg der 'controled pore glass' (CPG) Festphase werden in einem Standard Applied Biosystem Reaktionsgefäß für eine 1.5 µMol Synthese eingewogen. Die CPG-Festphase (1) trägt den geschützten 3'-Baustein (im Beispiel, rC) der zu synthetisierenden RNA.

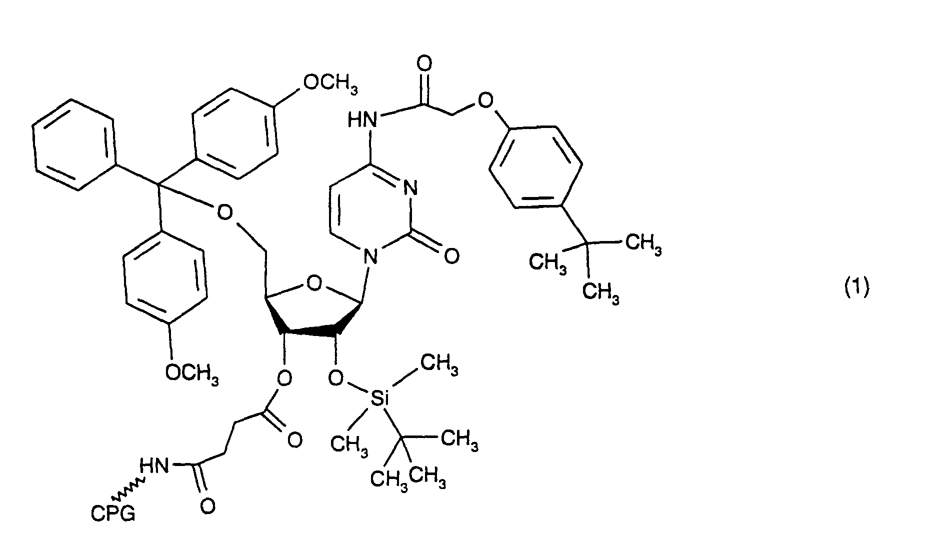

Für die Oligomerisierung werden die Phosphoramidite (2), (3), (4) und (5) eingesetzt.

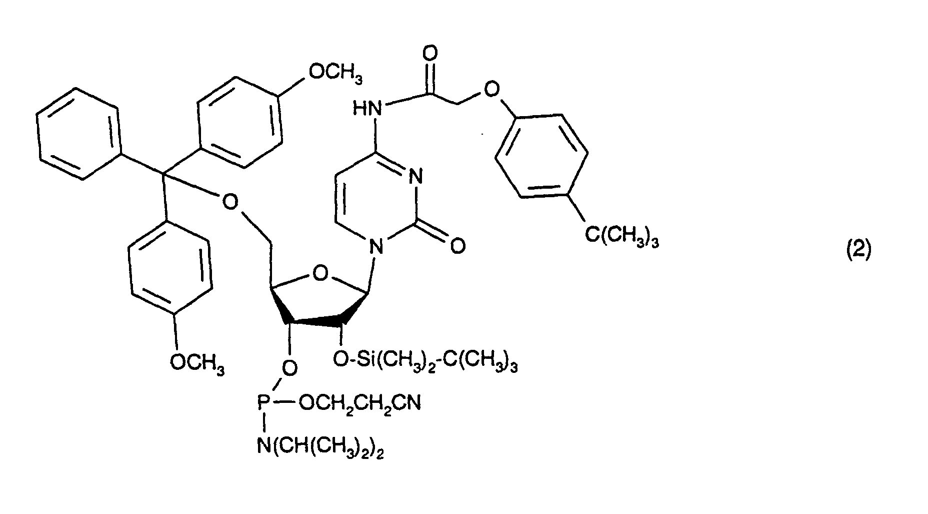

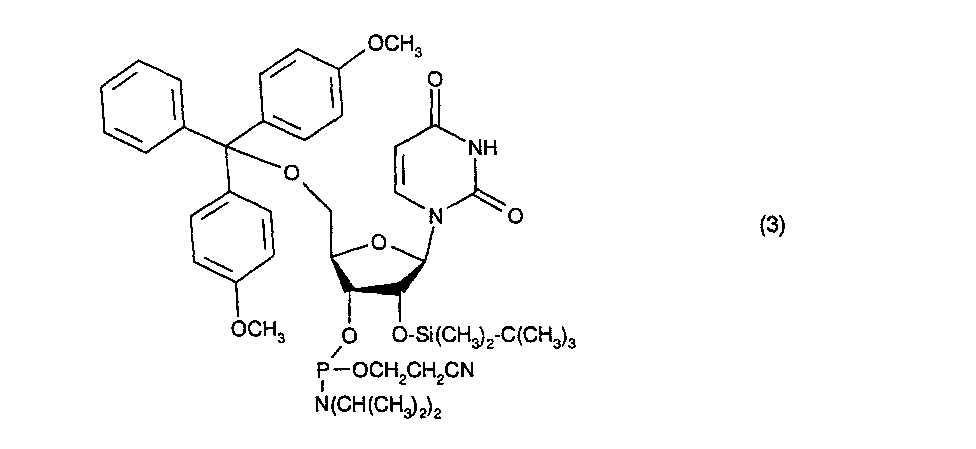

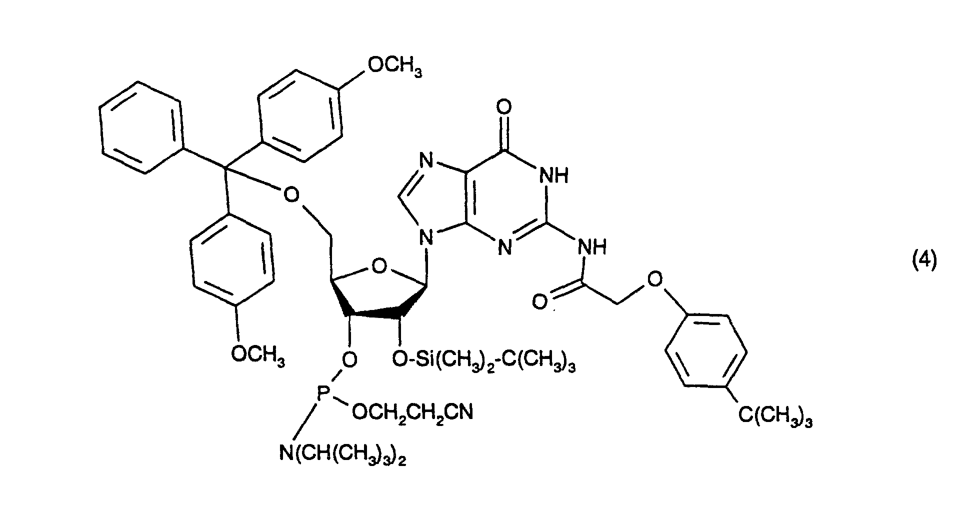

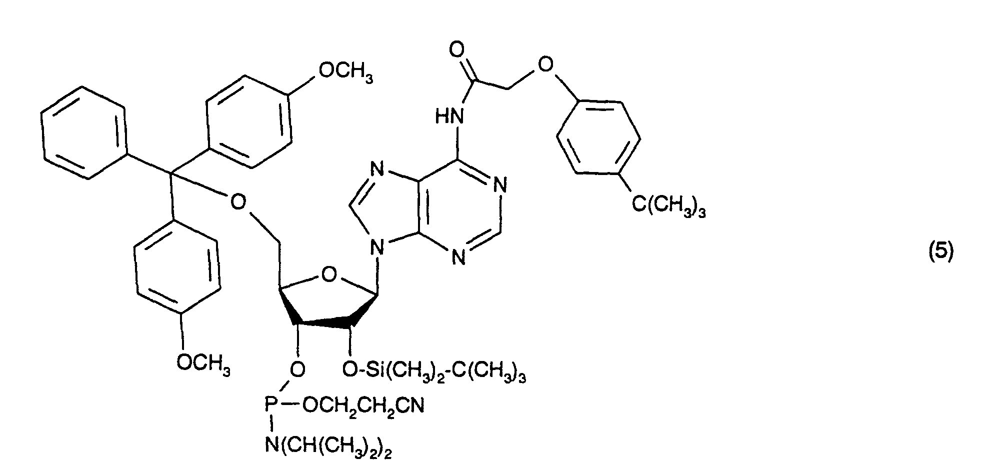

Die Synthesezyklen werden mit dem Syntheseautomat 394 von Applied Biosystem mit einer Veränderung (Kopplungszeit der Phosphoramidite der Ribo-Reihe beträgt 10 Minuten) nach dem Standardprotokoll der Firma Applied Biosystem durchgeführt (User Manual Version 2.0 (1992) 1.0 µMol Cyclus, Appen. I-41).

Als weitere käufliche Reagenzien werden eingesetzt:
0,1 M Phosphoramidit
Tetrazol/Acetonitril: 4 %, 96 %
Tert.-Butylphenoxyessigsäureanhydrid/Pyridin/Tetrahydrofuran: 10 %, 10 %, 80 %
N-Methylimidazol/Tetrahydrofuran: 16%, 84%
Trichloressigsäure/Dimethylchlormethan: 2%, 98%
Jod/Wasser/Pyridin/Tetrahydrofuran: 3%, 2%, 20%, 75%

Synthetisiert werden folgende Substrat-RNAs:

CG-690 5' r(CUA GCC GAC UGC CGA UCU CGC CAC UCU AC)

CG-1352 5' r(CUA GCC GAC UGC CGA UCU CGC UGA CUG AC)

### Beispiel E2: Abspaltung von der Festphase (CPG) und Entschützung der Base

Die Festphase (1.5 µMol Synthese) wird mit 800 µl ammoniakgesättigtem Ethanol versetzt und bei Raumtemperatur über Nacht inkubiert. Der ammoniakgesättigte Ethanol wird aus einem Teil Ethanol und drei Teilen Ammoniak 33% hergestellt. Nach der Inkubation wird die ammoniakgesättigte ethanolische Lösung abdekantiert, mit ammoniakalischem Ethanol das CPG nachgewaschen und die vereinten Lösungen lyophilisiert.

### Beispiel E3: Entschützung der Tertiärbutyl-dimethylsllyl (TBDMS) Schutzgruppe

Die lyophilisierte Probe wird mit 800 µl 1 M Tetrabutylammoniumfluorid-Tetrahydrofuran (TBAF/THF) Lösung versetzt. Die Probe wird 30 Minuten intensiv vermischt. Die Inkubation erfolgt 24 Stunden bei Raumtemperatur unter Lichtausschluss.
Die RNA wird mit 50 mM Triethylamin-hydrogencarbonat (TAHC) Lösung pH 7.0 (1 + 1) gemischt und direkt bei 4°C dialysiert. (Wasser hat Nanopure^{/}-Qualität)

### Beispiel E4: Dialyse

Dialysiert wird 3 mal gegen 7.5 mM TAHC-Lösung pH 7.0. (Die Lösung wird mit Nanopure^{/}-Qualität Wasser hergestellt, mit CO₂ auf pH 7.0 eingestellt und auf 4°C vorgekühlt.) Die Probe wird lyophilisiert und mit Diethylpyrocarbonat-behandeltem [Sambrook, Fritsch, Maniatis, Molecular Cloning, A Laboratory Manual, Second Edition, Cold Spring Habor Laboratory Press (1989)] und autoklaviertem H₂O (DEPC-H₂O) aufgenommen. Ein Aliquot wird zur Konzentrationsbestimmung bei 260 nm eingesetzt. Im weiteren Umgang mit RNA wird immer RNase- und Fremdmetallionen-frei gearbeitet.

### Beispiel E5: 5' Endmarkierung der Substrat-RNA mit ³³[P] γ-ATP

Zur enzymatischen Kinasereaktion werden 100 pMol RNA aus dem obigen Syntheseprotokoll in 20 µl Volumen bei 37°C 20 Minuten inkubiert.
Die Reaktionslösung enthält 0.5 µl T4-Polynukleotidkinase (Promega, 10 Units/µl), 2 µl Kinasepuffer (50 mM Tris-HCI pH 7.5, 10 mM MgCl₂, 5 mM 1,4 Dithio-DL-threitol, 0.1 mM Spermidin) und 0.5 µl ³³[P] γ-ATP (Amersham, >1000 Ci/mMol, 10µCi/µl).

Anschliessend werden 138 µl Tris-HCl/EDTA (10 mM/1 mM, pH 7.5), 2 µl Glycogen (35 mg/ml) und 40 µl NH₄CH₃COO (10 M) hinzugefügt. Nach Zugabe von 600 µl Ethanol wird die Probe 30 Minuten bei -20°C gekühlt und anschliessend 20 Minuten bei 4°C zentrifugiert. Das Pellet wird lyophilisiert, mit 15 µl Auftragspuffer (0.025 % Bromphenolblau, 0.025 % Xylen Cylanol in einer Mischung 1:1 aus 80 % Formamid und 7 M Harnstoff, 20 mM Citronensäure, 1 mM EDTA) versetzt, 1 Minute bei einer Temperatur von 95°C denaturiert, sofort auf Eis gestellt und für die gelelektrophoretische Auftrennung in eine 1.0 cm x 1 mm Tasche aufgetragen. Die gelelektrophoretische Trennung wird 2.5 h bei 55 Watt nach einem Vorlauf von 40 Minuten bei 55 Watt durchgeführt.

### Beispiel E6: Reinigung und Isolierung der kinasierten Substrat-RNA

Für die gelelektrophoretische Auftrennung der Kinasereaktion wird ein 12 %iges Polyacrylamidgel (1 mm x 30 cm x 40 cm) angefertigt. Die Polymerisationsreaktion wird in 170 ml durchgeführt. Hierzu werden 51 ml Acrylamidlösung (40 % Acrylamid/Bisacrylamid 10:1), 17 ml TBE-Puffer (0.89 M Tris-(hydroxymethyl)-aminomethan, 0.89 M Borsäure, 0.02 M Ethylendiamin-tetraessigsäure) und 71.4 g Harnstoff mit der entsprechenden Menge H₂O gemischt. Die Polymerisation wird gestartet mit 170 µl Ammoniumperoxidisulfat Lösung (25 % w/v) und 170 µl TEMED (N,N,N',N', Tetramethylethylendiamin). Das Gel kann nach 1 Stunde verwendet werden. Als Laufpuffer wird 10 fach verdünnter TBE-Puffer verwendet.
Nach der gelelektrophoretischen Auftennung wird die kinasierte RNA mittels eines aufgelegten Röntgenfilms detektiert und aus dem Gel ausgeschnitten. In einer Elektroelutions-Apparatur (Schleicher und Schuell) wird die RNA aus dem Gelstück unter Anlegen von 100 V eluiert (3.3 V/cm). Als Elutionspuffer wird 10 fach verdünnter TBE-Puffer verwendet.
Die isolierte RNA in 360 µl Eluat wird mit 40 µl NaCH₃COO (3M pH 5.2) und 1 ml Ethanol versetzt. Die Probe wird 20 Minuten bei -20 C gekühlt und anschliessend 20 Minuten bei 4°C zentrifugiert. Das Pellet wird lyophilisiert mit 30 µl H₂O aufgenommen. Die Lösung wird nach dem Czerenkow-Protokoll im Szintillationszähler vermessen und auf 12000 cpm/µl eingestellt.

### F Spaltungsexperimente mit Terpyridin-Lanthanid-Oligonukleotidkonjugaten

### Beispiel F1: Substrat-RNA Spaltung mit Oligonukleotid-Lanthanidkomplex-Konjugaten

Für die gelelektrophoretische Auftrennung und Identifizierung der RNA Produkte nach der Spaltreaktion wird ein 12 %iges Long Ranger™ Gel (AT Biochem, modifiziertes Polyacrylamidgel) (0.4 mm x 30 cm x 40 cm) angefertigt. Die Polymerisationsreaktion wird in 90 ml durchgeführt. Hierzu werden 21 ml Long Ranger™ Lösung (50%) 11 ml TBE-Puffer (0.89 M Tris-(hydroxymethyl)-aminomethan, 0.89 M Borsäure, 0.02 M Ethylendiamin-tetraessigsäure) und 37 g Harnstoff mit der entsprechenden Menge H₂O gemischt. Die Polymerisation wird gestartet mit 450 µl Ammoniumperoxidisulfat Lösung (10 % w/v) und 45 µl TEMED. Das Gel kann nach 1 h verwendet werden. Als Laufpuffer wird 16.66 fach verdünnter TBE-Puffer verwendet. Die Trennung erfolgt innerhalb 75 Minuten bei 60 Watt. Nach der gelelektrophoretischen Auftrennung werden die markierten Spaltprodukte (RNA-Oligomere) mittels eines aufgelegten Röntgenfilms oder mittels Phosphorimager detektiert, respektive ausgezählt.

Die Spaltreaktion wird in 10 µl Volumen durchgeführt.
Zu 1 µl Substrat-RNA (12000 cpm) werden 1 µl Oligonukleotidkonjugat (10 µM), 4 µl Tris-HCI Puffer (50 mM pH 7.4 bei 37°C) und die entsprechende Menge H₂O zupipettiert. Diese Mischung wird 1 Minute auf 85°C erhitzt und anschliessend 16 Stunden bei 37°C inkubiert. Die Reaktion wird beendet durch Zugabe von 5 µl Auftragspuffer (0.025 % Bromphenolblau, 0.025 % Xylen Cylanol in einer 1:1 Mischung aus 80 % Formamid mit 7 M Harnstoff, 20 mM Citronensäure und 1 mM EDTA). Für die gelelektrophoretische Trennung werden 7.5 µl der Probe 1 Minute bei 95°C denaturiert, sofort auf Eis gestellt und in eine Geltasche aufgetragen.

Die Substrat-RNA Konzentration wird als 25-facher Überschuss wie folgt abgeschätzt:
Bei 100 pMol Rohprodukt an RNA und einer Ausbeute von 10 % bei der Gelreinigung befinden sich nach dem beschriebenen Protokoll die Endkonzentrationen von 0.04 mM an Substrat-RNA und 1 mM Oligonukleotidkonjugat in der Reaktionsmischung.
Wird nur der Terpyridin-Lanthanidkomplex als Vergleich eingesetzt, so werden 400 mM Komplex benötigt, um etwa die gleiche Spaltung zu erreichen. Dabei handelt es sich um einen 10.000-fachen Überschuss von Komplex zur Substrat-RNA.

### Beispiel F2: Inkubation von Substrat-RNA CG-690 mit Oligonukleotid-Europiumkomplex-Konjugat Verbindung Nr. 3.2

Die Spaltungsreaktion erfolgt prinzipiell wie in Beispiel F1 beschrieben.
(80 % Ausgangsmaterial ungespalten) CG-690 5' r(CUA GCC GAC UGC CGA UCU CGC CAC UCU AC)
Hauptspaltprodukte
   (Σ 15%) 
5' r(CUA GCC GAC UGC CGA UCU CGC CAC UCU Acp
5' r(CUA GCC GAC UGC CGA UCU CGC CAC UCUcp
5' r(CUA GCC GAC UGC CGA UCU CGC Ccp
5' r(CUA GCC GAC UGC CGA UCU CGcp
Weitere Spaltprodukte
   (Σ 5%)

5' r(CUA GCC GAC UGC CGA UCU CGCcp
5' r(CUA GCC GAC UGC CGA UCU Ccp

### Beispiel F3: Inkubation von Substrat-RNA CG-690 mit Oligonukleotid-Lanthankomplex-Konjugat Verbindung Nr. 3.15

Die Spaltungsreaktion erfolgt prinzipiell wie in Beispiel F1 beschrieben.
(80 % Ausgangsmaterial ungespalten) CG-690 | 5' r(CUA GCC GAC UGC CGA UCU CGC CAC UCU AC)
Hauptspaltprodukte
   (Σ 20 %) 
5' r(CUA GCC GAC UGC CGA UCU CGC CAC UCUcp
5' r(CUA GCC GAC UGC CGA UCU CGC Ccp
 (cp = 2',3'-Cyclophosphat)

### Beispiel F4: Inkubation von Substrat-RNA CG-1352 mit Oligonukleotid-Europiumkomplex-Konjugat Verbindung Nr. 3.14

Die Spaltungsreaktion erfolgt prinzipiell wie in Beispiel F1 beschrieben.
(< 5 % Ausgangsmaterial ungespalten) CG-1352 5' r(CUA GCC GAC UGC CGA UCU CGC UGA CUG AC)
Hauptspaltprodukt (> 70 %) 5' r(CUA GCC GAC UGC CGA UCU CGC UGcp)
Restspaltprodukte
   (Σ 25 %)

5' r(CUA GCC GAC UGC CGA UCU CGC Ucp)
5' r(CUA GCC GAC UGC CGA UCU CGC UGAcp)
5' r(CUA GCC GAC UGC CGA UCU CGC UGA Ccp)
5' r(CUA GCC GAC UGC CGA UCU CGC UGA CUcp)
5' r(CUA GCC GAC UGC CGA UCU CGC UGACUGcp)

### Beispiel F5: Weitere Spaltungen der Substrat-RNA CG-1352 mit Oligonukleotid-Terpyridin-Metallkomplex-Konjugaten

Weitere Spaltungsreaktionen werden prinzipiell wie in Beispiel F1 beschrieben durchgeführt. Tabelle 4 zeigt die Resultate von weiteren Spaltungen der Substrat-RNA CG-1352 mit verschiedenen Terpyridin-Metallkomplex-Oligonukleotid-Konjugaten der Tabelle 3. Die Angabe "+3" bedeutet, dass die Hauptspaltung zwischen den Nukleotiden +3 und +4 erfolgt der Substrat-RNA erfolgt (siehe auch Fig. 1 und Fig.2 sowie Erläuterungen dazu). Man erkennt, dass die Spaltung in den dargestellten Fällen bevorzugt an der Position +3 stattfindet.

**Tabelle 4:**

| Hauptspaltprodukte (fettgedruckte Positionen) der Substrat-RNA CG-1352. Angegeben ist das jeweils verwendete Konjugat (siehe auch Tab. 3). | | | | | |
|---|---|---|---|---|---|
| 3.2 | 3.1 | 3.3 | 3.6 | 3.11 | 3.7 |
| **+3G** | **+3G** | **+3G** | **+3G** | **+3G** | **+3G** |
| 3.8 | 3.9 | 3.10 | 3.12 | 3.5 | 3.13 |
| **+3G** | **+3G** | **+3G** | **+3G** | **+3G** | **+3G** |
| 3.4 | 3.30 | 3.31 | 3.26 | 3.27 | 3.28 |
| **+3G** | **+3G** | **+3G** | **+3G** | **+3G** | **+3G** |
| 3.29 | 3.32 | 3.33 | 3.34 | 3.35 | 3.36 |
| **+3G** | **+3G** | **+3G** | **+3G** | **+3G** | **+3G** |
| 3.37 | 3.38 | 3.39 | 3.40 | 3.41 | 3.44 |
| **+3G** | **+3G** | **+3G** | **+3G** | **+3G** | **+3G** |
| 3.42 | 3.43 | 3.15 | 3.48 | 3.49 | 3.45 |
| **+3G** | **+3G** | **+3G** | **+3G** | **+3G** | **+3G** |

## Patentansprüche

1. Verbindung der Formel I 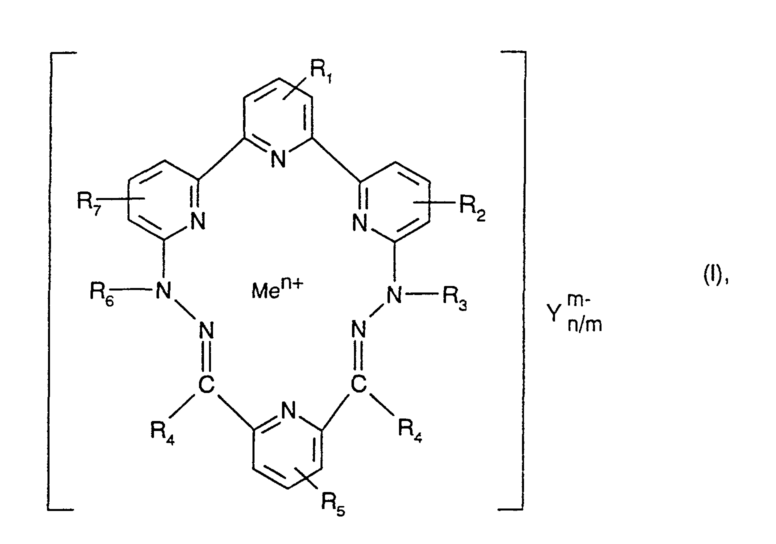 worin
R₁ H, C₁-C₄-Alkyl, C₁-C₄-Alkoxy, C₇-C₁₂-Aralkyl, C₆-C₁₆-Aryl, C₄-C₁₂-Heteroaryl mit O, S oder N als Heteroatome, C₁-C₄-Alkylthio, Di(C₁-C₄-Alkyl)amino, Halogenid, Sulfonamid oder Carboxamid und R₅ eine monovalente funktionelle Gruppe
oder
R₁ eine monovalente funktionelle Gruppe und R₅ H, C₁-C₄-Alkyl, C₁-C₄-Alkoxy, C₇-C₁₂-Aralkyl, C₆-C₁₆-Aryl, C₄-C₁₂-Heteroaryl mit O, S oder N als Heteroatome, C₁-C₄-Alkylthio, Di(C₁-C₄-Alkyl)amino, Halogenid, Sulfonamid oder Carboxamid darstellen,
wobei die funktionelle Gruppe direkt oder über eine Gruppe Z an den Pyridinring gebunden ist und die Gruppe Z einen gegebenenfalls durch -O-, -S-, -NR₁₂-, -C(O)-O- oder -C(O)-NR₁₂- unterbrochenen Rest ausgewählt aus der Gruppe bestehend aus C₁-C₂₀-Alkylen, C₂-C₁₂-Alkenylen, C₂-C₁₂-Alkinylen, C₅-C₈-Cycloalkylen, C₆-C₁₂-Arylen und C₇-C₁₂-Aralkylen bedeutet,
R₂ und R₇ unabhängig voneinander H, C₁-C₄-Alkyl, C₁-C₄-Alkoxy, C₇-C₁₂-Aralkyl, C₆-C₁₆-Aryl, Halogen, C₄-C₁₂-Heteroaryl mit O, S oder N als Heteroatome, C₁-C₄-Alkylthio, Di(C₁-C₄-Alkyl)amino, Sulfonamid oder Carboxamid bedeuten,
R₃ und R₆ unabhängig voneinander H, C₁-C₄-Alkyl, C₇-C₁₂-Aralkyl, C₆-C₁₆-Aryl, Halogen, C₄-C₁₂-Heteroaryl mit O, S oder N als Heteroatome, C₁-C₄-Alkylthio, Di(C₁-C₄-Alkyl)amino, Sulfonamid oder Carboxamid sind,
R₄ für H, C₁-C₂₀-Alkyl, C₅-C₈-Cycloalkyl, C₆-C₁₂-Aryl oder C₇-C₁₂-Aralkyl steht,
R₁₂H oder C₁-C₆-Alkyl bedeutet,
Me für ein Lanthanidmetall oder Yttrium steht,
Y für ein Anion einer Säure steht,
n die Zahlen 2 oder 3 bedeutet, und
m die Zahlen 1, 2 oder 3 bedeutet,
wobei die Reste Alkyl, Cycloalkyl, Aralkyl, Aryl und die Gruppe Z unsubstituiert oder mit C₁-C₄-Alkoxy, F, Cl, Br, -CN, C₁-C₄-Alkyl oder -NO₂ substituiert sind.

2. Verbindung gemäss Anspruch 1, **dadurch gekennzeichnet, dass** R₁ H, C₁-C₄-Alkyl, C₁-C₄-Alkoxy, C₇-C₁₂-Aralkyl, C₆-C₁₆-Aryl, und R₅ eine monovalente funktionelle Gruppe
oder
R₁ eine monovalente funktionelle Gruppe und R₅ H, C₁-C₄-Alkyl, C₁-C₄-Alkoxy, C₇-C₁₂-Aralkyl, C₆-C₁₆-Aryl, darstellen.

3. Verbindung gemäss Anspruch 1 oder 2, **dadurch gekennzeichnet, dass** die Gruppe Z C₁-C₃-Alkylen, C₃-Alkinylen, Phenylen oder C₇-Aralkylen bedeutet.

4. Verbindung gemäss einem der Ansprüche 1 bis 3, **dadurch gekennzeichnet, dass** die Gruppe Z C₂-C₃-Alkylen oder Phenylen ist.

5. Verbindung gemäss einem der Ansprüche 1 bis 4, **dadurch gekennzeichnet, dass** R₂ und R₇ unabhängig voneinander H, C₁-C₄-Alkyl, C₁-C₄-Alkoxy, C₇-C₁₂-Aralkyl, C₆-C₁₆-Aryl, oder Halogen bedeuten.

6. Verbindung gemäss einem der Ansprüche 1 bis 5, **dadurch gekennzeichnet, dass** R₂ und R₇ unabhängig voneinander H oder C₁-C₄-Alkyl bedeuten.

7. Verbindung gemäss einem der Ansprüche 1 bis 6, **dadurch gekennzeichnet, dass** R₃ und R₆ unabhängig voneinander H, C₁-C₄-Alkyl, C₇-C₁₂-Aralkyl oder C₆-C₁₆-Aryl sind.

8. Verbindung gemäss einem der Ansprüche 1 bis 7, **dadurch gekennzeichnet, dass** R₃ und R₆ unabhängig voneinander H oder C₁-C₄-Alkyl sind.

9. Verbindung gemäss einem der Ansprüche 1 bis 8, **dadurch gekennzeichnet, dass** R₄ für H oder C₁-C₂₀-Alkyl steht.

10. Verbindung gemäss einem der Ansprüche 1 bis 9, **dadurch gekennzeichnet, dass** die monovalente funktionelle Gruppe ausgewählt ist aus der Gruppe bestehend aus -OR₁₀, -SR₁₀, -NCO, -NCS, -NHR₁₁, -C(O)OR₁₁, -C(O)SH, -C(O)NHR₁₁, -C(O)Cl, -C(S)SR₁₁, -C(S)NHR₁₁, -C(S)OR₁₁, -SO₃R₁₁, -SO₂NHR₁₁, -SO₂Cl, -P(O)(OH)₂, -P(O)(OH)-NHR₁₁, -P(S)(SH)₂, -P(S)(SH)-NHR₁₁, -P(S)(OH)₂, -P(S)(OH)-NHR₁₁, -P(O)(SH)₂, -P(O)(SH)-NHR₁₁, -P(O)(OH)H, -P(O)(NHR₁₁)H, -P(S)(SH)H, -P(S)(NHR₁₁)H, -P(S)(OH)H, -P(O)(SH)H, wobei R₁₀ H, -C(O)NH₂, -C(S)NH₂, -C₁-C₆-Alkyl, -CₓH₂ₓ-NH₂, -CₓH₂ₓ-SH oder -(CₓH₂ₓO)_{y}-H bedeutet, R₁₁ H, -C₁-C₆-Alkyl, -CₓH₂ₓ-NH₂, -CₓH₂ₓ-SH oder -(CₓH₂ₓO)_{y}-H bedeutet und x gleich einer Zahl von 2 bis 6 und y gleich einer Zahl von 1 bis 20 ist.

11. Verbindung gemäss Anspruch 10, **dadurch gekennzeichnet, dass** R₁₀ für H steht.

12. Verbindung gemäss einem der Ansprüche 1 bis 11, worin die funktionelle Gruppe ausgewählt ist aus der Gruppe bestehend aus -OH, -SH, -NCO, -NCS, -NHR₁₁, -C(O)OR₁₁ und -P(O)(OH)₂.

13. Verbindung gemäss einem der Ansprüche 1 bis 12, worin die funktionelle Gruppe ausgewählt ist aus der Gruppe bestehend aus -NCS, -C(O)OR₁₁ und -P(O)(OH)₂.

14. Verbindung gemäss einem der Ansprüche 1 bis 13, worin das Lanthanidmetall La, Ce, Nd, Eu oder Gd ist.

15. Verbindung gemäss einem der Ansprüche 1 bis 14, worin das Lanthanidmetall La oder Eu ist.

16. Verbindung gemäss einem der Ansprüche 1 bis 15, worin das Lanthanidmetall Eu ist.

17. Verbindung gemäss einem der Ansprüche 1 bis 16, worin das Anion F⁻, Cl⁻, Br⁻, J⁻, PF₆⁻, SbF₆⁻, BF₄⁻, B(Ph)₄⁻, Acetat, NO₃⁻, Sulfat oder Phosphat ist.

18. Verbindung gemäss einem der Ansprüche 1 bis 17, worin das Anion Cl⁻, Acetat oder NO₃⁻ ist.

19. Verbindung der Formel V 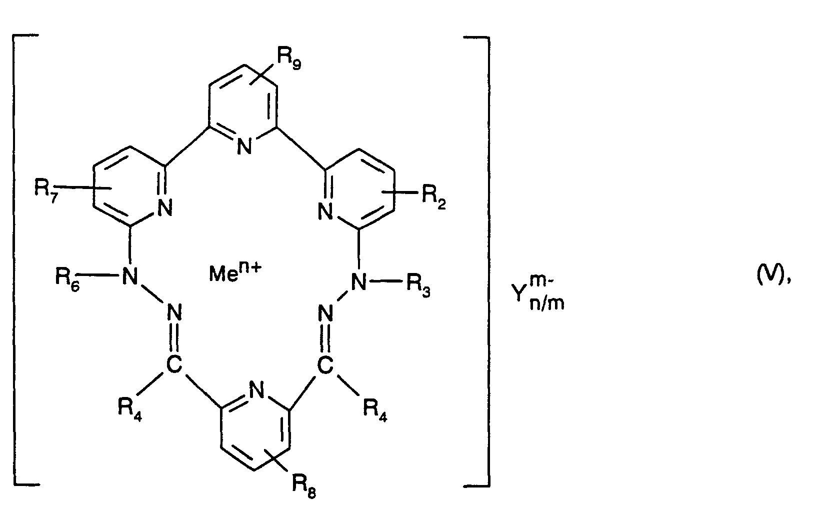 worin
R₂ und R₇ unabhängig voneinander H, C₁-C₄-Alkyl, C₁-C₄-Alkoxy, C₇-C₁₂-Aralkyl, C₆-C₁₆-Aryl, Halogen, C₄-C₁₂-Heteroaryl mit O, S oder N als Heteroatome, C₁-C₄-Alkylthio, Di(C₁-C₄-Alkyl)amino, Sulfonamid oder Carboxamid bedeuten,
R₃ und R₆ unabhängig voneinander H, C₁-C₄-Alkyl, C₇-C₁₂-Aralkyl, C₆-C₁₆-Aryl, Halogen, C₄-C₁₂-Heteroaryl mit O, S oder N als Heteroatome, C₁-C₄-Alkylthio, Di(C₁-C₄-Alkyl)amino, Sulfonamid oder Carboxamid sind,
R₄ für H, C₁-C₂₀-Alkyl, C₅-C₈-Cycloalkyl, C₆-C₁₂-Aryl oder C₇-C₁₂-Aralkyl steht,
wobei die Reste Alkyl, Cycloalkyl, Aralkyl und Aryl unsubstituiert oder mit C₁-C₄-Alkoxy, F, Cl, Br, -CN, C₁-C₄-Alkyl oder -NO₂ substituiert sind,
Me für ein Lanthanidmetall oder Yttrium steht,
Y für ein Anion einer Säure steht,
n die Zahlen 2 oder 3 bedeutet, und
m die Zahlen 1, 2 oder 3 bedeutet,
R₉ einen Rest der Formel VI
-Xₚ-A-X'_{q}-A'ᵣ-Oligo (VI)
darstellt, und R₈ H, C₁-C₄-Alkyl, C₁-C₄-Alkoxy, C₇-C₁₂-Aralkyl, C₆-C₁₆-Aryl, C₄-C₁₂-Heteroaryl mit O, S oder N als Heteroatome, C₁-C₄-Alkylthio, Di(C₁-C₄-Alkyl)amino, Halogenid, Sulfonamid oder Carboxamid bedeutet, oder
R₉ H, C₁-C₄-Alkyl, C₁-C₄-Alkoxy, C₇-C₁₂-Aralkyl, C₆-C₁₆-Aryl C₄-C₁₂-Heteroaryl mit O, S oder N als Heteroatome, C₁-C₄-Alkylthio, Di(C₁-C₄-Alkyl)amino, Halogenid, Sulfonamid oder Carboxamid darstellt und R₈ einen Rest der Formel VI bedeutet,
p, q und r unabhängig voneinander für 0 oder 1 stehen,
X und X' unabhängig voneinander einen unsubstituierten oder mit C₁-C₄-Alkoxy, F, Cl, Br, -CN, C₁-C₄-Alkyl oder -NO₂ substituierten Rest ausgewählt aus der Gruppe bestehend aus C₁-C₂₀-Alkylen, C₂-C₁₂-Alkenylen, C₂-C₁₂-Alkinylen, -(CₓH₂ₓO)_{y}-, worin x gleich einer Zahl von 2 bis 6 und y gleich einer Zahl von 1 bis 20 ist, C₅-C₈-Cycloalkylen, C₆-C₁₂-Arylen und C₇-C₁₂-Aralkylen bedeuten,
A und A'unabhängig voneinander -O-, -S-, -S-S-, -NR₁₂-CO-NR₁₂-, -NR₁₂-CS-NR₁₂-, -NR₁₂-, -NR₁₂-C(O)-O-, -C(O)O-, -C(O)S-, -C(O)NR₁₂-, -C(S)S-, -C(S)O-, -C(S)NR₁₂-, -SO₂NR₁₂-, -SO₂-, -P(O)(OH)O-, -P(S)(SH)S-, -P(S)(SH)O-, -P(S)(OH)0-, -P(O)(SH)S-, -P(O)(OH)S-, P(O)(SH)O-; -P(O)(OH)-NR₁₂-, -P(S)(SH)-NR₁₂-, -P(S)(OH)-NR₁₂-, -P(O)(SH)-NR₁₂-, -HP(O)O-, -HP(S)S-, -HP(O)NR₁₂- oder -HP(S)NR₁₂- bedeuten, wobei R₁₂ für H oder C₁-C₆-Alkyl steht; und
Oligo eine natürliche, modifizierte oder synthetische Sequenz aus natürlichen, modifizierten oder synthetischen Desoxynukleosiden oder Peptidnukleinsäurebausteinen darstellt, die über eine Nukleinbase, eine internukleotidische Brücke oder einen Zucker gebunden ist und deren innerer Bereich komplementär zu einer Ziel-RNA ist.

20. Verbindung gemäss Anspruch 19, **dadurch gekennzeichnet, dass** R₉ einen Rest der Formel VI
-Xₚ-A-X'_{q}-A'ᵣ-Oligo (VI)
darstellt, und R₈ H, C₁-C₄-Alkyl, C₁-C₄-Alkoxy, C₇-C₁₂-Aralkyl, oder C₆-C₁₆-Aryl bedeutet, oder
R₉ H, C₁-C₄-Alkyl, C₁-C₄-Alkoxy, C₇-C₁₂-Aralkyl oder C₆-C₁₆-Aryl darstellt und R₈ einen Rest der Formel VI bedeutet, und
p, q und r unabhängig voneinander für 0 oder 1 stehen.

21. Verbindung gemäss Anspruch 19 oder 20, **dadurch gekennzeichnet, dass** q für 1 steht.

22. Verbindung gemäss einem der Ansprüche 19 bis 21, **dadurch gekennzeichnet, dass** R₂ und R₇ unabhängig voneinander H, C₁-C₄-Alkyl, C₁-C₄-Alkoxy, C₇-C₁₂-Aralkyl, C₆-C₁₆-Aryl oder Halogen bedeuten.

23. Verbindung gemäss einem der Ansprüche 19 bis 22, **dadurch gekennzeichnet, dass** R₂ und R₇ unabhängig voneinander H oder C₁-C₄-Alkyl bedeuten.

24. Verbindung gemäss einem der Ansprüche 19 bis 23, **dadurch gekennzeichnet, dass** R₃ und R₆ unabhängig voneinander H, C₁-C₄-Alkyl, C₇-C₁₂-Aralkyl oder C₆-C₁₆-Aryl bedeuten.

25. Verbindung gemäss einem der Ansprüche 19 bis 24, **dadurch gekennzeichnet, dass** R₃ und R₆ unabhängig voneinander H oder C₁-C₄-Alkyl bedeuten.

26. Verbindung gemäss einem der Ansprüche 19 bis 25, **dadurch gekennzeichnet, dass** R₄ für H oder C₁-C₂₀-Alkyl steht.

27. Verbindung gemäss einem der Ansprüche 19 bis 26, worin das Lanthanidmetall La, Ce, Nd, Eu oder Gd ist.

28. Verbindung gemäss einem der Ansprüche 19 bis 27, worin das Lanthanidmetall La oder Eu ist.

29. Verbindung gemäss einem der Ansprüche 19 bis 28, worin das Lanthanidmetall Eu ist.

30. Verbindung gemäss einem der Ansprüche 19 bis 29, worin das Anion F⁻, Cl⁻, Br⁻, J⁻, PF₆⁻, SbF₆⁻, BF₄⁻, B(Ph)₄⁻, Acetat, NO₃⁻, Sulfat oder Phosphat ist.

31. Verbindung gemäss einem der Ansprüche 19 bis 30, worin das Anion Cl⁻, Acetat oder NO₃⁻ ist.

32. Verbindung gemäss einem der Ansprüche 19 bis 31, worin das Anion Cl⁻ ist.

33. Verbindung gemäss einem der Ansprüche 19 bis 32, **dadurch gekennzeichnet, dass** X C₁-C₃-Alkylen, C₃-Alkinylen, Phenylen oder C₇-Aralkylen bedeutet.

34. Verbindung gemäss einem der Ansprüche 19 bis 33, **dadurch gekennzeichnet, dass** X C₂-C₃-Alkylen oder Phenylen ist.

35. Verbindung gemäss einem der Ansprüche 19 bis 34, **dadurch gekennzeichnet, dass** A - NR₁₂-CS-NR₁₂- oder -C(O)NR₁₂- bedeutet.

36. Verbindung gemäss einem der Ansprüche 19 bis 35, **dadurch gekennzeichnet, dass** A - NH-CS-NH- oder -C(O)NH- bedeutet.

37. Verbindung gemäss einem der Ansprüche 19 bis 36, **dadurch gekennzeichnet, dass** X' C₁-C₂₀-Alkylen bedeutet.

38. Verbindung gemäss einem der Ansprüche 19 bis 37, **dadurch gekennzeichnet, dass** X' C₁-C₁₀-Alkylen bedeutet.

39. Verbindung gemäss einem der Ansprüche 19 bis 38, **dadurch gekennzeichnet, dass** A' nicht vorhanden ist oder -P(O)(OH)O- bedeutet.

40. Verbindung der Formel II 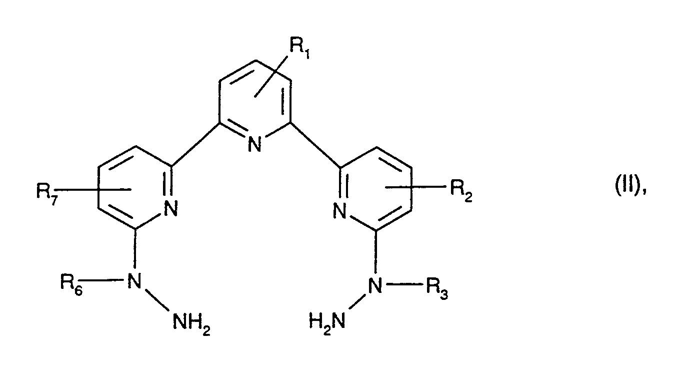 worin
R₁ H, C₁-C₄-Alkyl, C₁-C₄-Alkoxy, C₇-C₁₂-Aralkyl oder C₆-C₁₆-Aryl oder eine monovalente funktionelle Gruppe bedeutet, wobei die funktionelle Gruppe direkt oder über eine Gruppe Z an den Pyridinring gebunden ist und die Gruppe Z für einen gegebenenfalls durch -O-, -S-, -NR₁₂-, -C(O)-O- oder -C(O)-NR₁₂- unterbrochenen Rest ausgewählt aus der Gruppe bestehend aus C₁-C₂₀-Alkylen, C₂-C₁₂-Alkenylen, C₂-C₁₂-Alkinylen, C₅-C₈-Cycloalkylen, C₆-C₁₂-Arylen und C₇-C₁₂-Aralkylen steht,
R₂ und R₇ unabhängig voneinander H, C₁-C₄-Alkyl, C₁-C₄-Alkoxy, C₇-C₁₂-Aralkyl, C₆-C₁₆-Aryl oder Halogen bedeuten, und
R₃ und R₆ unabhängig voneinander H, C₁-C₄-Alkyl, C₇-C₁₂-Aralkyl oder C₆-C₁₆-Aryl sind,
R₁₂ H oder C₁-C₆-Alkyl bedeutet,
wobei die Reste Alkyl, Cycloalkyl, Aralkyl, Aryl und die Gruppe Z unsubstituiert oder mit C₁-C₄-Alkoxy, F, Cl, Br, -CN, C₁-C₄-Alkyl oder -NO₂ substituiert sind,
mit der Massgabe, dass solche Verbindungen der Formel (II) ausgenommen sind, bei denen R₂ und R₇ jeweils für H stehen, R₃ und R₆ jeweils für -CH₃ stehen, und R₁ für H oder für 4-Phenyl steht; oder bei denen R₁ für H steht, R₂ und R₇ jeweils für 4-Phenyl stehen, und R₃ und R₆ jeweils für H oder für -CH₃ stehen.

41. Verbindung der Formel III, 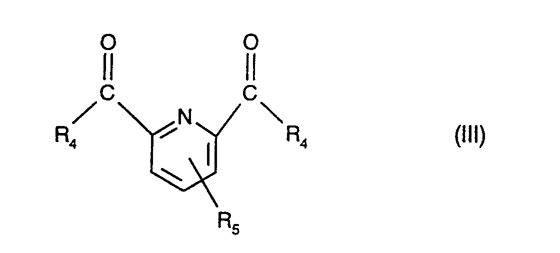 worin
R₅ eine über C₂-C₂₀-Alkylen an den Pyridinring gebundene monovalente funktionelle Gruppe darstellt, wobei die funktionelle Gruppe ausgewählt ist aus der Gruppe bestehend aus -C(O)-OR₁₂, -C(O)-NHR₁₂, -SO₂-R₁₂ und -SO₂-NHR₁₂, wobei R₁₂ H oder C₁-C₆-Alkyl ist, und
R₄ für H oder C₁-C₂₀-Alkyl steht.

42. Verfahren zur Herstellung der Verbindungen der Formel I, **dadurch gekennzeichnet, dass** man ein Terpyridin der Formel II 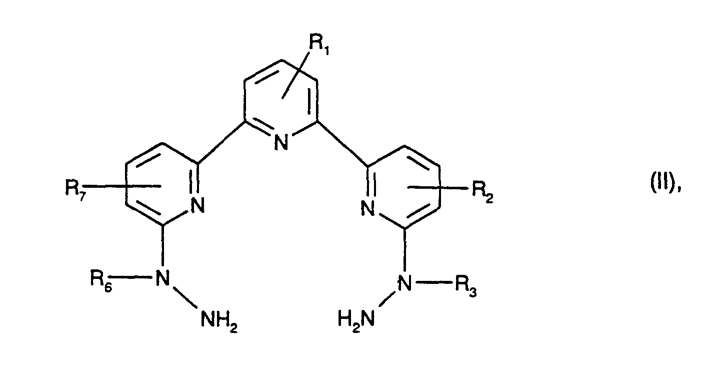 mit einem Pyridindialdehyd oder Pyridindiketon der Formel III 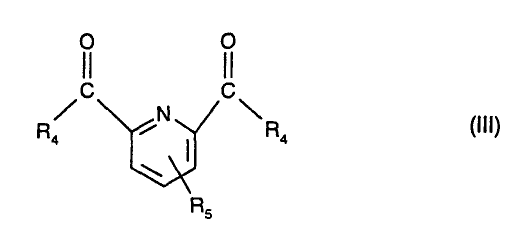 in Gegenwart eines Salzes der Formel IV
Meⁿ⁺(Y^{m-})_{n/m} (IV)
kondensiert, worin R₁, R₂, R₃, R₄, R₅, R₆, R₇, Me, Y, n und m die zuvor angegebenen Bedeutungen haben.

43. Verfahren zur Herstellung von Verbindungen der Formel (V) gemäss einem der Ansprüche 19 bis 39, **dadurch gekennzeichnet, dass** man eine Verbindung der Formel (I) gemäss einem der Ansprüche 1 bis 18
(a) mit einer Verbindung der Formel Vla
A"-X'-A'_{0 oder 1}-Oligo (VIa)
worin
A" eine geeignete monovalente funktionelle Gruppe ist ausgewählt aus der Gruppe bestehend aus -OR₁₀, -SR₁₀, -NCO, -NCS, -NHR₁₁, -C(O)OR₁₁, -C(O)SH, -C(O)NHR₁₁, -C(O)Cl, -C(S)SR₁₁, -C(S)NHR₁₁, -C(S)OR₁₁, -SO₃R₁₁, -SO₂NHR₁₁, -SO₂Cl, -P(O)(OH)₂, -P(O)(OH)-NHR₁₁, -P(S)(SH)₂, -P(S)(SH)-NHR₁₁, -P(S)(OH)₂, -P(S)(OH)-NHR₁₁, -P(O)(SH)₂, -P(O)(SH)-NHR₁₁, -P(O)(OH)H, -P(O)(NHR₁₁)H, -P(S)(SH)H, -P(S)(NHR₁₁)H, -P(S)(OH)H, -P(O)(SH)H, wobei R₁₀ H, -C(O)NH₂, -C(S)NH₂, -C₁-C₆-Alkyl, -CₓH₂ₓ-NH₂, -CₓH₂ₓ-SH oder -(CₓH₂ₓO)_{y}-H bedeutet, und R₁₁ H, -C₁-C₆-Alkyl, -CₓH₂ₓ-NH₂, -CₓH₂ₓ-SH oder -(CₓH₂ₓO)_{y}-H bedeutet, und x gleich einer Zahl von 2 bis 6 und y gleich einer Zahl von 1 bis 20 ist. X' einen unsubstituierten oder mit C₁-C₄-Alkoxy, F, Cl, Br, -CN, C₁-C₄-Alkyl oder -NO₂ substituierten Rest ausgewählt aus der Gruppe bestehend aus C₁-C₂₀-Alkylen, C₂-C₁₂-Alkenylen, C₂-C₁₂-Alkinylen, -(CₓH₂ₓO)_{y}-, worin x gleich einer Zahl von 2 bis 6 und y gleich einer Zahl von 1 bis 20 ist, C₅-C₈-Cycloalkylen, C₆-C₁₂-Arylen und C₇-C₁₂-Aralkylen bedeutet, A'-O-, -S-, -S-S-, -NR₁₂-CO-NR₁₂-, -NR₁₂-CS-NR₁₂-, -NR₁₂-, -NR₁₂-C(O)-O-, -C(O)O-, -C(O)S-, -C(O)NR₁₂-, -C(S)S-, -C(S)O-, -C(S)NR₁₂-, -SO₂NR₁₂-, -SO₂-, -P(O)(OH)O-, -P(S)(SH)S-, -P(S)(SH)O-, -P(S)(OH)O-, -P(O)(SH)S-, -P(O)(OH)S-, -P(O)(SH)O-, -P(O)(OH)-NR₁₂-, -P(S)(SH)-NR₁₂-, -P(S)(OH)-NR₁₂-, -P(O)(SH)-NR₁₂-, -HP(O)O-, -HP(S)S-, -HP(O)NR₁₂- oder -HP(S)NR₁₂- bedeutet, wobei R₁₂ für H oder C₁-C₆-Alkyl steht; und
Oligo eine natürliche, modifizierte oder synthetische Sequenz aus natürlichen, modifizierten oder synthetischen Desoxynukleosiden oder Peptidnukleinsäurebausteinen darstellt, die über eine Nukleinbase, eine internukleotidische Brücke oder einen Zucker gebunden ist und deren innerer Bereich komplementär zu einer Ziel-RNA ist, umsetzt, oder
(b) mit einer Verbindung der Formel Vlb
A"-Oligo (VIb)
worin
A" und Oligo die in (a) beschriebenen Bedeutungen haben, umsetzt.

44. Verfahren gemäss Anspruch 43, **dadurch gekennzeichnet, dass** R₁₀ für H steht.

45. Verfahren zur Spaltung der Phosphatnukleotidbrücke von Ribonukleinsäuren unter physiologischen Bedingungen und unter Einwirkung eines Konjugats aus Metallkomplex und Oligonukleotid, **dadurch gekennzeichnet, dass** man (a) die Ziel-RNA mit einer Verbindung gemäss einem der Ansprüche19 bis 39 komplexiert, und (b) dann reagieren lässt und spaltet.

46. Verbindungen der Formel (V) gemäss einem der Ansprüche 19 bis 39 zur Anwendung in einem therapeutischen Verfahren zur Behandlung von Krankheiten bei Warmblütern einschliesslich des Menschen durch Inaktivierung von Nukleotidsequenzen im Körper.

47. Pharmazeutisches Präparat auf Basis einer wässrigen Lösung oder Suspension, enthaltend eine wirksame Menge der Verbindungen der Formel (V) gemäss einem der Ansprüche 19 bis 39 alleine oder zusammen mit anderen Wirkstoffen, Wasser als pharmazeutisches Trägermaterial und gegebenenfalls Hilfsstoffe.

## Claims

1. A compound of formula I 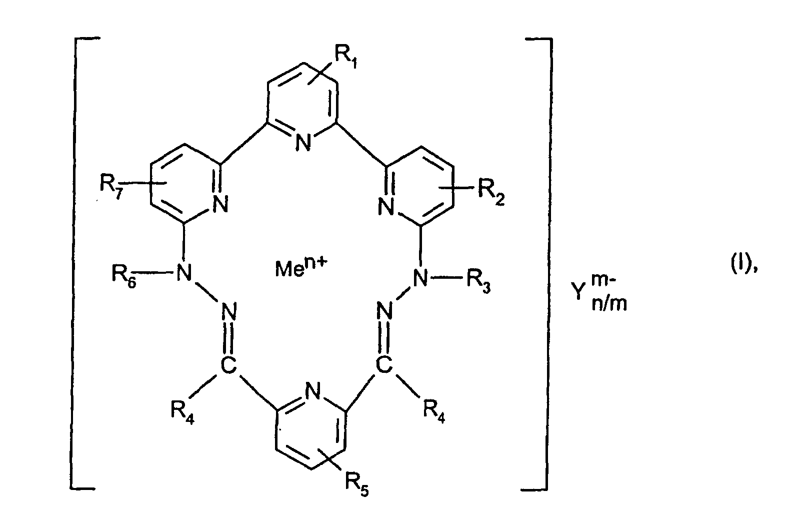 wherein
R₁ is H, C₁-C₄alkyl, C₁-C₄alkoxy, C₇-C₁₂aralkyl, C₆-C₁₆aryl, C₄-C₁₂heteroaryl having O, S or N as hetero atoms, C₁-C₄alkylthio, di(C₁-C₄alkyl)amino, halide, sulfonamide or carboxamide and R₅ is a monovalent functional group
or
R₁ is a monovalent functional group and R₅ is H, C₁-C₄alkyl, C₁-C₄alkoxy, C₇-C₁₂aralkyl, C₆-C₁₆aryl, C₄-C₁₂heteroaryl having O, S or N as hetero atoms, C₁-C₄alkylthio, di(C₁-C₄alkyl)-amino, halide, sulfonamide or carboxamide,
the functional group being bonded to the pyridine ring directly or *via* a group Z and the group Z being a radical selected from the group consisting of C₁-C₂₀alkylene, C₂-C₁₂alkenylene, C₂-C₁₂alkynylene, C₅-C₈cycloalkylene, C₆-C₁₂arylene and C₇-C₁₂aralkylene, which radical is uninterrupted or interrupted by -O-, -S-, -NR₁₂-, -C(O)-O- or by -C(O)-NR₁₂-,
R₂ and R₇ are each independently of the other H, C₁-C₄alkyl, C₁-C₄alkoxy, C₇-C₁₂aralkyl, C₆-C₁₆aryl, halogen, C₄-C₁₂heteroaryl having O, S or N as hetero atoms, C₁-C₄alkylthio, di(C₁-C₄alkyl)amino, sulfonamide or carboxamide,
R₃ and R₆ are each independently of the other H, C₁-C₄alkyl, C₇-C₁₂aralkyl, C₆-C₁₆aryl, halogen, C₄-C₁₂heteroaryl having O, S or N as hetero atoms, C₁-C₄alkylthio, di(C₁-C₄alkyl)-amino, sulfonamide or carboxamide,
R₄ is H, C₁-C₂₀alkyl, C₅-C₈cycloalkyl, C₆-C₁₂aryl or C₇-C₁₂aralkyl,
R₁₂ is H or C₁-C₆alkyl,
Me is a lanthanide metal or yttrium,
Y is an anion of an acid,
n is the number 2 or 3, and
m is the number 1, 2 or 3,
the radicals alkyl, cycloalkyl, aralkyl, aryl and the group Z being unsubstituted or substituted by C₁-C₄alkoxy, F, Cl, Br, -CN, C₁-C₄alkyl or by -NO₂.

2. A compound according to claim 1, wherein R₁ is H, C₁-C₄alkyl, C₁-C₄alkoxy, C₇-C₁₂aralkyl or C₆-C₁₆aryl, and R₅ is a monovalent functional group, or
R₁ is a monovalent functional group and R₅ is H, C₁-C₄alkyl, C₁-C₄alkoxy, C₇-C₁₂aralkyl or C₆-C₁₆aryl.

3. A compound according to claim 1 or 2, wherein the group Z is C₁-C₃alkylene, C₃alkynylene, phenylene or C₇aralkylene.

4. A compound according to any one of claims 1 to 3, wherein the group Z is C₂-C₃alkylene or phenylene.

5. A compound according to any one of claims 1 to 4, wherein R₂ and R₇ are each independently of the other H, C₁-C₄alkyl, C₁-C₄alkoxy, C₇-C₁₂aralkyl, C₆-C₁₆aryl or halogen.

6. A compound according to any one of claims 1 to 5, wherein R₂ and R₇ are each independently of the other H or C₁-C₄alkyl.

7. A compound according to any one of claims 1 to 6, wherein R₃ and R₆ are each independently of the other H, C₁-C₄alkyl, C₇-C₁₂aralkyl or C₆-C₁₆aryl.

8. A compound according to any one of claims 1 to 7, wherein R₃ and R₆ are each independently of the other H or C₁-C₄alkyl.

9. A compound according to any one of claims 1 to 8, wherein R₄ is H or C₁-C₂₀alkyl.

10. A compound according to any one of claims 1 to 9, wherein the monovalent functional group is selected from the group consisting of -OR₁₀, -SR₁₀, -NCO, -NCS, -NHR₁₁, -C(O)OR₁₁, -C(O)SH, -C(O)NHR₁₁, -C(O)Cl, -C(S)SR₁₁, -C(S)NHR₁₁, -C(S)OR₁₁, -SO₃R₁₁, -SO₂NHR₁₁, -SO₂Cl, -P(O)(OH)₂, -P(O)(OH)-NHR₁₁, -P(S)(SH)₂, -P(S)(SH)-NHR₁₁, -P(S)(OH)₂, -P(S)(OH)-NHR₁₁, -P(O)(SH)₂, -P(O)(SH)-NHR₁₁, -P(O)(OH)H, -P(O)(NHR₁₁)H, -P(S)(SH)H, -P(S)(NHR₁₁)H, -P(S)(OH)H and -P(O)(SH)H, with R₁₀ being H, -C(O)NH₂, -C(S)NH₂, -C₁-C₆alkyl, -CₓH₂ₓ-NH₂, -CₓH₂ₓ-SH or-(CₓH₂ₓO)_{y}-H, R₁₁ being H, -C₁-C₆alkyl, -CₓH₂ₓ-NH₂, -CₓH₂ₓ-SH or -(CₓH₂ₓO)_{y}-H and x being a number from 2 to 6 and y being a number from 1 to 20.

11. A compound according to claim 10, wherein R₁₀ is H.

12. A compound according to any one of claims 1 to 11, wherein the functional group is selected from the group consisting of -OH, -SH, -NCO, -NCS, -NHR₁₁, -C(O)OR₁₁ and -P(O)(OH)₂.

13. A compound according to any one of claims 1 to 12, wherein the functional group is selected from the group consisting of -NCS, -C(O)OR₁₁ and -P(O)(OH)₂.

14. A compound according to any one of claims 1 to 13, wherein the lanthanide metal is La, Ce, Nd, Eu or Gd.

15. A compound according to any one of claims 1 to 14, wherein the lanthanide metal is La or Eu.

16. A compound according to any one of claims 1 to 15, wherein the lanthanide metal is Eu.

17. A compound according to any one of claims 1 to 16, wherein the anion is F⁻, Cl⁻, Br⁻, I⁻, PF₆⁻, SbF₆⁻, BF₄⁻, B(Ph)₄⁻, acetate, NO₃⁻, sulfate or phosphate.

18. A compound according to any one of claims 1 to17, wherein the anion is Cl⁻, acetate or NO₃⁻.

19. A compound of formula V 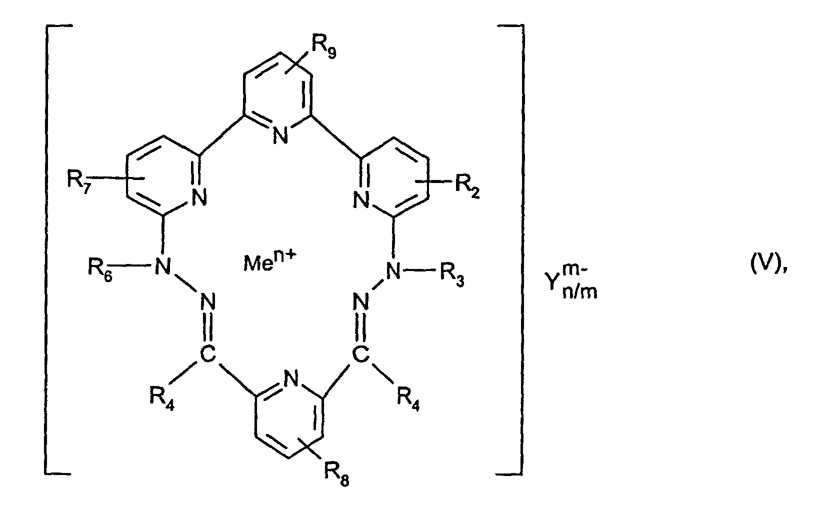 wherein
R₂ and R₇ are each independently of the other H, C₁-C₄alkyl, C₁-C₄alkoxy, C₇-C₁₂aralkyl, C₆-C₁₆aryl, halogen, C₄-C₁₂heteroaryl having O, S or N as hetero atoms, C₁-C₄alkylthio, di(C₁-C₄alkyl)amino, sulfonamide or carboxamide,
R₃ and R₆ are each independently of the other H, C₁-C₄alkyl, C₇-C₁₂aralkyl,C₆-C₁₆aryl, halogen, C₄-C₁₂heteroaryt having O, S or N as hetero atoms, C₁-C₄alkylthio, di(C₁-C₄alkyl)-amino, sulfonamide or carboxamide,
R₄ is H, C₁-C₂₀alkyl, C₅-C₈cycloalkyl, C₆-C₁₂aryl or C₇-C₁₂aralkyl,
the radicals alkyl, cycloalkyl, aralkyl and aryl being unsubstituted or substituted by C₁-C₄-alkoxy, F, Cl, Br, -CN, C₁-C₄alkyl or by -NO₂,
Me is a lanthanide metal or yttrium,
Y is an anion of an acid,
n is the number 2 or 3, and
m is the number 1, 2 or 3,
R₉ is a radical of formula VI
-Xₚ-A-X'_{q}-A'ᵣ-oligo (VI)
and R₈ is H, C₁-C₄alkyl, C₁-C₄alkoxy, C₇-C₁₂aralkyl, C₆-C₁₆aryl, C₄-C₁₂heteroaryl having O, S or N as hetero atoms, C₁-C₄alkylthio, di(C₁-C₄alkyl)amino, halide, sulfonamide or carboxamide, or
R₉ is H, C₁-C₄alkyl, C₁-C₄alkoxy, C₇-C₁₂aralkyl, C₆-C₁₆aryl, C₄-C₁₂heteroaryl having O, S or N as hetero atoms, C₁-C₄alkylthio, di(C₁-C₄alkyl)amino, halide, sulfonamide or carboxamide and R₈ is a radical of formula VI,
p, q and r are each independently of the others 0 or 1,
X and X' are each independently of the other a radical selected from the group consisting of C₁-C₂₀alkylene, C₂-C₁₂alkenylene, C₂-C₁₂alkynylene, -(CₓH₂ₓO)_{y}-, wherein x is a number from 2 to 6 and y is a number from 1 to 20, C₅-C₈cycloalkylene, C₆-C₁₂arylene and C₇-C₁₂aralkylene, which radical is unsubstituted or substituted by C₁-C₄alkoxy, F, Cl, Br, -CN, C₁-C₄alkyl or by -NO₂,
A and A' are each independently of the other -O-, -S-, -S-S-, -NR₁₂-CO-NR₁₂-, -NR₁₂-CS-NR₁₂-, -NR₁₂-, -NR₁₂-C(O)-O-, -C(O)O-, -C(O)S-, -C(O)NR₁₂-, -C(S)S-, -C(S)O-, -C(S)NR₁₂-, -SO₂NR₁₂-, -SO₂-, -P(O)(OH)O-, -P(S)(SH)S-, -P(S)(SH)O-, -P(S)(OH)O-, -P(O)(SH)S-, -P(O)(OH)S-, -P(O)(SH)O-, -P(O)(OH)-NR₁₂-, -P(S)(SH)-NR₁₂-, -P(S)(OH)-NR₁₂-, -P(O)(SH)-NR₁₂-, -HP(O)O-, -HP(S)S-, -HP(O)NR₁₂- or -HP(S)NR₁₂-, with R₁₂ being H or C₁-C₆alkyl; and
"oligo" denotes a natural, modified or synthetic sequence of natural, modified or synthetic deoxynucleosides or peptide nucleic acid building blocks that is bonded *via* a nucleic base, an internucleotidic bridge or a sugar and the internal region of which is complementary to a target RNA.

20. A compound according to claim 19, wherein R₉ is a radical of formula VI
-Xₚ-A-X'_{q}-A'ᵣ-oligo (VI)
and R₈ is H, C₁-C₄alkyl, C₁-C₄alkoxy, C₇-C₁₂aralkyl or C₆-C₁₆aryl, or
R₉ is H, C₁-C₄alkyl, C₁-C₄alkoxy, C₇-C₁₂aralkyl or C₆-C₁₆aryl and R₈ is a radical of formula VI, and
p, q and r are each independently of the others 0 or 1.

21. A compound according to claim 19 or 20, wherein q is 1.

22. A compound according to any one of claims 19 to 21, wherein R₂ and R₇ are each independently of the other H, C₁-C₄alkyl, C₁-C₄alkoxy, C₇-C₁₂aralkyl, C₆-C₁₆aryl or halogen.

23. A compound according to any one of claims 19 to 22, wherein R₂ and R₇ are each independently of the other H or C₁-C₄alkyl.

24. A compound according to any one of claims 19 to 23, wherein R₃ and R₆ are each independently of the other H, C₁-C₄alkyl, C₇-C₁₂aralkyl or C₆-C₁₆aryl.

25. A compound according to any one of claims 19 to 24, wherein R₃ and R₆ are each independently of the other H or C₁-C₄alkyl.

26. A compound according to any one of claims 19 to 25, wherein R₄ is H or C₁-C₂₀alkyl.

27. A compound according to any one of claims 19 to 26, wherein the lanthanide metal is La, Ce, Nd, Eu or Gd.

28. A compound according to any one of claims 19 to 27, wherein the lanthanide metal is La or Eu.

29. A compound according to any one of claims 19 to 28, wherein the lanthanide metal is Eu.

30. A compound according to any one of claims 19 to 29, wherein the anion is F⁻, Cl⁻, Br⁻, I⁻, PF₆⁻, SbF₆⁻, BF₄⁻, B(Ph)₄⁻, acetate, NO₃⁻, sulfate or phosphate.

31. A compound according to any one of claims 19 to 30, wherein the anion is Cl⁻, acetate or NO₃⁻.

32. A compound according to any one of claims 19 to 31, wherein the anion is Cl⁻.

33. A compound according to any one of claims 19 to 32, wherein X is C₁-C₃alkylene, C₃alkynylene, phenylene or C₇aralkylene.

34. A compound according to any one of claims 19 to 33, wherein X is C₂-C₃alkylene or phenylene.

35. A compound according to any one of claims 19 to 34, wherein A is -NR₁₂-CS-NR₁₂- or -C(O)NR₁₂-.

36. A compound according to any one of claims 19 to 35, wherein A is -NH-CS-NH- or -C(O)NH-.

37. A compound according to any one of claims 19 to 36, wherein X' is C₁-C₂₀alkylene.

38. A compound according to any one of claims 19 to 37, wherein X' is C₁-C₁₀alkylene.

39. A compound according to any one of claims 19 to 38, wherein A' is absent or is -P(O)(OH)O-.

40. A compound of formula II 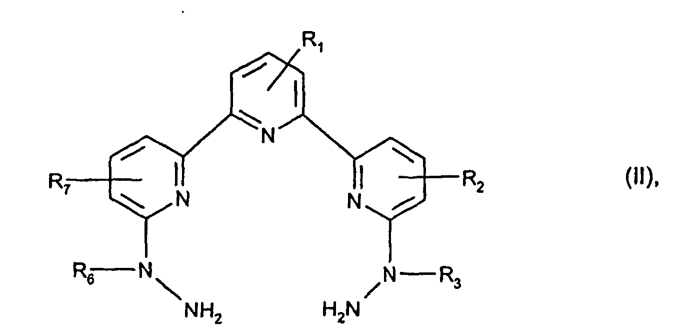 wherein
R₁ is H, C₁-C₄alkyl, C₁-C₄alkoxy, C₇-C₁₂aralkyl or C₆-C₁₆aryl or a monovalent functional group, the functional group being bonded to the pyridine ring directly or *via* a group Z and the group Z being a radical selected from the group consisting of C₁-C₂₀alkylene, C₂-C₁₂-alkenylene, C₂-C₁₂alkynylene, C₅-C₈cycloalkylene, C₆-C₁₂arylene and C₇-C₁₂aralkylene, which radical is uninterrupted or interrupted by -O-, -S-, -NR₁₂-, -C(O)-O- or by -C(O)-NR₁₂-, R₂ and R₇ are each independently of the other H, C₁-C₄alkyl, C₁-C₄alkoxy, C₇-C₁₂aralkyl, C₆-C₁₆aryl or halogen, and
R₃ and R₆ are each independently of the other H, C₁-C₄alkyl, C₇-C₁₂aralkyl or C₆-C₁₆aryl, R₁₂ is H or C₁-C₆alkyl,
the radicals alkyl, cycloalkyl, aralkyl, aryl and the group Z being unsubstituted or substituted by C₁-C₄alkoxy, F, Cl, Br, -CN, C₁-C₄alkyl or by -NO₂,
with the proviso that compounds of formula (II) wherein R₂ and R₇ are each H, R₃ and R₆ are each -CH₃ and R₁ is H or 4-phenyl; or wherein R₁ is H, R₂ and R₇ are each 4-phenyl and R₃ and R₆ are each H or -CH₃, are excluded.

41. A compound of formula III 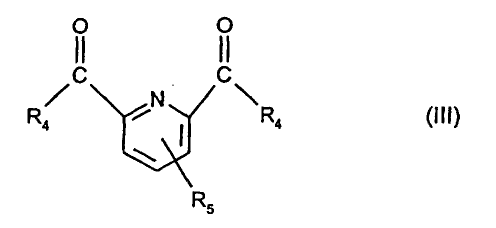 wherein
R₅ is a monovalent functional group bonded to the pyridine ring *via* C₂-C₂₀alkylene, the functional group being selected from the group consisting of -C(O)-OR₁₂, -C(O)-NHR₁₂, -SO₂-R₁₂ and -SO₂-NHR₁₂, with R₁₂ being H or C₁-C₆alkyl, and
R₄ is H or C₁-C₂₀alkyl.

42. A process for the preparation of compounds of formula I, which process comprises condensing a terpyridine of formula II 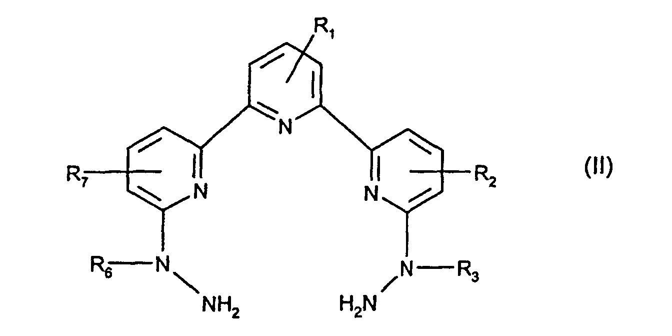 with a pyridine dialdehyde or pyridine diketone of formula III 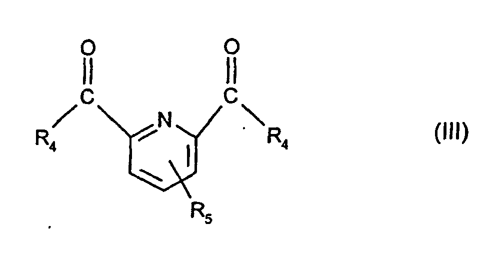 in the presence of a salt of formula IV
Meⁿ⁺(Y^{m-})_{n/m} (IV),
wherein R₁, R₂, R₃, R₄, R₅, R₆, R₇, Me, Y, n and m are as defined above.

43. A process for the preparation of compounds of formula (V) according to any one of claims 19 to 39, wherein a compound of formula (I) according to any one of claims 1 to 18 is reacted
(a) with a compound of formula VIa
A"-X'-A'_{0 or 1}-oligo (VIa)
wherein
A" is a suitable monovalent functional group selected from the group consisting of -OR₁₀, -SR₁₀, -NCO, -NCS, -NHR₁₁, -C(O)OR₁₁, -C(O)SH, -C(O)NHR₁₁, -C(O)Cl, -C(S)SR₁₁, -C(S)NHR₁₁, -C(S)OR₁₁, -SO₃R₁₁, -SO₂NHR₁₁, -SO₂Cl, -P(O)(OH)₂, -P(O)(OH)-NHR₁₁, -P(S)(SH)₂, -P(S)(SH)-NHR₁₁, -P(S)(OH)₂, -P(S)(OH)-NHR₁₁, -P(O)(SH)₂, -P(O)(SH)-NHR₁₁, -P(O)(OH)H, -P(O)(NHR₁₁)H, -P(S)(SH)H, -P(S)(NHR₁₁)H, -P(S)(OH)H and -P(O)(SH)H, with R₁₀ being H, -C(O)NH₂, -C(S)NH₂, -C₁-C₆alkyl, -CₓH₂ₓ-NH₂, -CₓH₂ₓ-SH or -(CₓH₂ₓO)_{y}-H and R₁₁ being H, -C₁-C₆alkyl, -CₓH₂ₓ-NH₂, -CₓH₂ₓ-SH or -(CₓH₂ₓO)_{y}-H, and x being a number from 2 to 6 and y being a number from 1 to 20,
X' is a radical selected from the group consisting of C₁-C₂₀alkylene, C₂-C₁₂alkenylene, C₂-C₁₂alkynylene, -(CₓH₂ₓO)_{y}-, wherein x is a number from 2 to 6 and y is a number from 1 to 20, C₅-C₈cycloalkylene, C₆-C₁₂arylene and C₇-C₁₂aralkylene, which radical is unsubstituted or substituted by C₁-C₄alkoxy, F, Cl, Br, -CN, C₁-C₄alkyl or by -NO₂,
A' is -O-, -S-, -S-S-, -NR₁₂-CO-NR₁₂-, -NR₁₂-CS-NR₁₂-, -NR₁₂-, -NR₁₂-C(O)-O-, -C(O)O-, -C(O)S-, -C(O)NR₁₂-, -C(S)S-, -C(S)O-, -C(S)NR₁₂-, -SO₂NR₁₂-, -SO₂-, -P(O)(OH)O-, -P(S)(SH)S-, -P(S)(SH)O-, -P(S)(OH)O-, -P(O)(SH)S-, -P(O)(OH)S-, -P(O)(SH)O-, -P(O)(OH)-NR₁₂-, -P(S)(SH)-NR₁₂-, -P(S)(OH)-NR₁₂-, -P(O)(SH)-NR₁₂-, -HP(O)O-, -HP(S)S-, -HP(O)NR₁₂- or -HP(S)NR₁₂-, with R₁₂ being H or C₁-C₆alkyl; and
"oligo" denotes a natural, modified or synthetic sequence of natural, modified or synthetic deoxynucleosides or peptide nucleic acid building blocks that is bonded *via* a nucleic base, an internucleotidic bridge or a sugar and the internal region of which is complementary to a target RNA, or
(b) with a compound of formula VIb
A"-oligo (VIb)
wherein
A" and oligo are as defined in (a).

44. A process according to claim 43, wherein R₁₀ is H.

45. A method of cleaving the phosphate nucleotide bridge of ribonucleic acids under physiological conditions and under the action of a conjugate of metal complex and oligonucleotide, in which method (a) the target RNA is complexed with a compound according to any one of claims 19 to 39, and (b) then allowed to react and cleaved.

46. A compound of formula (V) according to any one of claims 19 to 39 for use in a therapeutic method for the treatment of diseases in warm-blooded animals, including human beings, by the inactivation of nucleotide sequences in the body.

47. A pharmaceutical composition based on an aqueous solution or suspension comprising an effective amount of a compound of formula (V) according to any one of claims 19 to 39 alone or together with other active ingredients, water as pharmaceutical carrier and optionally excipients.

## Revendications

1. Composé de formule I 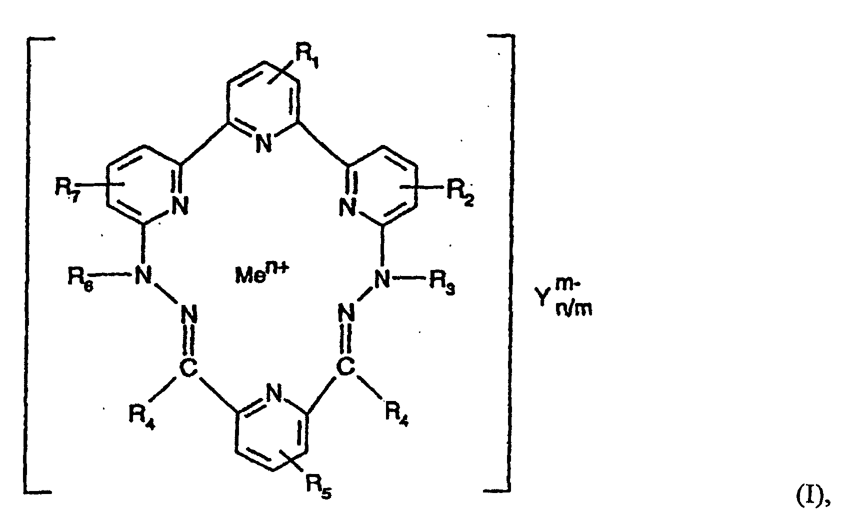 dans laquelle
R₁ est H, un alkyle en C₁ à C₄, un alcoxy en C₁ à C₄, un aralkyle en C₇ à C₁₂, un aryle en C₆ à C₁₆, un hétéroaryle en C₄ à C₁₂ avec O, S ou N en tant qu'hétéroatomes, un alkylthio en C₁ à C₄, un di(alkyle en C₁ à C₄)amino, un halogénure, un sulfonamide ou un carboxamide, et R₅ représente un groupe fonctionnel monovalent,
ou
R₁ représente un groupe fonctionnel monovalent et R₅ est H, un alkyle en C₁ à C₄, un alcoxy en C₁ à C₄, un aralkyle en C₇ à C₁₂, un aryle en C₆ à C₁₆, un hétéroaryle en C₄ à C₁₂ avec O, S ou N en tant qu'hétéroatomes, un alkylthio en C₁ à C₄, un di(alkyle en C₁ à C₄)amino, un halogénure, un sulfonamide ou un carboxamide,
le groupe fonctionnel étant lié directement ou par un groupe Z au cycle pyridine, et le groupe Z représentant un radical, éventuellement interrompu par -O-, -S-, -NR₁₂-, -C(O)-O- ou -C(O)-NR₁₂, choisi dans le groupe composé d'un alkylène en C₁ à C₂₀, un alcénylène en C₂ à C₁₂, un alcynylène en C₂ à C₁₂, un cycloalkylène en C₅ à C₈, un arylène en C₆ à C₁₂ et un aralkylène en C₇ à C₁₂,
R₂ et R₇ représentent, indépendamment l'un de l'autre, H, un alkyle en C₁ à C₄, un alcoxy en C₁ à C₄, un aralkyle en C₇ à C₁₂, un aryle en C₆ à C₁₆, un halogène, un hétéroaryle en C₄ à C₁₂ avec O, S ou N en tant qu'hétéroatomes, un alkylthio en C₁ à C₄, un di(alkyle en C₁ à C₄)amino, un sulfonamide ou un carboxamide,
R₃ et R₆ représentent, indépendamment l'un de l'autre, H, un alkyle en C₁ à C₄, un aralkyle en C₇ à C₁₂, un aryle en C₆ à C₁₆, un halogène, un hétéroaryle en C₄ à C₁₂ avec O, S ou N en tant qu'hétéroatomes, un alkylthio en C₁ à C₄, un di(alkyle en C₁ à C₄)amino, un sulfonamide ou un carboxamide,
R₄ est H, un alkyle en C₁ à C₂₀, un cycloalkyle en C₅ à C₈, un aryle en C₆ à C₁₂ ou un aralkyle en C₇ à C₁₂,
R₁₂ est H ou un alkyle en C₁ à C₆,
Me est un métal des lanthanides ou l'yttrium,
Y est un anion d'un acide,
n vaut 2 ou 3, et
m vaut 1, 2 ou 3,
les radicaux alkyle, cycloalkyle, aralkyle, aryle et le groupe Z étant non substitués ou substitués par un alcoxy en C₁ à C₄, F, Cl, Br, -CN, un alkyle en C₁ à C₄ ou -NO₂.

2. Composé selon la revendication 1, **caractérisé en ce que** R₁ est H, un alkyle en C₁ à C₄, un alcoxy en C₁ à C₄, un aralkyle en C₇ à C₁₂, un aryle en C₆ à C₁₆, et R₅ est un groupe fonctionnel monovalent,
ou
R₁ est un groupe fonctionnel monovalent, et R₅ est H, un alkyle en C₁ à C₄, un alcoxy en C₁ à C₄, un aralkyle en C₇ à C₁₂, un aryle en C₆ à C₁₆.

3. Composé selon la revendication 1 ou 2, **caractérisé en ce que** le groupe Z est un alkylène en C₁ à C₃, un alcynylène en C₃, un phénylène ou un aralkylène en C₇.

4. Composé selon l'une des revendications 1 à 3, **caractérisé en ce que** le groupe Z est un alkylène en C₂ à C₃ ou un phénylène.

5. Composé selon l'une des revendications 1 à 4, **caractérisé en ce que** R₂ et R₇, indépendamment l'un de l'autre, représentent H, un alkyle en C₁ à C₄, un alcoxy en C₁ à C₄, un aralkyle en C₇ à C₁₂, un aryle en C₆ à C₁₆ ou un halogène.

6. Composé selon l'une des revendications 1 à 5, **caractérisé en ce que** R₂ et R₇, indépendamment l'un de l'autre, représentent H ou un alkyle en C₁ à C₄.

7. Composé selon l'une des revendications 1 à 6, **caractérisé en ce que** R₃ et R₆, indépendamment l'un de l'autre, représentent H, un alkyle en C₁ à C₄, un aralkyle en C₇ à C₁₂ ou un aryle en C₆ à C₁₆.

8. Composé selon l'une des revendications 1 à 7, **caractérisé en ce que** R₃ et R₆, indépendamment l'un de l'autre, représentent H ou un alkyle en C₁ à C₄.

9. Composé selon l'une des revendications 1 à 8, **caractérisé en ce que** R₄ représente H ou un alkyle en C₁ à C₂₀.

10. Composé selon l'une des revendications 1 à 9, **caractérisé en ce que** le groupe fonctionnel monovalent est choisi dans le groupe composé de -OR₁₀, SR₁₀, -NCO, -NCS, -NHR₁₁, -C(O)OR₁₁, -C(O)SH, -C(O)NHR₁₁, -C(O)Cl, -C(S)SR₁₁, -C(S)NHR₁₁, -C(S)OR₁₁, -SO₃R₁₁, -SO₂NHR₁₁, -SO₂Cl, -P(O)(OH)₂, -P(O)(OH)-NHR₁₁, -P(S)(SH)₂, -P(S)(SH)-NHR₁₁, -P(S)(OH)₂, -P(S)(OH)-NHR₁₁, -P(O)(SH)₂, -P(O)(SH)-NHR₁₁, -P(O)(OH)H, -P(O)(NHR₁₁)H, -P(S)(SH)H, -P(S)(NHR₁₁)H, -P(S)(OH)H, -P(O)(SH)H, où R₁₀ représente H, -C(O)NH₂, -C(S)NH₂, -alkyle en C₁ à C₆, -CₓH₂ₓ-NH₂, -CₓH₂ₓ-SH ou -(CₓH₂ₓO)_{y}-H, où R₁₁ représente H, -alkyle en C₁ à C₆, -CₓH₂ₓ-NH₂, -CₓH₂ₓ-SH ou -(CₓH₂ₓO)_{y}-H et x est un nombre compris entre 2 et 6 et y est un nombre compris entre 1 et 20.

11. Composé selon la revendication 10, **caractérisé en ce que** R₁₀ représente H.

12. Composé selon l'une des revendications 1 à 11, dans lequel le groupe fonctionnel est choisi dans le groupe composé de -OH, -SH, -NCO, -NCS, -NHR₁₁, -C(O)OR₁₁ et -P(O)(OH)₂.

13. Composé selon l'une des revendications 1 à 12, dans lequel le groupe fonctionnel est choisi dans le groupe composé de -NCS, -C(O)OR₁₁ et -P(O)(OH)₂.

14. Composé selon l'une des revendications 1 à 13, dans lequel le métal des lanthanides est La, Ce, Nd, Eu ou Gd.

15. Composé selon l'une des revendications 1 à 14, dans lequel le métal des lanthanides est La ou Eu.

16. Composé selon l'une des revendications 1 à 15, dans lequel le métal des lanthanides est Eu.

17. Composé selon l'une des revendications 1 à 16, dans lequel l'anion est F⁻, Cl⁻, Br⁻, J⁻, PF₆⁻, SbF₆⁻, BF₄⁻, B(Ph)₄⁻, acétate, NO₃⁻, sulfate ou phosphate.

18. Composé selon l'une des revendications 1 à 17, dans lequel l'anion est Cl⁻, acétate ou NO₃⁻.

19. Composé de formule V 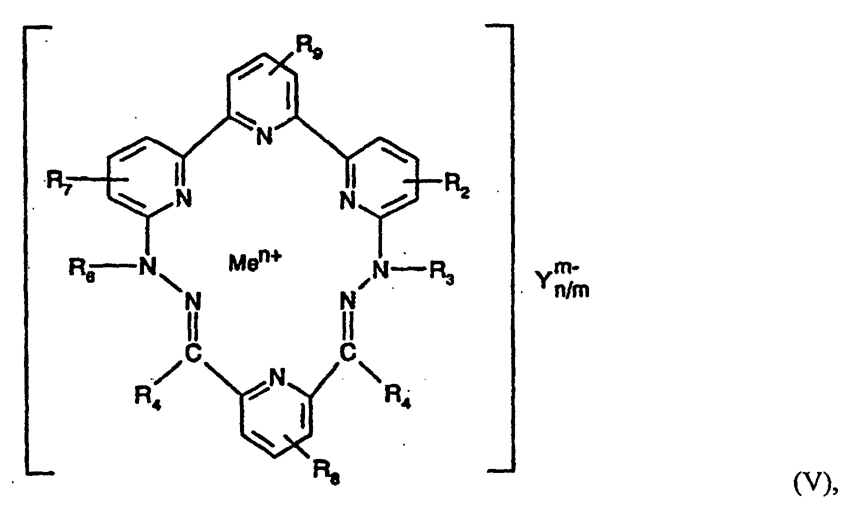 dans laquelle
R₂ et R₇ représentent, indépendamment l'un de l'autre, H, un alkyle en C₁ à C₄, un alcoxy en C₁ à C₄, un aralkyle en C₇ à C₁₂, un aryle en C₆ à C₁₆, un halogène, un hétéroaryle en C₄ à C₁₂ avec O, S ou N en tant qu'hétéroatomes, un alkylthio en C₁ à C₄, un di(alkyle en C₁ à C₄)amino, un sulfonamide ou un carboxamide,
R₃ et R₆ représentent, indépendamment l'un de l'autre, H, un alkyle en C₁ à C₄, un aralkyle en C₇ à C₁₂, un aryle en C₆ à C₁₆, un halogène, un hétéroaryle en C₄ à C₁₂ avec O, S ou N en tant qu'hétéroatomes, un alkylthio en C₁ à C₄, un di(alkyle en C₁ à C₄)amino, un sulfonamide ou un carboxamide,
R₄ est H, un alkyle en C₁ à C₂₀, un cycloalkyle en C₅ à C₈, un aryle en C₆ à C₁₂ ou un aralkyle en C₇ à C₁₂,
les radicaux alkyle, cycloalkyle, aralkyle et aryle étant non substitués ou substitués par un alcoxy en C₁ à C₄, F, Cl, Br, -CN, un alkyle en C₁ à C₄ ou -NO₂,
Me est un métal des lanthanides ou l'yttrium,
Y est un anion d'un acide,
n vaut 2 ou 3, et
m vaut 1,2 ou 3,
R₉ est un radical de formule VI
-Xₚ-A-X'_{q}-A'ᵣ-oligo (VI)
et R₈ représente H, un alkyle en C₁ à C₄, un alcoxy en C₁ à C₄, un aralkyle en C₇ à C₁₂, un aryle en C₆ à C₁₆, un hétéroaryle en C₄ à C₁₂ avec O, S ou N en tant qu'hétéroatomes, un alkylthio en C₁ à C₄, un di(alkyle en C₁ à C₄)amino, un halogénure, un sulfonamide ou un carboxamide,
R₉ est H, un alkyle en C₁ à C₄, un alcoxy en C₁ à C₄, un aralkyle en C₇ à C₁₂, un aryle en C₆ à C₁₆, un hétéroaryle en C₄ à C₁₂ avec O, S ou N en tant qu'hétéroatomes, un alkylthio en C₁ à C₄, un di(alkyle en C₁ à C₄)amino, un halogénure, un sulfonamide ou un carboxamide, et R₈ est un radical de formule VI,
p, q et r valent, indépendamment l'un de l'autre, 0 ou 1,
X et X' sont, indépendamment l'un de l'autre, un radical non substitué ou substitué par un alcoxy en C₁ à C₄, F, Cl, Br, -CN, un alkyle en C₁ à C₄ ou -NO₂, choisi dans le groupe composé d'un alkylène en C₁ à C₂₀, un alcénylène en C₂ à C₁₂, un alcynylène en C₂ à C₁₂, -(CₓH₂ₓO)_{y}-, où x est un nombre compris entre 2 et 6 et y est un nombre compris entre 1 et 20, un cycloalkylène en C₅ à C₈, un arylène en C₆ à C₁₂ et un aralkylène en C₇ à C₁₂,
A et A' sont, indépendamment l'un de l'autre, -O-, -S-, -S-S-, -NR₁₂-CO-NR₁₂-, -NR₁₂-CS-NR₁₂-, -NR₁₂-, -NR₁₂-C(O)-O-, -C(O)O-, -C(O)S-, -C(O)NR₁₂-, -C(S)S-, -C(S)O-, -C(S)NR₁₂, -SO₂NR₁₂-, -SO₂-, -P(O)(OH)O-, -P(S)(SH)S-, -P(S)(SH)O-, -P(S)(OH)O-, -P(O)(SH)S-, -P(O)(OH)S-, -P(O)(SH)O-, -P(O)(OH)-NR₁₂-, -P(S)(SH)-NR₁₂-, -P(S)(OH)-NR₁₂-, -P(O)(SH)-NR₁₂-, -HP(O)O-, -HP(S)S-, -HP(O)NR₁₂- ou -HP(S)NR₁₂-, où R₁₂ est H ou un alkyle en C₁ à C₆ ; et
oligo représente une séquence naturelle, modifiée ou synthétique d'éléments désoxynucléosides ou acides nucléiques peptidiques naturels, modifiés ou synthétiques, qui est liée par une base nucléique, un pont internucléotidique ou un sucre et dont la région interne est complémentaire d'un ARN cible.

20. Composé selon la revendication 19, **caractérisé en ce que** R₉ représente un radical de formule VI
-Xₚ-A-X'_{q}-A'ᵣ-oligo (VI)
et R₈ représente H, un alkyle en C₁ à C₄, un alcoxy en C₁ à C₄, un aralkyle en C₇ à C₁₂, ou un aryle en C₆ à C₁₆, ou
R₉ représente H, un alkyle en C₁ à C₄, un alcoxy en C₁ à C₄, un aralkyle en C₇ à C₁₂, ou un aryle en C₆ à C₁₆, et R₈ représente un radical de formule VI, et
p, q et r valent, indépendamment l'un de l'autre, 0 ou 1.

21. Composé selon la revendication 19 ou 20, **caractérisé en ce que** q vaut 1.

22. Composé selon l'une des revendications 19 à 21, **caractérisé en ce que** R₂ et R₇ représentent, indépendamment l'un de l'autre, H, un alkyle en C₁ à C₄, un alcoxy en C₁ à C₄, un aralkyle en C₇ à C₁₂, un aryle en C₆ à C₁₆ ou un halogène.

23. Composé selon l'une des revendications 19 à 22, **caractérisé en ce que** R₂ et R₇ représentent, indépendamment l'un de l'autre, H ou un alkyle en C₁ à C₄.

24. Composé selon l'une des revendications 19 à 23, **caractérisé en ce que** R₃ et R₆ représentent, indépendamment l'un de l'autre, H, un alkyle en C₁ à C₄, un aralkyle en C₇ à C₁₂ ou un aryle en C₆ à C₁₆.

25. Composé selon l'une des revendications 19 à 24, **caractérisé en ce que** R₃ et R₆ représentent, indépendamment l'un de l'autre, H ou un alkyle en C₁ à C₄.

26. Composé selon l'une des revendications 19 à 25, **caractérisé en ce que** R₄ représente H ou un alkyle en C₁ à C₂₀.

27. Composé selon l'une des revendications 19 à 26, dans lequel le métal des lanthanides est La, Ce, Nd, Eu ou Gd.

28. Composé selon l'une des revendications 19 à 27, dans lequel le métal des lanthanides est La ou Eu.

29. Composé selon l'une des revendications 19 à 28, dans lequel le métal des lanthanides est Eu.

30. Composé selon l'une des revendications 19 à 29, dans lequel l'anion est F⁻, Cl⁻, Br⁻, J⁻, PF₆⁻, SbF₆⁻, BF₄⁻, B(Ph)₄⁻, acétate, NO₃⁻, sulfate ou phosphate.

31. Composé selon l'une des revendications 19 à 30, dans lequel l'anion est Cl⁻, acétate ou NO₃⁻.

32. Composé selon l'une des revendications 19 à 31, dans lequel l'anion est Cl⁻.

33. Composé selon l'une des revendications 19 à 32, **caractérisé en ce que** X est un alkylène en C₁ à C₃, un alcynylène en C₃, un phénylène ou un aralkylène en C₇.

34. Composé selon l'une des revendications 19 à 33, **caractérisé en ce que** X est un alkylène en C₂ à C₃ ou un phénylène.

35. Composé selon l'une des revendications 19 à 34, **caractérisé en ce que** A est -NR₁₂-CS-NR₁₂- ou -C(O)NR₁₂-.

36. Composé selon l'une des revendications 19 à 35, **caractérisé en ce que** A est -NH-CS-NH ou -C(O)NH-.

37. Composé selon l'une des revendications 19 à 36, **caractérisé en ce que** X' est un alkylène en C₁ à C₂₀.

38. Composé selon l'une des revendications 19 à 37, **caractérisé en ce que** X' est un alkylène en C₁ à C₁₀.

39. Composé selon l'une des revendications 19 à 38, **caractérisé en ce que** A' n'est pas présent ou représente -P(O)(OH)O-.

40. Composé de formule II 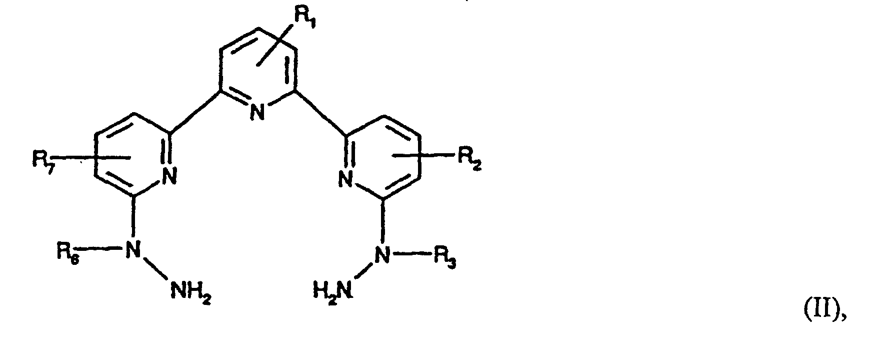 dans laquelle
R₁ est H, un alkyle en C₁ à C₄, un alcoxy en C₁ à C₄, un aralkyle en C₇ à C₁₂ ou un aryle en C₆ à C₁₆, ou un groupe fonctionnel monovalent, le groupe fonctionnel étant lié directement ou par un groupe Z au cycle pyridine, et le groupe Z représentant un radical, éventuellement interrompu par -O-, -S-, -NR₁₂-, -C(O)-O- ou -C(O)-NR₁₂-, choisi dans le groupe composé d'un alkylène en C₁ à C₂₀, un alcénylène en C₂ à C₁₂, un alcynylène en C₂ à C₁₂, un cycloalkylène en C₅ à C₈, un arylène en C₆ à C₁₂ et un aralkylène en C₇ à C₁₂,
R₂ et R₇ représentent, indépendamment l'un de l'autre, H, un alkyle en C₁ à C₄, un alcoxy en C₁ à C₄, un aralkyle en C₇ à C₁₂, un aryle en C₆ à C₁₆ ou un halogène, et
R₃ et R₆ représentent, indépendamment l'un de l'autre, H, un alkyle en C₁ à C₄, un aralkyle en C₇ à C₁₂ ou un aryle en C₆ à C₁₆,
R₁₂ représente H ou un alkyle en C₁ à C₆,
où les radicaux alkyle, cycloalkyle, aralkyle, aryle, et le groupe Z, sont non substitués ou substitués par un alcoxy en C₁ à C₄, F, Cl, Br, -CN, un alkyle en C₁ à C₄ ou -NO₂,
à la condition que soient exclus les composés de formule (II) dans lesquels R₂ et R₇ représentent respectivement H, R₃ et R₆ représentent respectivement -CH₃, et R₁ représente H ou un 4-phényle ; ou dans lesquels R₁ représente H, R₂ et R₇ représentent respectivement un 4-phényle, et R₃ et R₆ représentent respectivement H ou -CH₃.

41. Composé de formule III, 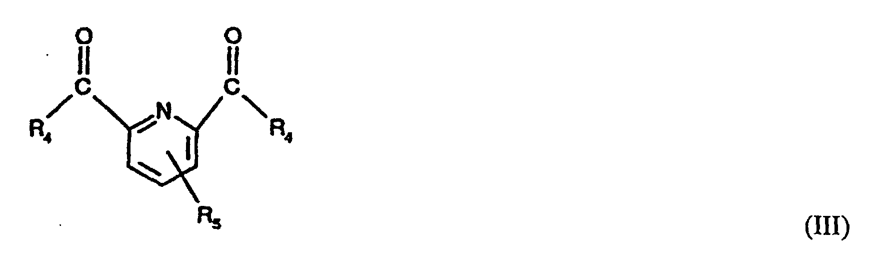 dans laquelle
R₅ représente un groupe fonctionnel monovalent lié au cycle pyridine par un alkylène en C₂ à C₂₀, le groupe fonctionnel étant choisi dans le groupe composé de -C(O)-OR₁₂, -C(O)-NHR₁₂, -SO₂-R₁₂ et -SO₂-NHR₁₂, où R₁₂ représente H ou un alkyle en C₁ à C₆, et
R₄ représente H ou un alkyle en C₁ à C₂₀.

42. Procédé de préparation de composés de formule I, **caractérisé en ce que** l'on condense une terpyridine de formule II 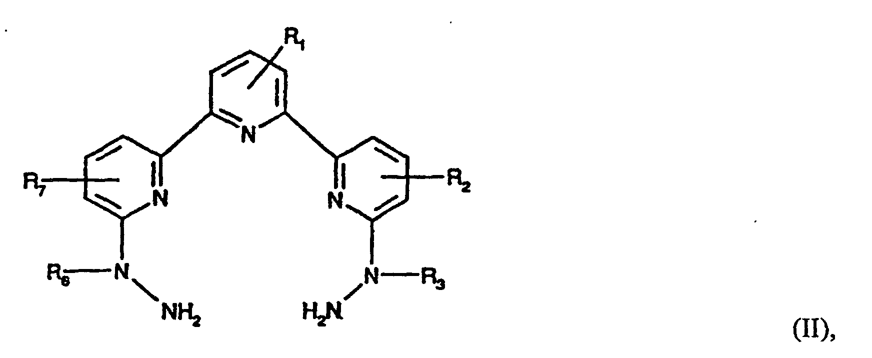 avec un pyridine-dialdéhyde ou une pyridinedicétone de formule III 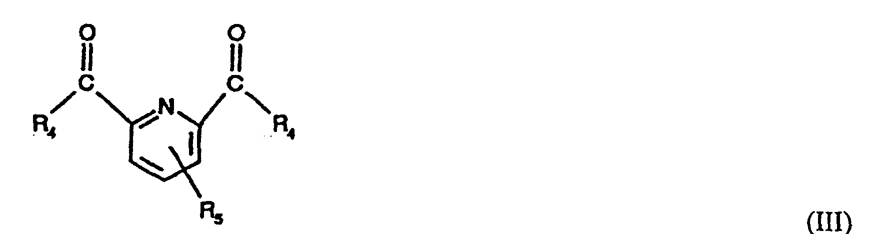 en présence d'un sel de formule IV
Meⁿ⁺ (Y^{m-})_{n/m} (IV)
où R₁, R₂, R₃, R₄, R₅, R₆, R₇, Me, Y, n et m ont la signification précédemment mentionnée.

43. Procédé de préparation de composés de formule (V) selon l'une des revendications 19 à 39, **caractérisé en ce que** l'on fait réagir un composé de formule (I) selon l'une des revendications 1 à 18
(a) avec un composé de formule VIa
A"-X'-A'_{0 ou 1}-oligo (VIa)
dans laquelle
A" est un groupe fonctionnel monovalent approprié choisi dans le groupe composé de -OR₁₀, -SR₁₀, -NCO, -NCS, -NHR₁₁, -C(O)OR₁₁, -C(O)SH, -C(O)NHR₁₁, -C(O)Cl, -C(S)SR₁₁, -C(S)NHR₁₁, -C(S)OR₁₁, -SO₃R₁₁, -SO₂NHR₁₁, -SO₂Cl, -P(O)(OH)₂, -P(O)(OH)-NHR₁₁, -P(S)(SH)₂, -P(S)(SH)-NHR₁₁, -P(S)(OH)₂, -P(S)(OH)-NHR₁₁, -P(O)(SH)₂, -P(O)(SH)-NHR₁₁, -P(O)(OH)H, -P(O)(NHR₁₁)H, -P(S)(SH)H, -P(S)(NHR₁₁)H, -P(S)(OH)H, -P(O)(SH)H, où R₁₀ représente H, -C(O)NH₂, -C(S)NH₂, -alkyle en C₁ à C₆, -CₓH₂ₓ-NH₂, -CₓH₂ₓ-SH ou -(CₓH₂ₓO)_{y}-H, et R₁₁ représente H, -alkyle en C₁ à C₆, -CₓH₂ₓ-NH₂, -CₓH₂ₓ-SH ou -(CₓH₂ₓO)_{y}-H et x est un nombre compris entre 2 et 6 et y est un nombre compris entre 1 et 20,
X' est un radical non substitué ou substitué par un alcoxy en C₁ à C₄, F, Cl, Br, -CN, un alkyle en C₁ à C₄ ou -NO₂, choisi dans le groupe composé d'un alkylène en C₁ à C₂₀, un alcénylène en C₂ à C₁₂, un alcynylène en C₂ à C₁₂, -(CₓH₂ₓO)_{y}-, où x est un nombre compris entre 2 et 6 et y est un nombre compris entre 1 et 20, un cycloalkylène en C₅ à C₈, un arylène en C₆ à C₁₂ et un aralkylène en C₇ à C₁₂,
A' représente -O-, -S-, -S-S-, -NR₁₂-CO-NR₁₂-, -NR₁₂-CS-NR₁₂-, -NR₁₂-, -NR₁₂-C(O)-O-, -C(O)O-, -C(O)S-, -C(O)NR₁₂-, -C(S)S-, -C(S)O-, -C(S)NR₁₂, -SO₂NR₁₂-, -SO₂-, -P(O)(OH)O-, -P(S)(SH)S-, -P(S)(SH)O-, -P(S)(OH)O-, -P(O)(SH)S-, -P(O)(OH)S-, -P(O)(SH)O-, -P(O)(OH)-NR₁₂-, -P(S)(SH)-NR₁₂-, -P(S)(OH)-NR₁₂-, -P(O)(SH)-NR₁₂-, -HP(O)O-, -HP(S)S-, -HP(O)NR₁₂- ou -HP(S)NR₁₂-, où R₁₂ est H ou un alkyle en C₁ à C₆; et
oligo représente une séquence naturelle, modifiée ou synthétique d'éléments désoxynucléosides ou acides nucléiques peptidiques naturels, modifiés ou synthétiques, qui est liée par une base nucléique, un pont internucléotidique ou un sucre et dont la région interne est complémentaire d'un ARN cible, ou
(b) avec un composé de formule VIb
A"-oligo (VIb)
dans laquelle
A" et oligo ont les significations mentionnées au point (a).

44. Procédé selon la revendication 43, **caractérisé en ce que** R₁₀ représente H.

45. Procédé de séparation du pont nucléotidique phosphate d'acides ribonucléiques dans des conditions physiologiques et sous l'action d'un conjugué de complexe métallique et d'oligonucléotide, **caractérisé en ce que** (a) on complexe l'ARN cible avec un composé selon l'une des revendications 19 à 39, et (b) on fait réagir et on sépare.

46. Composés de formule (V) selon l'une des revendications 19 à 39, destinés à une application dans un procédé thérapeutique de traitement de maladies chez des animaux à sang chaud, y compris l'homme, par inactivation de séquences nucléotidiques du corps.

47. Préparation pharmaceutique à base d'une solution aqueuse ou d'une suspension, contenant une quantité efficace des composés de formule (V) selon l'une des revendications 19 à 39 seuls ou conjointement à d'autres principes actifs, de l'eau en tant qu'agent véhiculeur pharmaceutique et éventuellement des auxiliaires.
